# EUROPEAN PATENT APPLICATION

(11) **EP 4 559 937 A1**
(43) Date of publication of application: **28.05.2025**
(21) Application number: 23383206.2
(22) Date of filing: 24.11.2023
(51) Int. Cl.: C07K 16/28, A61K 39/00, A61P 35/02

(54) **CD7 TARGETING MOIETIES FOR THE TREATMENT OF CD7 POSITIVE CANCER**

(71) Applicant: Universitat de Barcelona, 08028 Barcelona (ES); Fundació de Recerca Clínic Barcelona-Institut d'Investigacions Biomèdiques August Pi I Sunyer, 08036 Barcelona (ES); Hospital Clínic de Barcelona, 08036 Barcelona (ES)
(72) Inventor: GUEDÁN CARRIÓ, Sonia, 08036 Barcelona (ES); JUAN OTERO, Manuel, 08036 Barcelona (ES); DELGADO GONZÁLEZ, Julio, 08036 Barcelona (ES); URBANO ISPIZUA, Álvaro, 08036 Barcelona (ES); URIBE HERRANZ, Mireia, 08036 Barcelona (ES); ORTIZ DE LANDAZURI PASCAL, Iñaki, 08036 Barcelona (ES); SORIA CASTELLANO, Marta, 08036 Barcelona (ES)
(74) Representative: Hoffmann Eitle

(57) **Abstract**

The present invention relates to a CD7 targeting moiety, wherein the CD7 targeting moiety is an antibody, F(ab')2, Fab, scFab or scFv. The present invention further provides CD7 retention domains, CARs, nucleic acid, cells, pharmaceutical compositions and kits comprising the CD7 targeting moiety. Lastly, methods of treatment of a CD7-positive cancer, preferably T-ALL are also provided.

## Description

### TECHNICAL FIELD

The present invention provides therapeutics for the treatment of CD7-positive cancers. In particular, the present invention provides three CD7 targeting moieties and uses thereof.

### BACKGROUND ART

T-cell malignancies are a highly heterogeneous group of diseases with poor prognoses that stem from T-cell precursors as well as mature T-cells and can be classified into T-cell leukemia and peripheral T-cell lymphomas (PTCLs). T-cell lineage acute lymphoblastic leukemia (T-ALL) is a malignant disorder resulting from leukemic transformation of thymic T-cell precursors. T-ALL is phenotypically and genetically heterogeneous and is commonly associated with genetic alterations/mutations in transcription factors involved in hematopoietic stem/progenitor cell (HSPC) homeostasis and in master regulators of T-cell development. T-ALL comprises 10-15% and 20-25% of all acute leukemia's diagnosed in children and adults, respectively with a median diagnostic age of 9 years 5-7. Intensive chemotherapy regimens have led to the improved survival of patients with T-ALL. However, the event-free (EFS) and overall (OS) survival remains <70%, and relapsed/refractory (R/R) T-ALL has a particularly poor outcome. There are currently no potential curative options beyond hematopoietic cell transplantation and conventional chemotherapy, which is linked to large trade-offs in toxicities reinforcing the need for novel targeted therapies.

Immunotherapy has generated unprecedented expectations in cancer treatment and relies on the immune system as a powerful weapon against cancer. In recent years, adoptive transfer of T cells genetically modified to express chimeric antigen receptors (CAR-T) has shown great potential. The introduction of a CAR into a T-cell allows the specific recognition and specific elimination of antigen-expressing tumor cells in a major histocompatibility complex-independent fashion. CAR-T cells have demonstrated exceptional clinical efficacy against B cell malignancies. However, the development of CAR-T cell therapy against T cell malignancies has proven problematic, in part due to the shared expression of target antigens between malignant T cells and effector T cells. Expression of the target antigens on CAR-T cells may induce fratricide of CAR-T cells, preventing proper CAR-T cell manufacturing, loss of efficacy after CAR-T cell infusion and therefore, reduced clinical benefit. Therefore CAR-T cells that do not induce fratricide but are effective in the treatment of T cell malignancies are needed.

Many T cell malignancies overexpress CD7, providing an attractive target for immunotherapy of T cell cancers. However, normal T cells, including those used to generate the CAR-T cell infusion product also express CD7 (86%). Thus, CD7-targeted CAR-T cells induce T cell fratricide, limiting therapeutic potential. Consequently, there remains a need for a therapy that can successfully treat T-ALL, with a reduced fratricide. The present invention aims to provide a therapy for treating CD7-positive T-ALL.

### SUMMARY OF INVENTION

The present invention provides a **CD7 targeting-moiety,** wherein the CD7 targeting-moiety is an antibody, F(ab')2, Fab, scFab or scFv, comprising a VL domain and a VH domain, wherein:
- the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, or
- the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9.

Preferably, the CD7 targeting-moiety is a scFv comprising a VL domain and VH domain, wherein:
- the VL domain consists of SEQ ID NO: 20 **(CD7_142.24 VL)** and the VH domain consists of SEQ ID NO: 21 **(CD7_142.24 VH),** or
- the VL domain consists of SEQ ID NO: 1 **(CD7_124.1D1 VL)** and the VH domain consists of SEQ ID NO: 2 **(CD7_124.1D1 VH).**

Preferably, the CD7 targeting-moiety is a scFv consisting of SEQ ID NO: 11 **(CD7_142.24)** or SEQ ID NO: 3 **(CD7_124.1D1).**

The present invention also provides a **chimeric antigen receptor (CAR)** comprising:
a. an extracellular domain comprising a CD7 targeting-moiety, wherein the CD7 targeting-moiety is as defined above;
b. a transmembrane domain; and
c. at least one intracellular signaling domain.

Preferably, the transmembrane domain comprises the transmembrane domain of CD8. Preferably, the intracellular signaling domain comprises the intracellular domain of CD3ζ. Preferably, the CAR further comprises a costimulatory signaling domain, wherein said costimulatory signaling domain comprises the intracellular domain of CD137. Preferably, the CAR comprises or consists of SEQ ID NO: 12 **(CD7 CAR 142.24)** or 13 **(CD7 CAR 124.1D1).**

The present invention also provides a **CD7-trap protein** comprising the CD7 targeting moiety defined above, and further comprising a CD7-retention sequence comprising SEQ ID NO: 30 (ER retention sequence). Preferably, the CD7-trap protein comprises SEQ ID NO: 31 **(CD7 trap 124.1D1).**

The present invention also provides a **combination therapy** comprising administering:
i) the CAR against CD7 above, or a nucleic acid molecule encoding thereof, and
ii) the CD7-trap protein as defined above, or a nucleic acid molecule encoding thereof.

Preferably, in the combination therapy:
i) the CAR against CD7, or a nucleic acid molecule encoding thereof, comprises:
   (a) an extracellular domain comprising a CD7 targeting moiety that is a ScFv comprising a VL domain and a VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, and
   (b) a transmembrane domain, preferably the transmembrane domain of CD8;
   (c) an intracellular signaling domain, preferably the intracellular domain of CD3ζ, and
   (d) optionally, at least a costimulatory signaling domain, preferably the intracellular domain of CD137, and
ii) the CD7-trap protein, or the nucleic acid molecule encoding thereof, comprises:
   (a) a ScFv comprising a VL and a VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
   (b) a retention sequence that comprises SEQ ID NO: 30 [AEKDEL].

Preferably, in the combination therapy:
i) the CAR against CD7 consists of SEQ ID NO: 12, or a nucleic acid molecule encoding thereof, and
ii) the CD7-trap protein consists of SEQ ID NO: 31, or a nucleic acid molecule encoding thereof.

The present invention also provides a **nucleic acid** encoding for one or more of the CD7 targeting moiety as defined above, the CAR as defined above, or the CD7-trap as defined above. Preferably, the nucleic acid is a bicistronic nucleic acid and comprises:
- a first nucleotide sequence encoding for the CAR as defined above, and
- a second nucleotide sequence encoding for the CD7-trap protein as defined above.

Preferably, the bicistronic nucleic acid comprises:
- a first nucleotide encoding for a CAR against CD7 that comprises:
   (a) an extracellular domain comprising a CD7 targeting moiety that is a ScFv comprising a VL domain and a VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, and
   (b) a transmembrane domain, preferably the transmembrane domain of CD8;
   (c) an intracellular signaling domain, preferably the intracellular domain of CD3ζ, and
   (d) optionally, at least a costimulatory signaling domain, preferably the intra-cellular domain of CD137, and
- a second nucleotide sequence encoding for the CD7-trap protein that comprises:
   (a) a ScFv comprising a VL and a VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
   (b) a retention sequence comprising SEQ ID NO: 30 [AEKDEL],

Preferably, the bicistronic nucleic acid comprises:
- a first nucleotide sequence encoding for a CAR anti-CD7 that comprises SEQ ID NO: 12 **(CD7_142.24),** and
- a second nucleotide sequence encoding for a CD7-trap that comprises SEQ ID NO: 31 **(CD7_124.1D1),**
preferably wherein the first and second nucleotide sequences are connected by a nucleic acid sequence encoding for a 2A cleavage peptide, preferably a T2A cleavage peptide.

Preferably, the bicistronic nucleic acid sequence encodes for the amino acid sequence of SEQ ID NO: 75 or 73.

The present invention also provides a cell comprising and/or expressing one or more of:
- the CD7 targeting-moiety as defined above,
- the CAR as defined above,
- the CD7-trap protein as defined above, and/or
- the nucleic acid as defined above.

Preferably, the cell comprises and/or expresses both the CD7-trap protein as defined above and the CAR as defined above, wherein preferably the CAR consists of SEQ ID NO: 12 **(CD7 CAR 142.24)** and the CD7-trap protein comprises SEQ ID NO: 31 **(CD7 trap 124.1D1).**

The present invention also provides a composition, preferably a pharmaceutical composition, comprising the cell as defined above.

The present invention also provides the CD7 targeting-moiety as defined above, the CAR as defined above, the CD7-trap protein as defined above, the combination therapy as defined above, the nucleic acid as defined above, the cell as defined above, the composition as defined above, **for use** in therapy or in the manufacture of a medicament.

Preferably, the use in a method of treating a CD7-positive cancer. Preferably the CD7-positive cancer is T cell acute lymphoblastic leukemia (T-ALL).

### BRIEF DESCRIPTION OF DRAWINGS

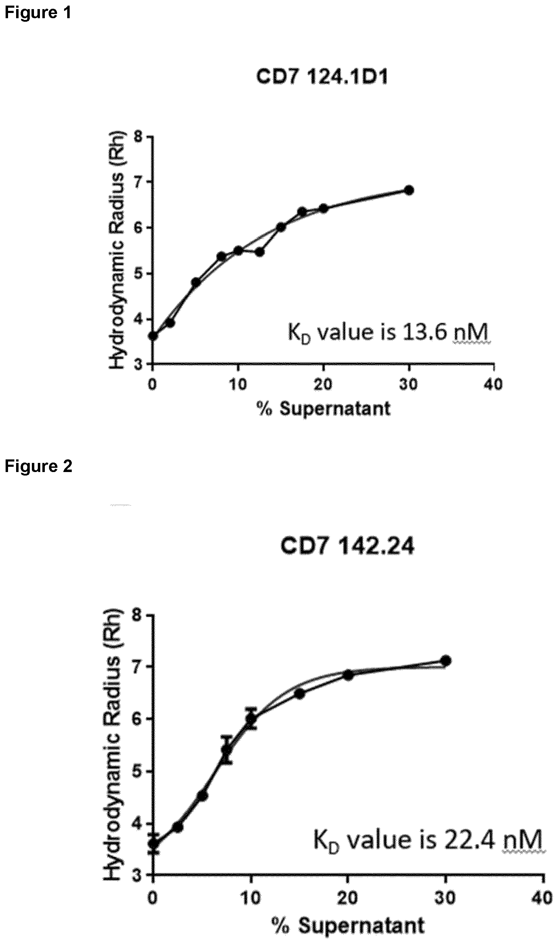

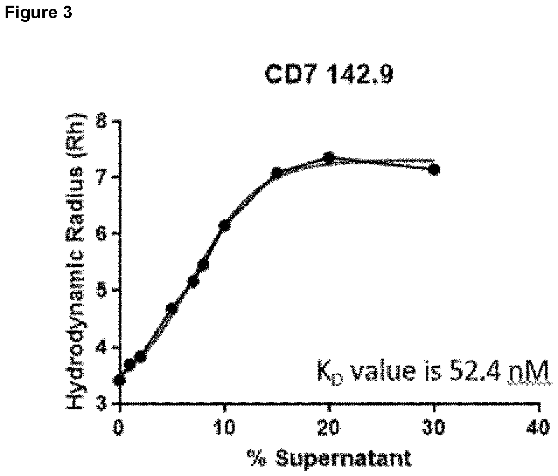

**Fig. 1, 2** **and** **3****: Quantification and characterization of the protein binding affinities of the anti-CD7 antibodies.** Fluorescently labelled CD7 and hybridoma's 124.1D1 (Fig. 1), 142.24 (Fig. 2) and 142.9 (Fig. 3) supernatants were mixed. Change in Hydrodynamic radius (Rh) upon binding was measured on the Fluidity One-W system. K_{D} values were calculated by Bayesian inference. Representative plots from 1 out of 3 experiments.

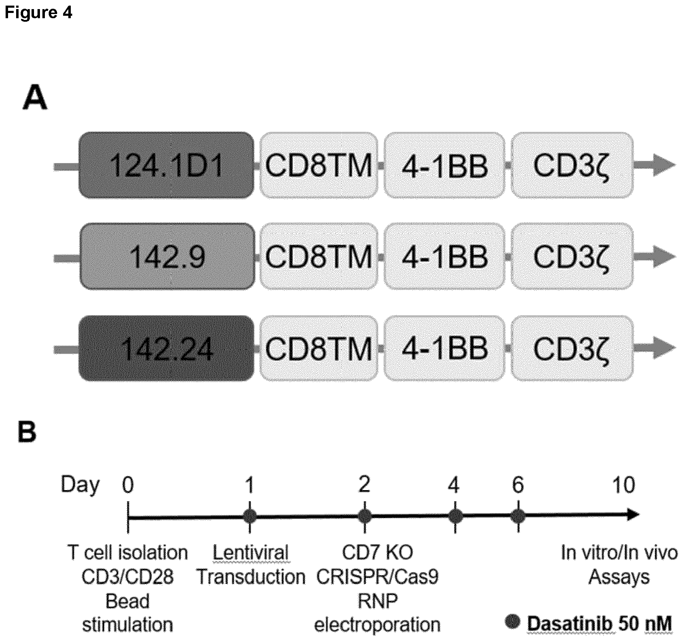

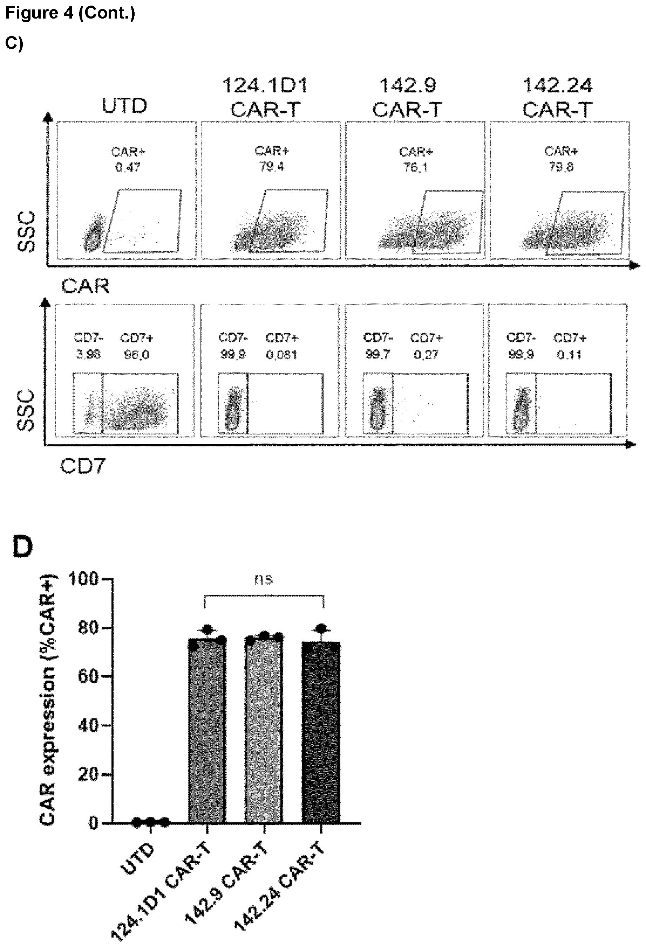

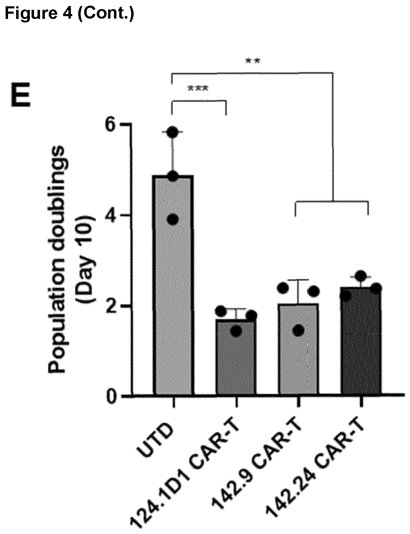

**Fig. 4****: Development of CD7KO anti-CD7 CAR-T cells.** A) Schematic diagram of second-generation CARs designed for this study. CARs differ in the scFv used: 124.1D1, 142.9 and 142.24. All CARs contain the CD8 hinge and transmembrane domain, and the 4-1BB and CD3z intracellular domains. B) Protocol layout for CAR-T cell generation and expansion. Human primary T cells were isolated from buffy coats of normal donors and activated by bead stimulation at day 0. Lentiviral transduction was done at day 1 and culture media was supplemented with dasatinib at 50 nM from day 1 to day 6. At day 3 of expansion, activation beads were removed and CRISPR/Cas9 CD7KO was performed. CD7KO CAR-T cells were expanded for 10 days and then used for in vitro and in vivo experiments. C) Representative dot plots showing expression of CAR and CD7 on the T cell surface assessed by flow cytometry. CAR expression was determined at day 9 and CD7 at day 6 of expansion. D) Quantification of CAR expression at day 9 of expansion (n=3). E) Population doublings of CAR-T cells at day 10 of expansion. All data are means +/- standard deviation (SD) from three normal donors; each dot represents a different normal donor. *indicates p< 0.05; ** indicates p<0.01; ***indicates p<0.001; **** indicates p<0.0001; ns indicates non-significant differences, one-way ANOVA. UTD: Untransduced T cells.

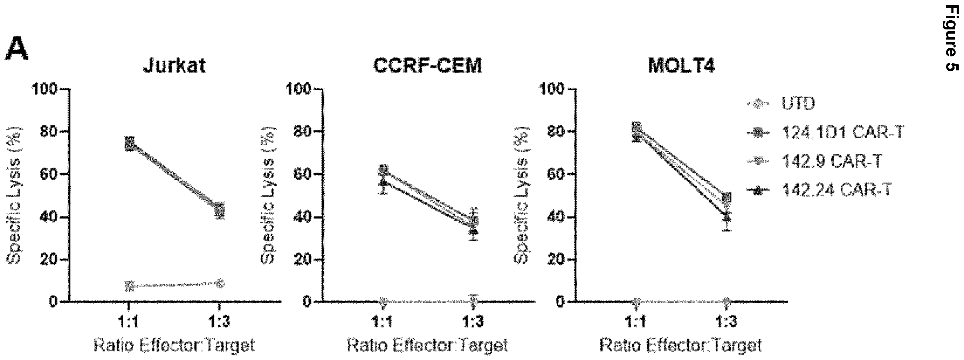

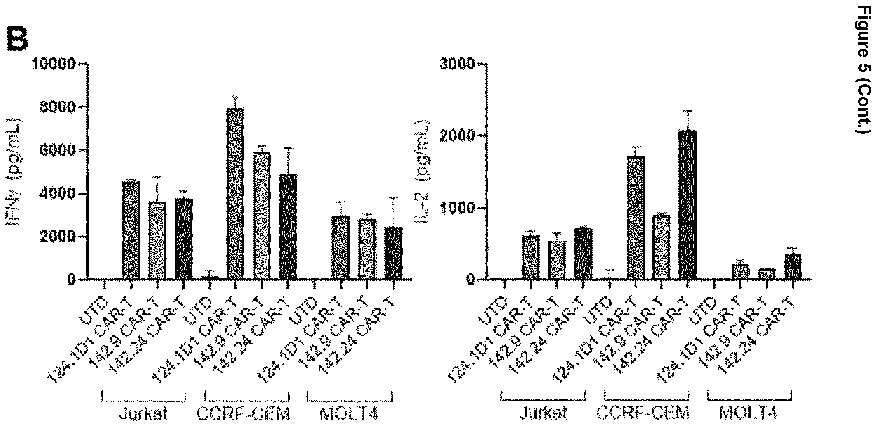

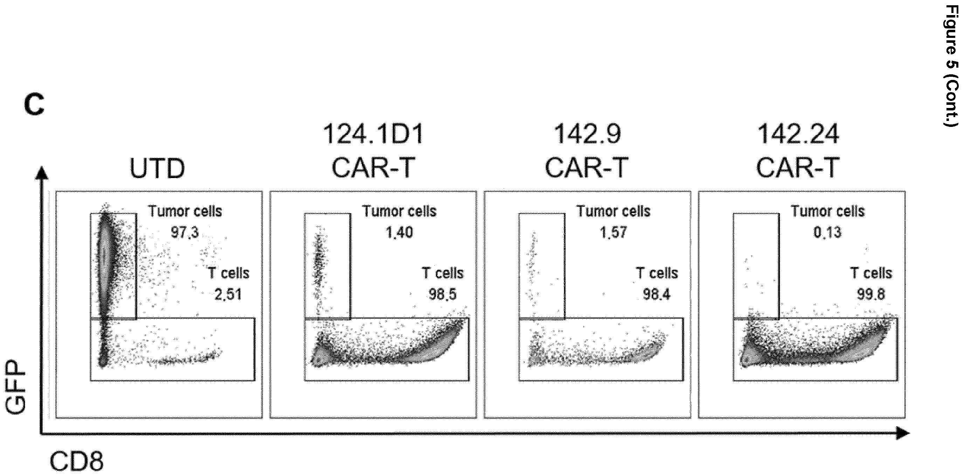

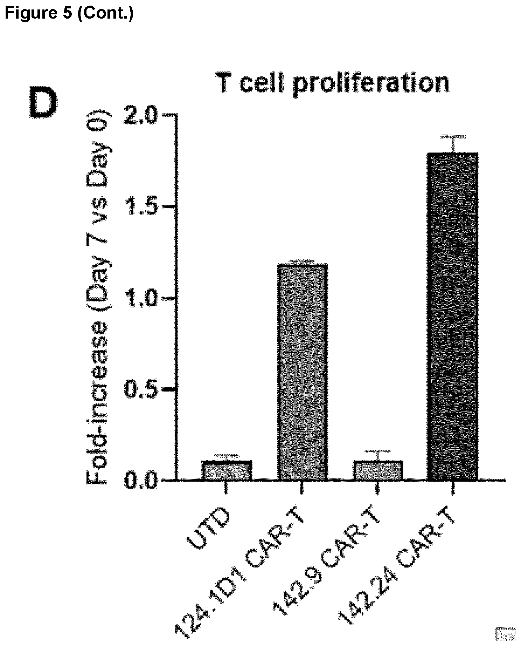

**Fig. 5****: Functional characterization of CD7KO anti-CD7 CAR-T cells *in vitro.* A)** CD7KO anti-CD7 CAR-T cells specifically kill CD7+ target cells (Jurkat, CCRF-CEM and MOLT4). Indicated CAR-T cells and target cells were mixed at an effector:target ratio (E:T) of 1:1 and 1:3 and cytotoxicity was evaluated 24 hours later in a luciferase-based assay. B) CD7KO anti-CD7 CAR-T cells secrete cytokines upon coculture with CD7+ cells (Jurkat, CCRF-CEM and MOLT4). CAR-T cells were mixed with tumor cells at an E:T=3:1. IFNγ and IL-2 secretion was determined by ELISA after 24 hours of stimulation with target cells. C-D) CD7KO anti-CD7 CAR-T cells proliferate upon coculture with CD7+ cells (Jurkat, CCRF-CEM and MOLT4). CAR-T and CD7+ Jurkat cells were cocultured at a E:T=1:2 for 7 days. Absolute count of live cells was measured by flow cytometry using counting beads at the end of coculture. C) Representative flow cytometry plots showing the percentages of tumor and CAR-T cells. D) Quantification of the fold increase of CAR-T cells at day 7 versus day 0. Representative results from one of three normal donors are shown. UTD: Untransduced T cells.

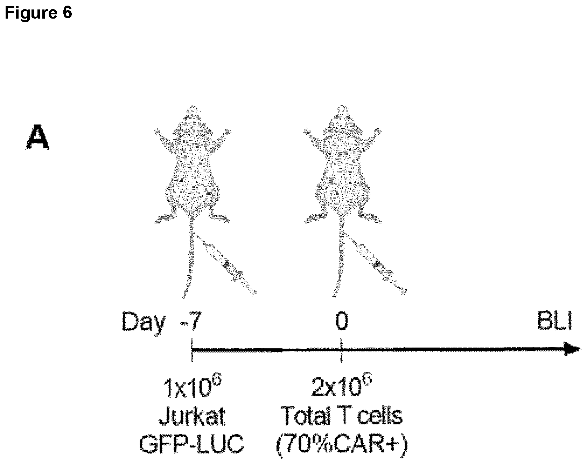

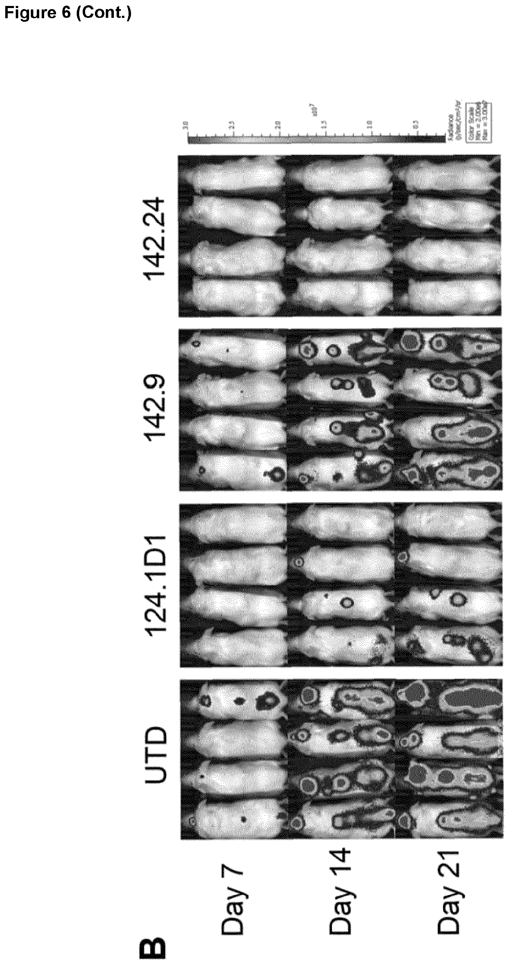

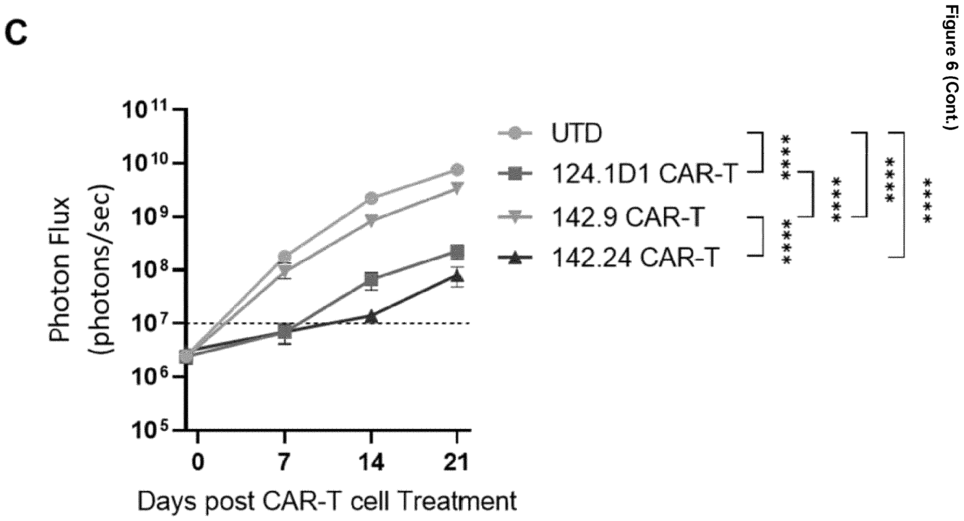

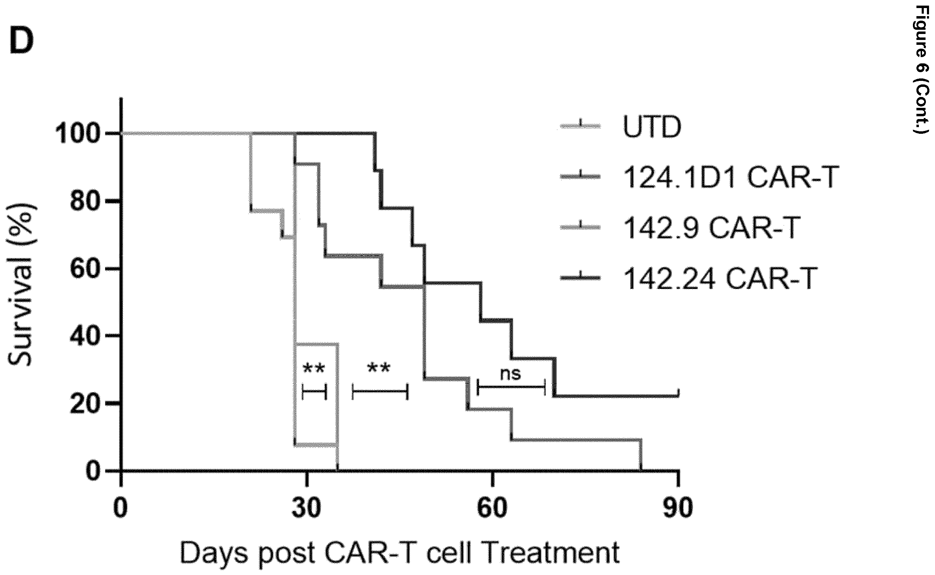

**Fig. 6****: CD7KO anti-CD7 CAR-T cells control progression of systemic T cell acute lymphoblastic leukemia (T-ALL) in a mouse xenograft model.** A) Schematic procedure to generate a T-ALL mouse xenograft model. NSG mice were injected intravenously with 1×10⁶ GFP-LUC Jurkat cells followed by a single intravenous injection of 2×10⁶ of control T cell (untransduced, UTD) or CD7KO anti-CD7 CAR-T cells 7 days later. Tumor growth was monitored weekly via bioluminescent imaging (BLI) using an IVIS Lumina. B) Representative images of four animals of each group during the first three weeks after treatment. C) Overall kinetics of systemic tumor progression in mice during the first three weeks after treatment. All data are means +/- standard error of the mean (SEM). Two-way ANOVA of data at day 21. D) Kaplan-Meier survival curve (log-rank test) of mice treated with control or CD7KO anti-CD7 CAR-T cells. Mixed data from three independent in vivo experiments; UTD (n=14), 124.1D1 (n=11), 142.9 (n=17), 142.24 (n=10). Ns indicates not significant,* indicates p< 0.05; ** indicates p<0.01; ***indicates p<0.001; **** indicates p<0.0001

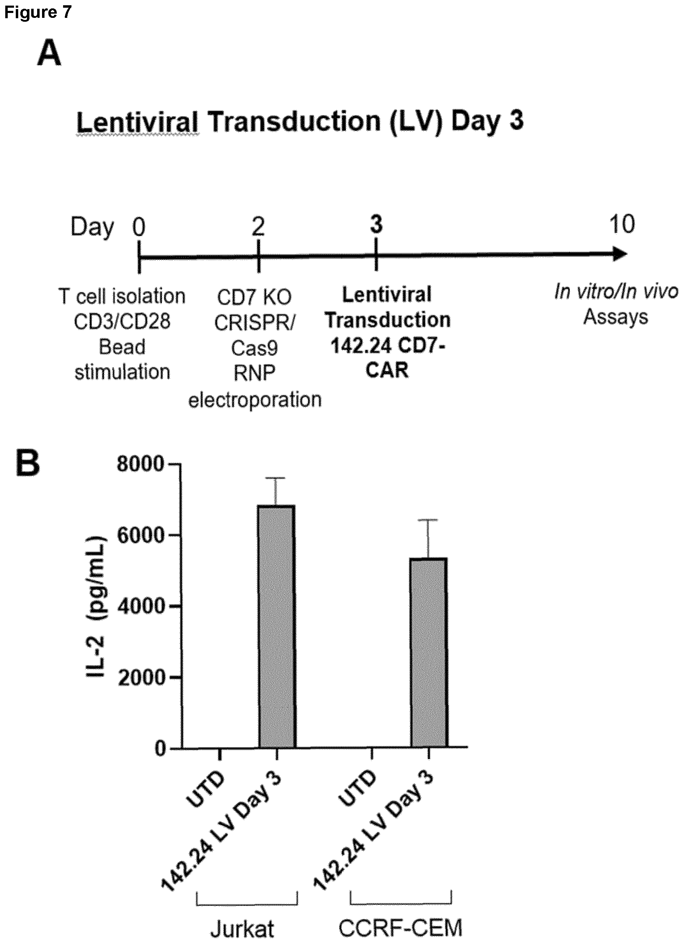

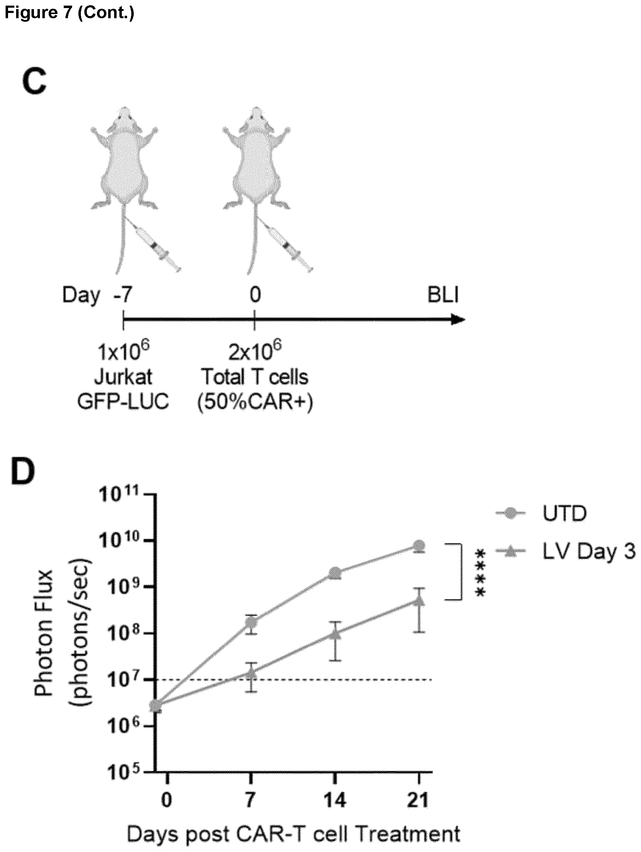

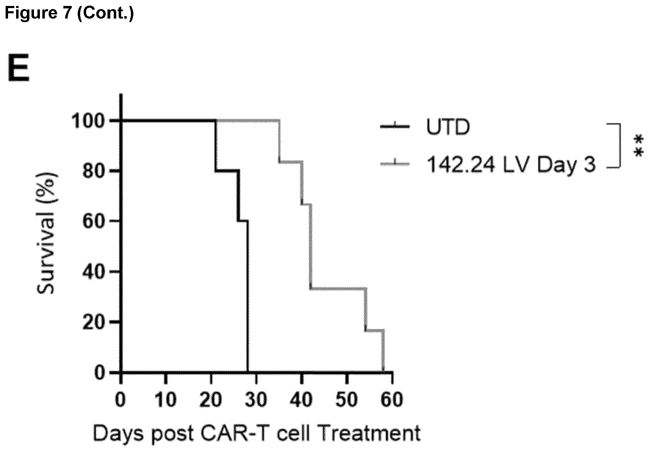

**Fig. 7****: Generation of anti-CD7 142.24 CAR-T cells by lentiviral transduction after disrupting the CD7 gene by CRISPR/Cas9 technology.** A) Protocol layout for CAR-T cell generation and expansion. Primary T cells were isolated from buffy coats of normal donors and activated by bead stimulation at day 0. Activation beads were removed at day 2 of expansion and CRISPR/Cas9-mediated CD7KO was performed. Lentiviral transduction (LV) was done one day after disrupting the CD7 gene (day 3). CD7KO anti-CD7 CAR-T cells were expanded for 10 days and used for in vitro and in vivo experiments. B) Anti-CD7 CAR-T cells secrete cytokines upon CD7+ target cells stimulation (Jurkat and CCRF-CEM tumor cells). CAR-T cells were mixed with tumor cells at an E:T=3:1. IL-2 secretion was determined by ELISA after 24h stimulation with target cells. C) Schematic procedure to generate a T-ALL mouse xenograft model. NSG mice were injected intravenously with 1×10⁶ GFP-LUC Jurkat cells followed by a single intravenous injection of 2×10⁶ of untransduced (UTD) or CD7KO LV Day 3 CAR-T cells 7 days later. Tumor growth was monitored weekly via bioluminescent imaging (BLI) using an IVIS Spectrum. D) Overall kinetics of systemic tumor progression in mice during the first three weeks after treatment. All data are means +/- standard error of the mean (SEM). Two-way ANOVA of data at day 21. E) Kaplan-Meier survival curves (log-rank test) of mice treated with control or CD7KO LV Day 3 CAR-T cells (n=6).* indicates p< 0.05; ** indicates p<0.01; ***indicates p<0.001; **** indicates p<0.0001.

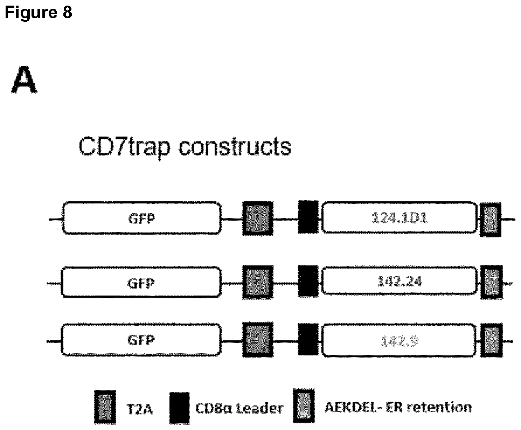

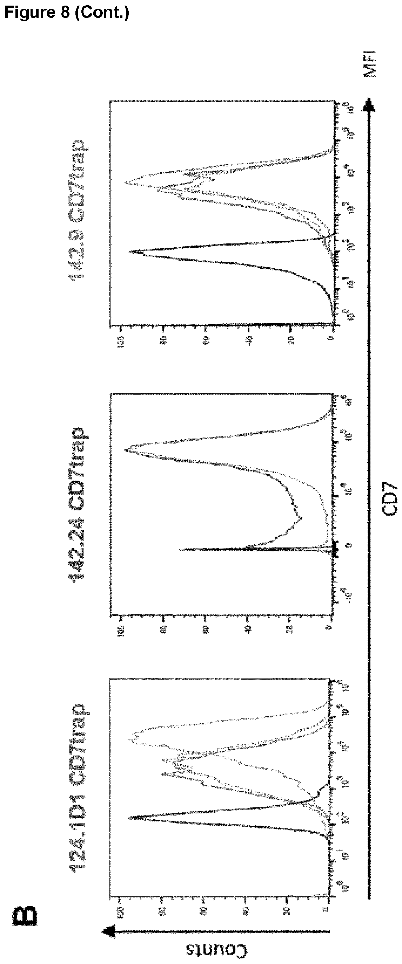

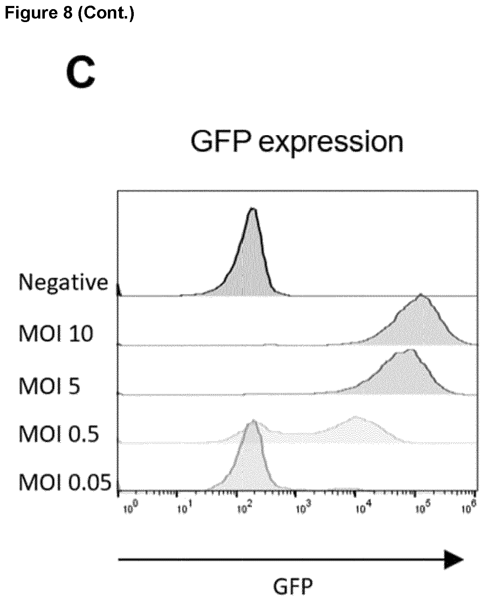

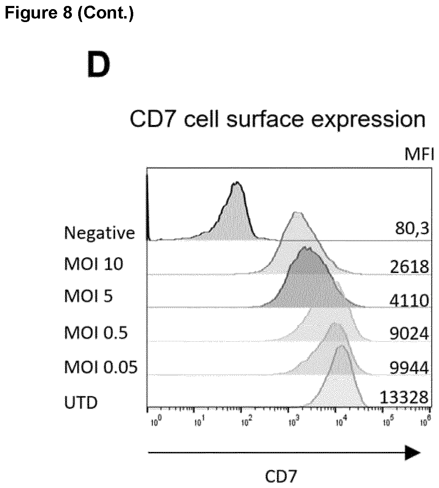

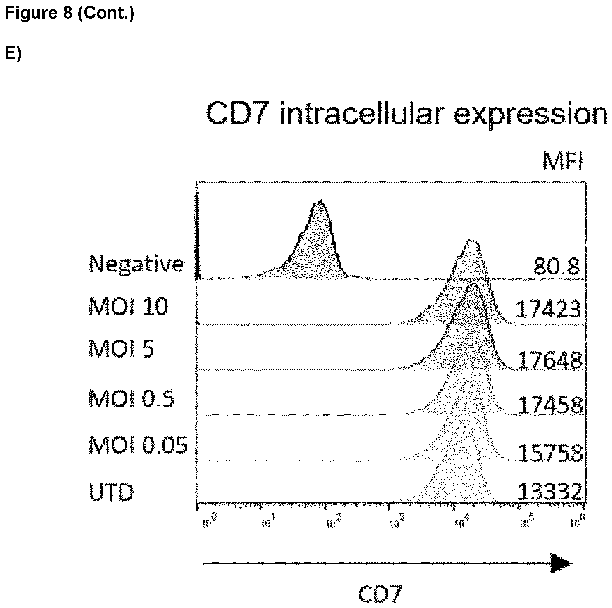

**Fig. 8****: Functional characterization of the CD7trap constructs.** A) Schematic representation of the CD7trap constructs. All differ in the scFv (124.1D1, 142.24, 142.9) that binds to an endoplasmic reticulum retention sequence (AEKDEL-ER retention). B) Representative histogram plots showing CD7 cell surface expression on CD7trap transduced human primary T cells. Colored line represents CD7trap transduced human primary T cells. Black line represents unstained human primary T cells as negative control and grey line shows human primary T cells stained as positive control. C-D) Jurkat cells were transduced with a lentiviral vectors expressing the 124.1D1 CD7trap constructs at different multiplicities of infection (MOI). The expression of C) GFP, D) cell surface CD7 or E) intracellular CD7 was analyzed by flow cytometry. MFI: median fluorescence intensity.

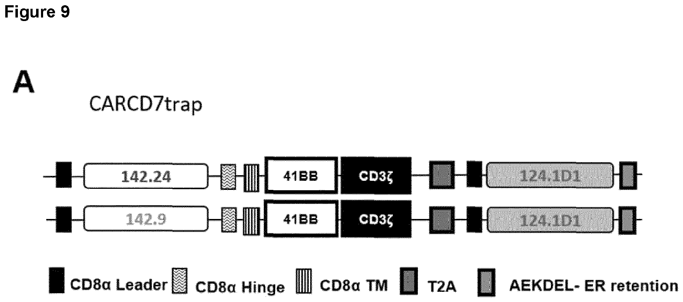

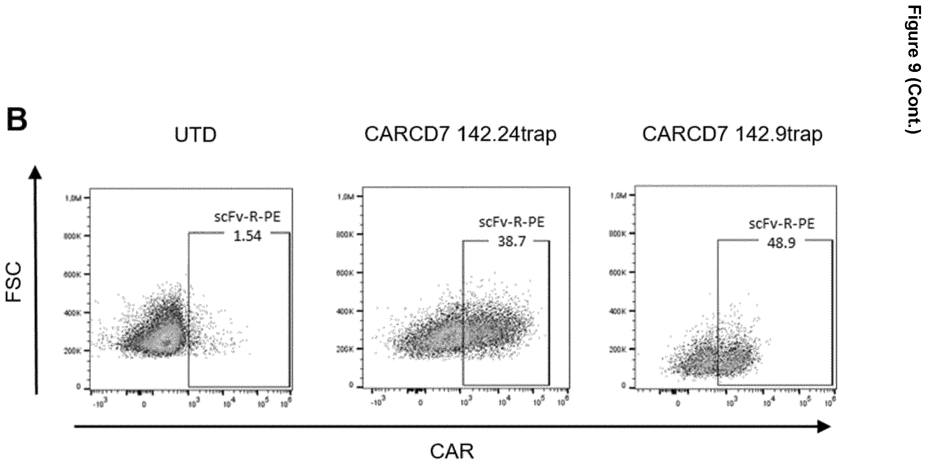

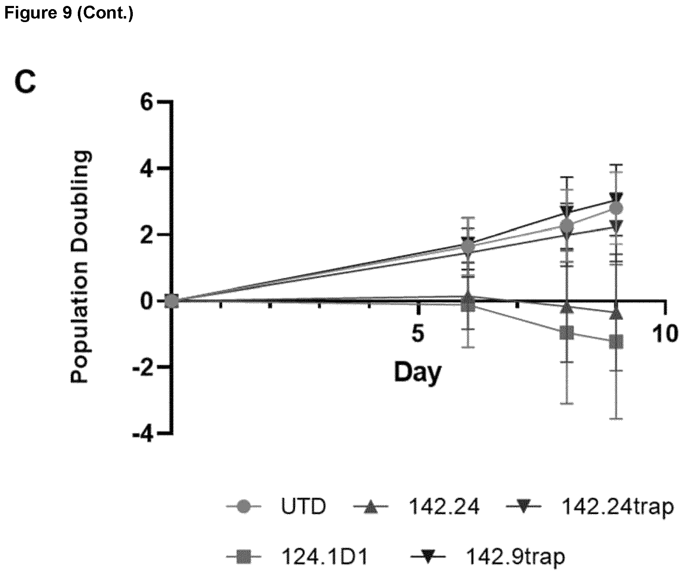

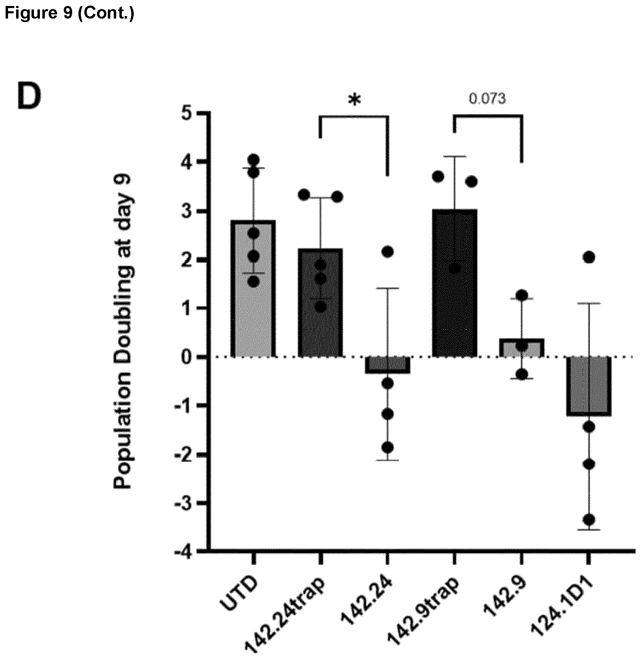

**Fig. 9****: Generation of anti-CD7 CAR-T cells containing the 142.24trap or 142.9trap.** A) Schematic diagram of a bicistronic construct that allows the coexpression of a TRAP based on the 124.1D1 scFv and the anti-CD7 CAR (CARCD7trap). The constructs differ in the scFv (142.24 or 142.9) included in the CAR. Both constructs contain the CD8 hinge and transmembrane domain, the 4-1BB and CD3ζ intracellular domains. The CD7trap contain the anti-CD7 124.1D1 scFv and is linked to an endoplasmic reticulum retention sequence (AEKDEL-ER retention). B) Representative dot plots showing CAR expression on the T cell surface assessed by flow cytometry at day 10 of expansion. C) Population doublings for CARCD7 T cells (142.24 or 124.1D1 41BB-based CARs) and CARCD7trap T cells at different timepoints during ex vivo expansion following activation with anti-CD3/CD28 beads. Expression of the CD7trap in CD7CAR-T cells allows proper T cell expansion. D) Population doublings of CAR-T cells at day 9 of expansion. All data are means +/- standard deviation (SD) from UTD n=5, CARCD7 142.24trap n=5, CARCD7 142.24 n=4, CARCD7 142.9trap n=3, CARCD7 142.9 n=3 and CARCD7 124.1D1 n=4. Each dot represents a different normal donor. * indicates p< 0.05.

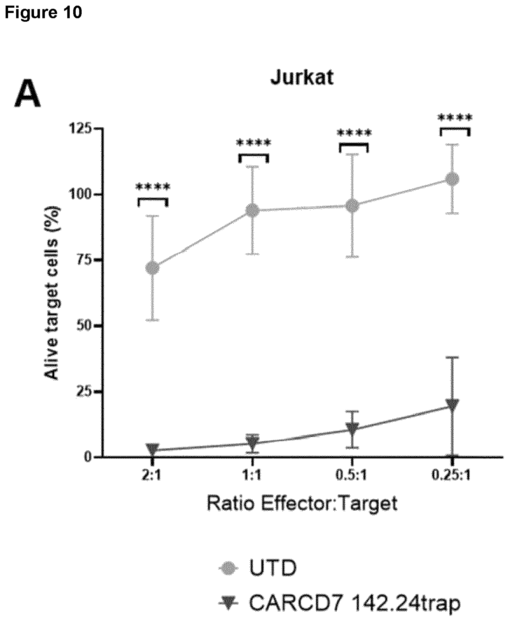

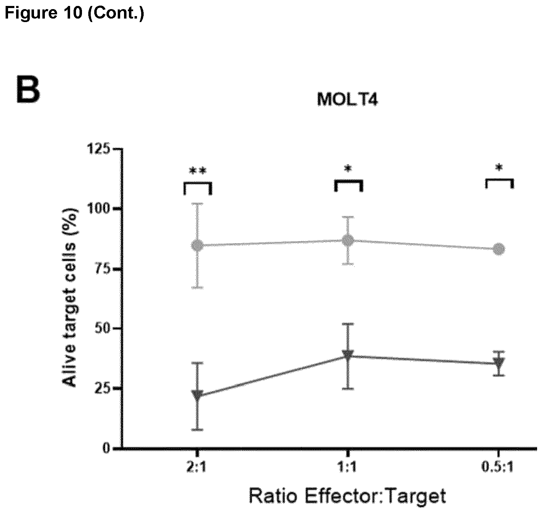

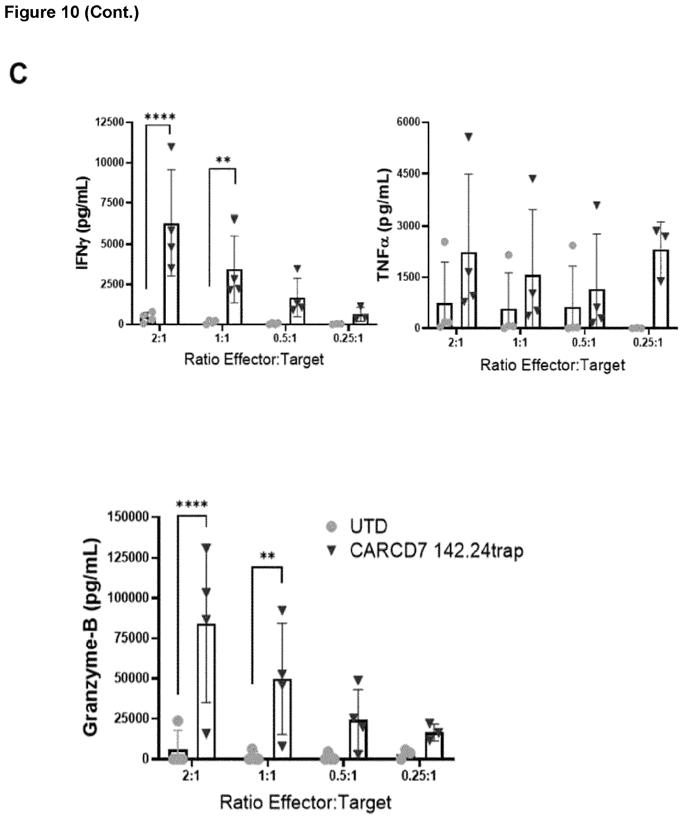

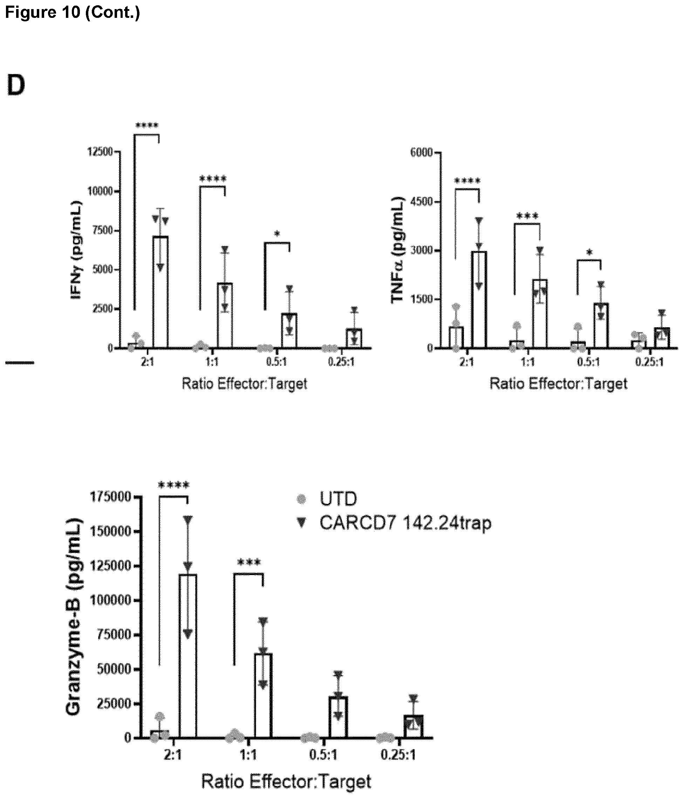

**Fig. 10****: CARCD7 142.24trap effector function *in vitro.*** CARCD7 142.24trap cells specifically kill CD7+ target (A) Jurkat cells and (B) MOLT4 cells. Indicated CAR-T cells and target cells were mixed at an E:T ratio of 2:1, 1:1 and 0.5:1 (Ratio 0.25:1 only in Jurkat). Cytotoxicity was evaluated 24 hours later by flow cytometry in a GFP-based assay. Mean of 4 and 3 normal donors ± SD in A and B, respectively. C-D) Cytokine production (IFNγ, TNFα, and Granzyme-B) of CARCD7 142.24trap cells after 24 hours co-culture with (C) Jurkat cells and (D) MOLT4 cells measured by ELISA. Mean of 4 and 3 experiments respectively ± SD is shown. Each dot represents a different normal donor. Mean ± SD. * indicates p< 0.05; ** indicates p<0.01; ***indicates p<0.001; **** indicates p<0.0001, two-way ANOVA.

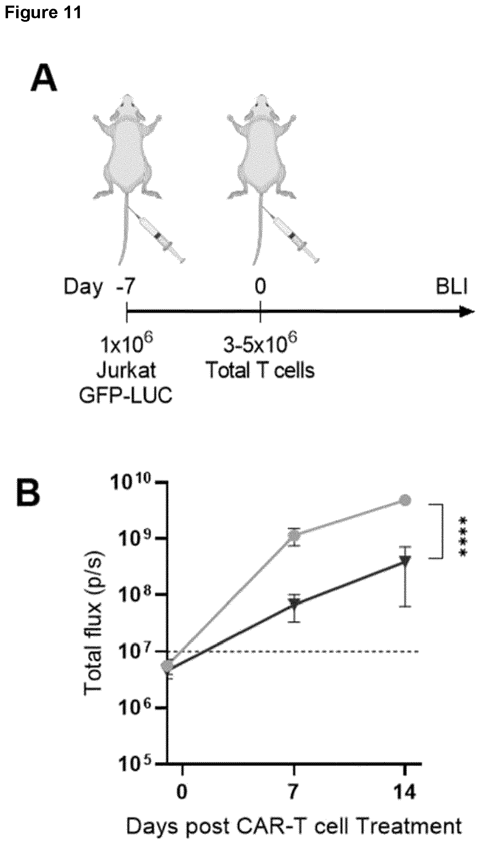

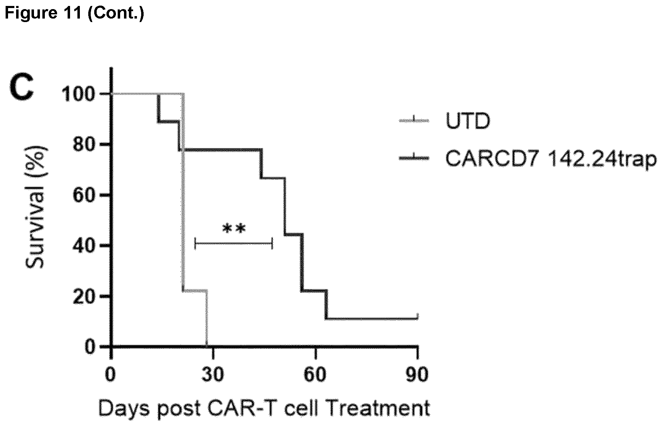

**Fig. 11****: CARCD7 142.24trap T cells control progression of systemic T-ALL in a mouse xenograft model.** A) Schematic procedure to generate a T-ALL mouse xenograft model. NSG mice were injected intravenously with 1×10⁶ GFP-LUC Jurkat cells. Seven days later, animals were treated with a single injection of 3-5×10⁶ of untransduced (UTD) or CARCD7 142.24trap T cells. Tumor growth was monitored weekly via bioluminescent imaging (BLI) using an IVIS Lumina. B) Overall kinetics of systemic tumor progression in mice during the first two weeks after treatment. All data are means +/- standard error of the mean (SEM). Two-way ANOVA of data at day 14. C) Kaplan-Meier survival curve (log-rank test) of mice treated with control or CARCD7 142.24trap T cells. Mixed data from two independent in vivo experiment (n=9 for each group). * indicates p< 0.05; ** indicates p<0.01; ***indicates p<0.001; **** indicates p<0.0001.

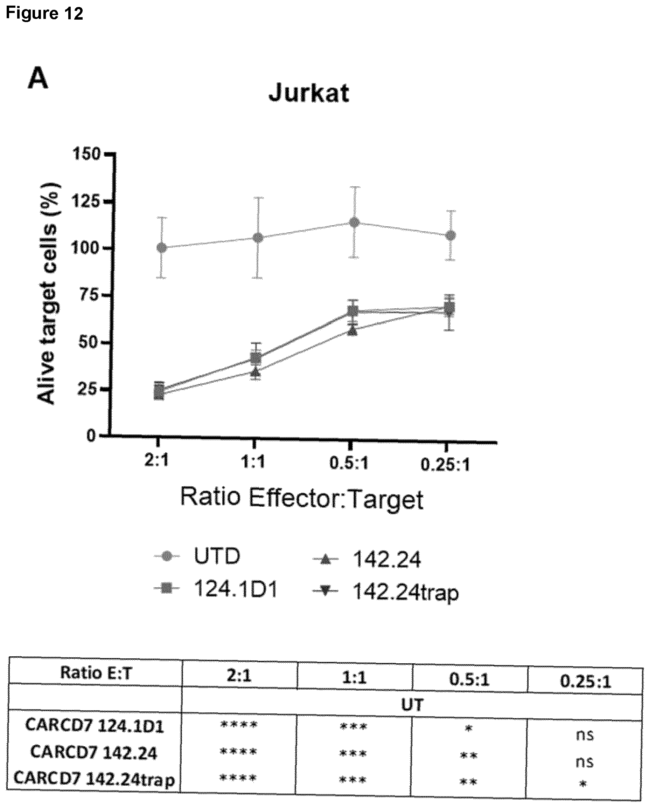

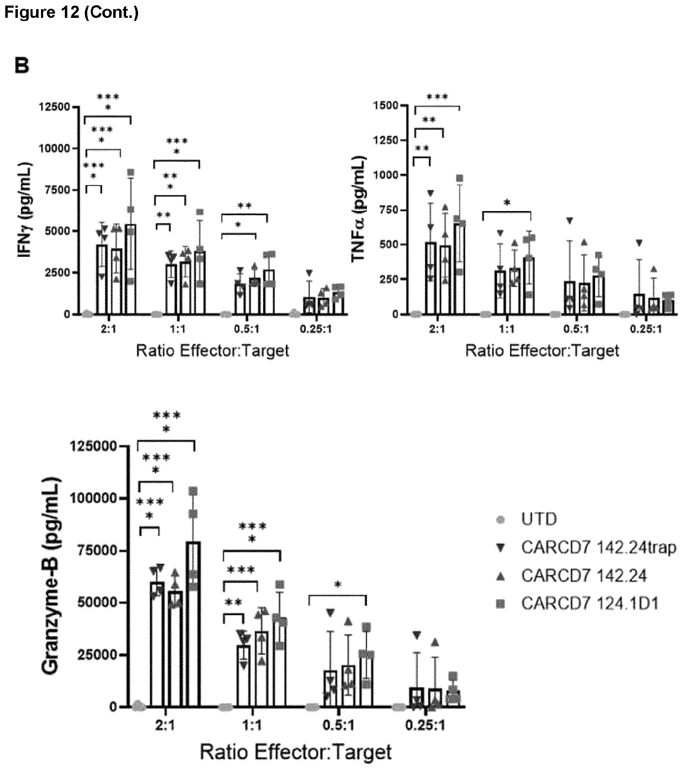

**Fig. 12****: *In vitro* functionality of CD7-negative T cells containing the CARCD7 124.1D1, 142.24 or 142.24trap**. A) CARCD7 124.1D1,142.24 and 142.24trap CD7negative-T cells specifically kill CD7+ target Jurkat cells. Indicated CAR-T cells and target cells were mixed at a fixed E:T ratio of 2:1, 1:1, 0.5:1 and 0.25:1, and cytotoxicity was evaluated 24 hours later by flow cytometry in a GFP-based assay. Mean of 3 healthy donors +/- SEM. B) Cytokine production (IFNγ, TNFα, and Granzyme-B) of CARCD7 124.1D1, 142.24 and 142.24trap CD7negative-T cells after 24 hours of coculture with Jurkat cells measured by ELISA. Mean of 4 healthy donors respectively ± SD is shown. Each dot represents a different normal donor. Ns indicates not significant * indicates p< 0.05; ** indicates p<0.01; ***indicates p<0.001; **** indicates p<0.0001. Two-way ANOVA.

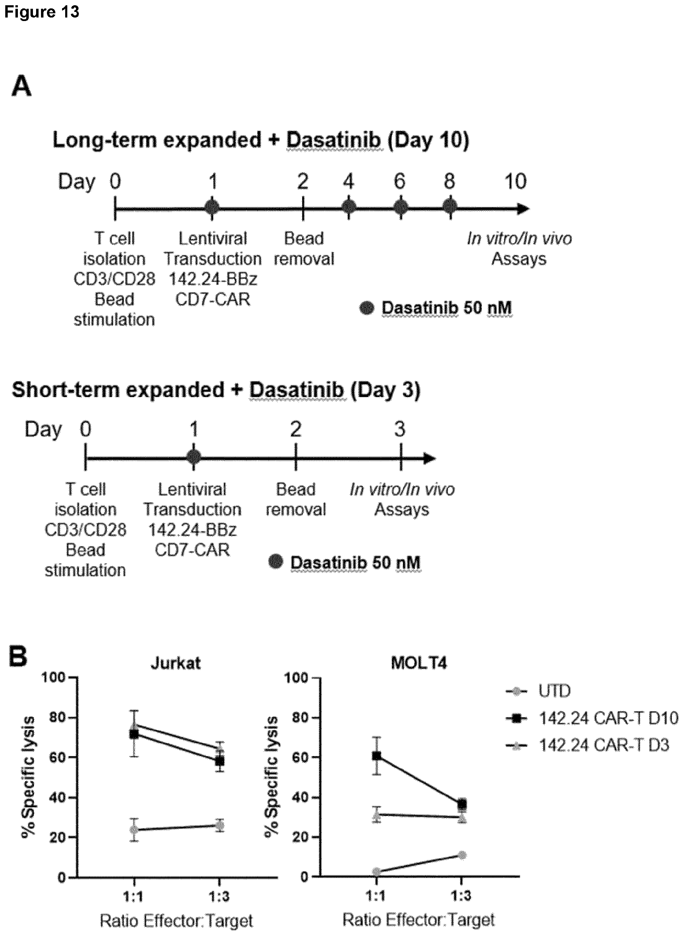

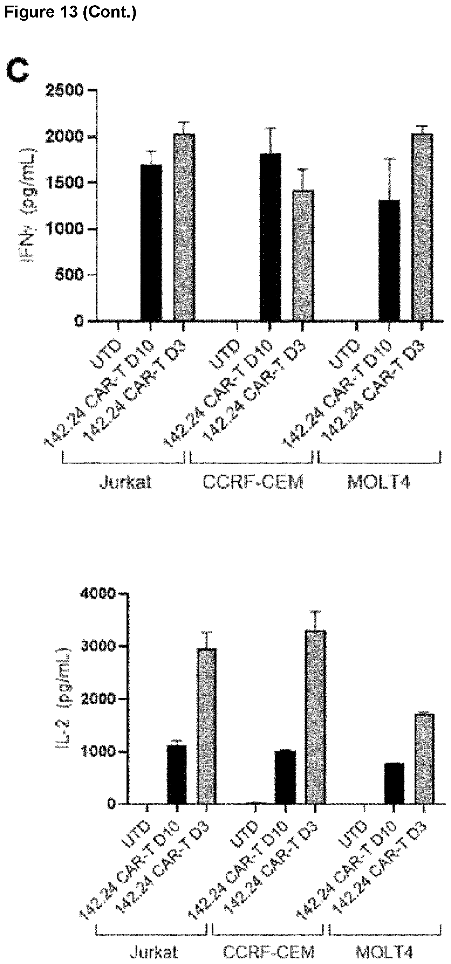

**Fig. 13****: Generation of anti-CD7 142.24-CAR T cells by non-gene editing strategies.** A) Protocol layout for CAR-T cell generation and expansion. Primary T cells were isolated from buffy coats of normal donors and activated by bead stimulation at day 0. Lentiviral transduction was done at day 1 and activation beads were removed at day 2 of expansion. CD7 CAR-T cells were expanded for 10 (D10) or 3 days (D3) and dasatinib was added to the media every 48 hours at 50 nM from day 1 until the end of the expansion. B) CD7 CAR-T cells specifically kill CD7+ Jurkat and MOLT4 cell lines. Indicated CAR-T cells and target cells were mixed at an E:T=1:1 or E:T=1:3 and cytotoxicity was evaluated 24 hours later in a luciferase-based assay. C) CD7 CAR-T cells groups secrete cytokines upon CD7+ target cells stimulation (Jurkat, CCRF-CEM, MOLT4). CAR-T cells were mixed with target cells at an E:T=3:1. IFNγ and IL-2 secretion was determined by ELISA after 24 hours of stimulation with tumor cells.

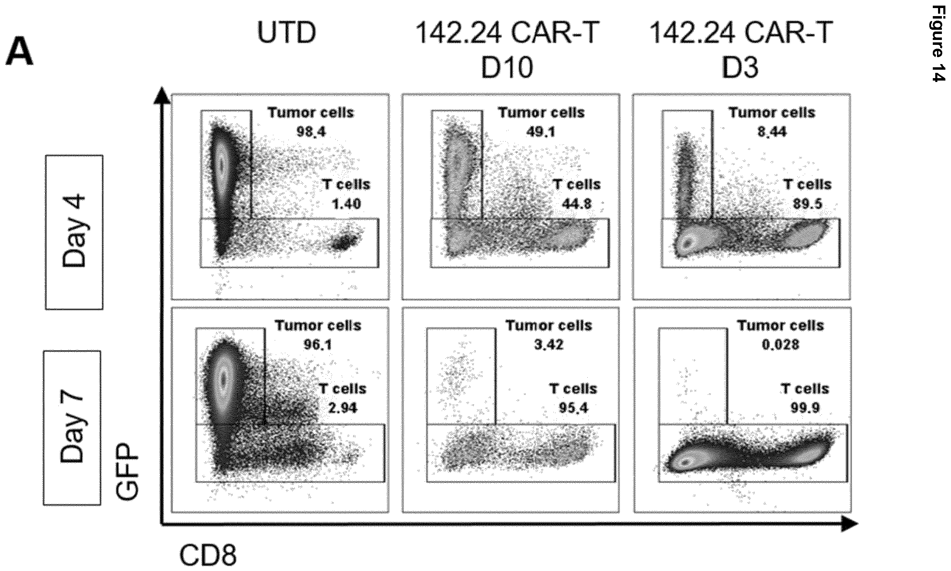

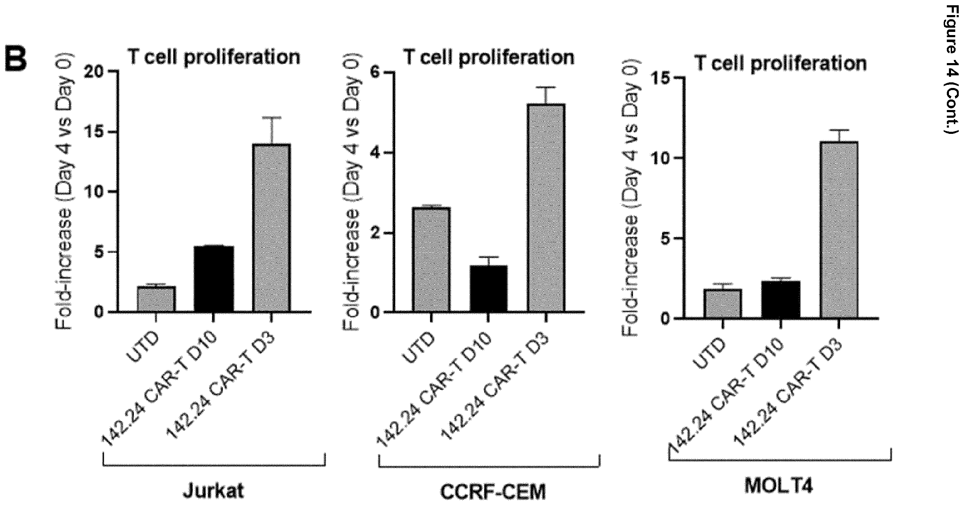

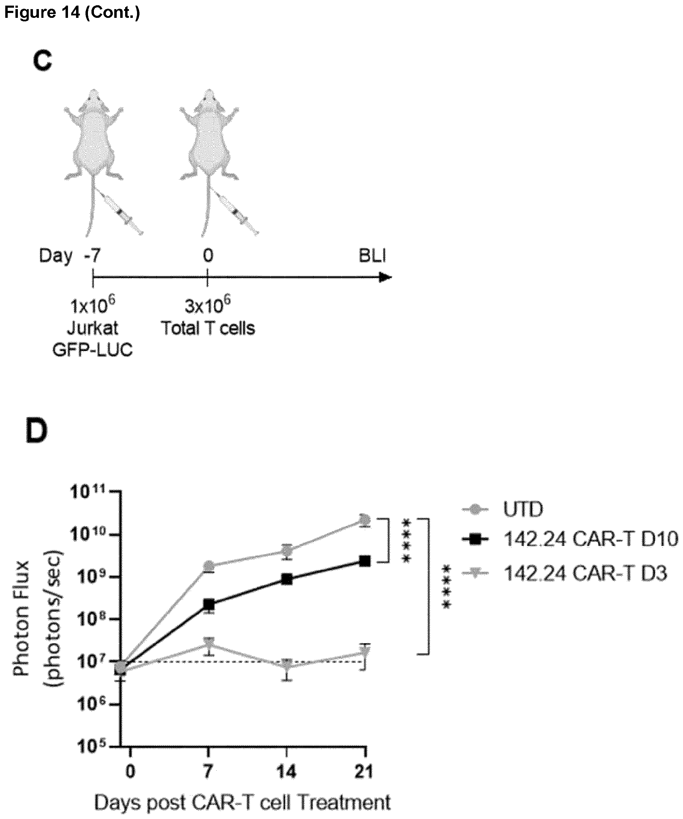

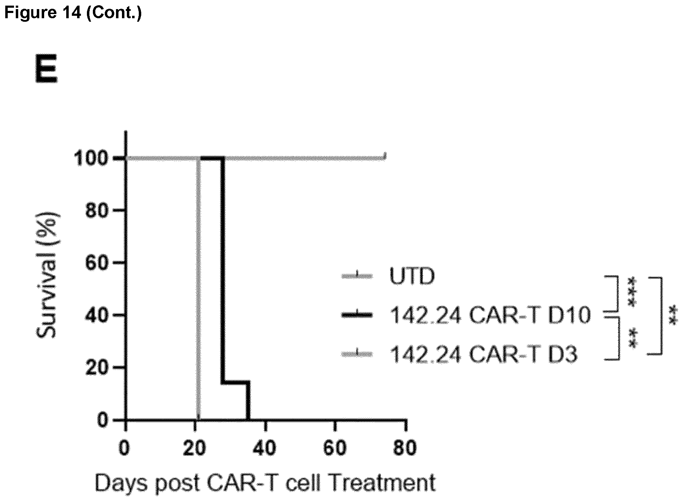

**Fig. 14****: Non-genetically modified anti-CD7 142.24-CAR T control progression of systemic T-ALL**. A-B) CD7CAR-T and CD7+ target cells were cocultured at a ratio of E:T=1:4. Absolute count of live cells was measured at day 4 and day 7 of coculture by flow cytometry using counting beads. A) Representative flow cytometry analysis showing the percentages of tumor and CAR-T cells at day 4 and day 7 of coculture. B) Quantification of the fold increase of CAR-T cells at day 4 of coculture with three CD7+ cell lines (Jurkat, CCRF-CEM, MOLT4). C) Schematic procedure to generate a T-ALL mouse xenograft model. NSG mice were injected intravenously with 1×10⁶ GFP-LUC Jurkat cells followed by a single intravenous injection of 3×10⁶ of control T-cells (untransduced, UTD) or CD7 CAR T-cells 7 days later. Tumor growth was monitored weekly via bioluminescent imaging (BLI) using an IVIS Spectrum. D) Overall kinetics of systemic tumor progression in mice during the first three weeks after treatment. All data are means +/- standard error of the mean (SEM). Two-way ANOVA of data at day 21. E) Kaplan-Meier survival curve (log-rank test) of mice treated with control or CD7 CAR-T cells; UTD (n=5), Long-term (D10) Dasa (n=8), Short-term (D3) Dasa (n=4).* indicates p< 0.05; ** indicates p<0.01; ***indicates p<0.001; **** indicates p<0.0001.

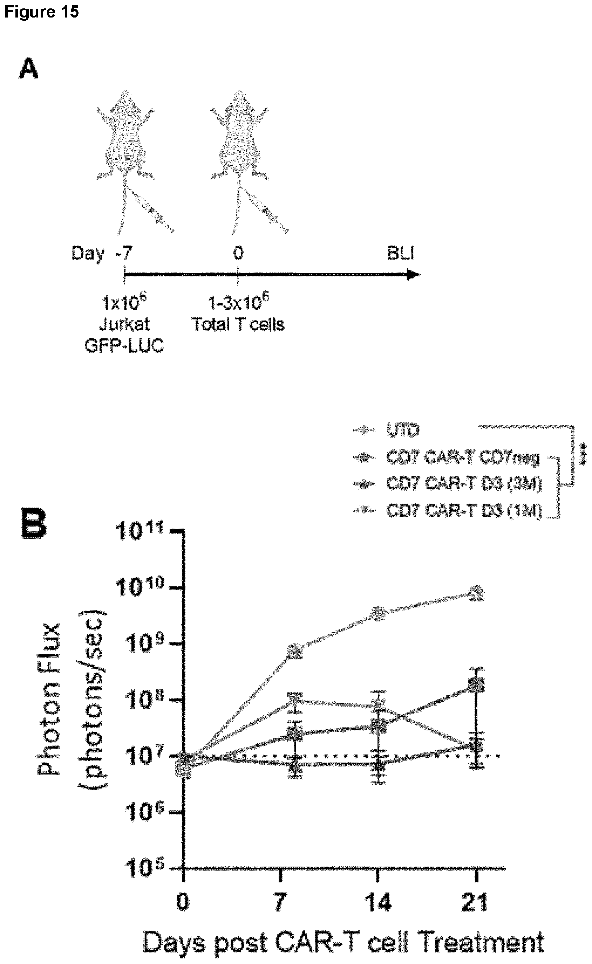

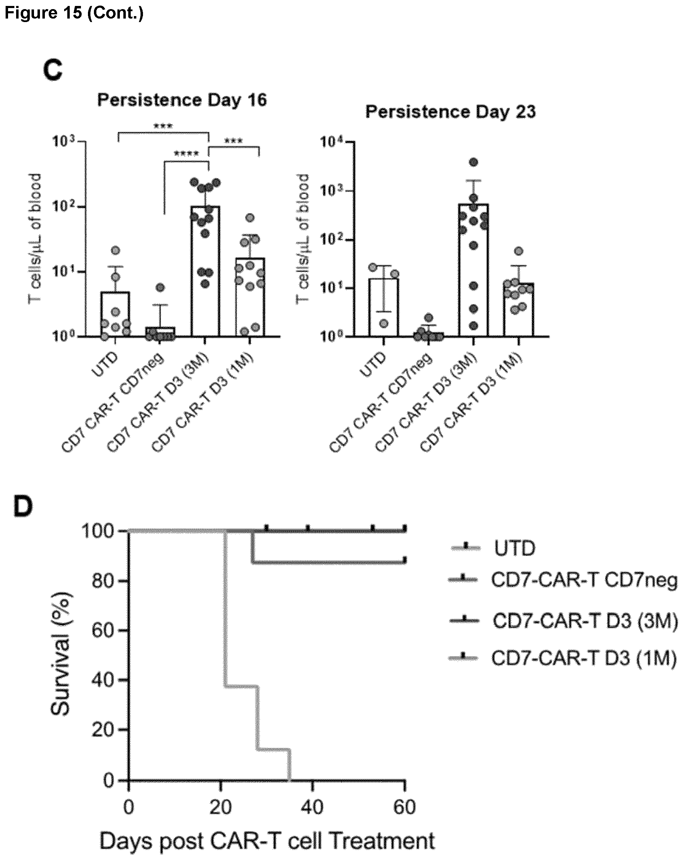

**Fig. 15****: Non-genetically modified anti-CD7 142.24 CAR-T cells are able to control progression of systemic T cell acute lymphoblastic leukemia (T-ALL) in a mouse xenograft model**. A) Schematic procedure to generate a T-ALL mouse xenograft model. NSG mice were injected intravenously with 1×10⁶ GFP-Luc Jurkat cells. Seven days later, animals were treated with a single intravenous injection of 3×10⁶ control T cells (untransduced, UTD), CD7negative anti-CD7 CAR-T cells, anti-CD7 TRAP CAR-T cells or short-term expanded anti-CD7 CAR-T cells (Day 3: D3) obtained from three different normal donors (n=2-5 animals per ND). A group of animals were also treated with a dose of 1×10⁶ anti-CD7 CAR-T D3 cells. Tumor growth was monitored weekly via bioluminescent imaging using an IVIS Spectrum. B) Overall kinetics of systemic tumor progression in mice during the first three weeks after treatment. All data are means +/standard error of the mean (SEM). Two-way ANOVA of data at day 21. C) Blood was collected by retro-orbital bleed on the indicated days and T cell persistence was analyzed. Absolute peripheral blood CD45+ T cell counts at the indicated timepoints after T cell injection measured using TruCount assay are plotted. Error bars represent ± SEM. One-way ANOVA. D) Kaplan-Meier survival curve (log-rank test) of mice treated with control or anti-CD7 CAR-T cells; UTD (n=8), CD7neg (n=8), TRAP (n=11), D3 (3M) (n=13) and D3 (1M) (n=10). * indicates p< 0.05; ** indicates p<0.01; ***indicates p<0.001; **** indicates p<0.0001.

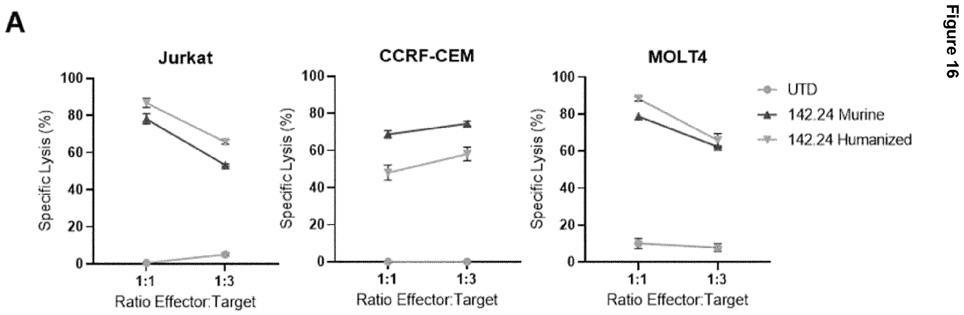

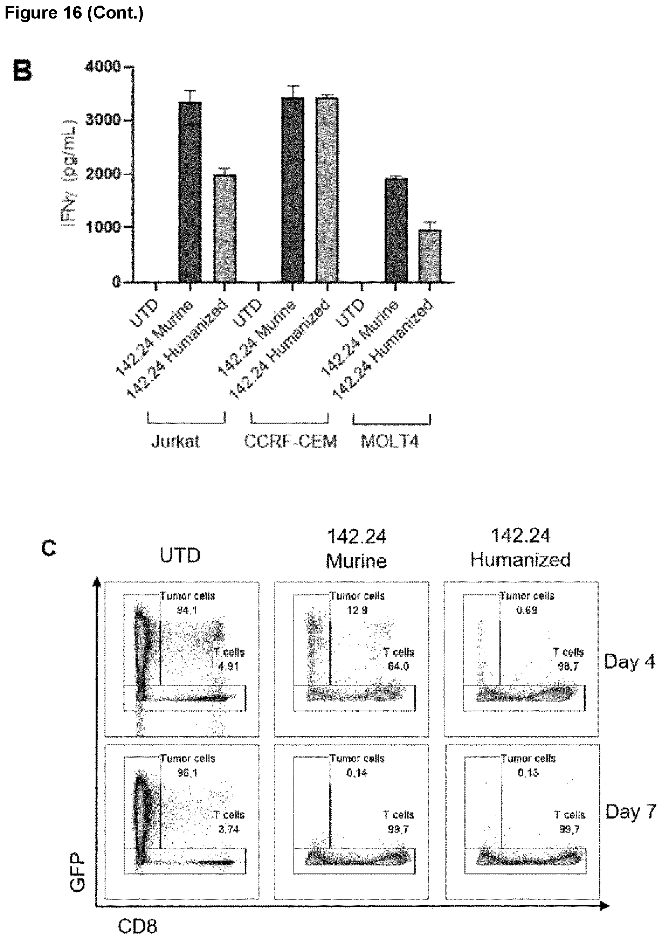

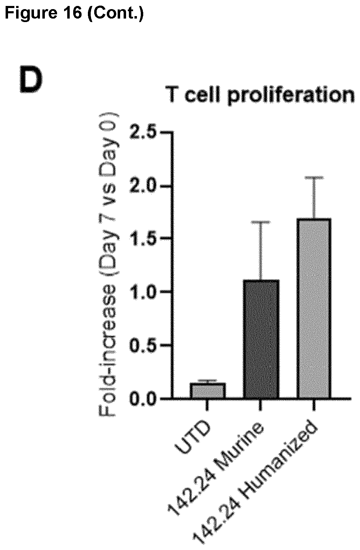

**Fig. 16****: Functional characterization of humanized 142.24 CAR-T cells *in vitro.*** A) Murine and humanized CD7KO anti-CD7 CAR-T cells specifically kill CD7+ target cells (Jurkat, CCRF-CEM and MOLT4). Indicated CAR-T cells and target cells were mixed at an effector:target ratio (E:T) of 1:1 and 1:3 and cytotoxicity was evaluated 24 hours later in a luciferase-based assay. B) The CD7KO anti-CD7 murine and humanized CAR-T cells secrete IFNγ upon coculture with CD7+ cells (Jurkat, CCRF-CEM and MOLT4). CAR-T cells were mixed with tumor cells at an E:T=3:1. IFNγ secretion was determined by ELISA after 24 hours of stimulation with target cells. C-D) Murine and humanized CAR-T and CD7+ Jurkat cells were cocultured at a E:T=1:2 for 4 or 7 days. Absolute count of live cells was measured by flow cytometry using counting beads at the end of coculture. C) Representative flow cytometry plots showing the percentages of tumor (GFP+) and T cells. D) Quantification of the fold increase of T cells at day 7 versus day 0. Data are means +/- standard deviation (SD).

### GENERAL DEFINITIONS

"Administering" or "administration of" a medicament to a patient (and grammatical equivalents of this phrase) refers to direct administration, which may be administration to a patient by a medical professional, and/or indirect administration, which may be the act of prescribing a drug. E.g., a physician who instructs a patient to self-administer a medicament or provides a patient with a prescription for a drug is administering the drug to the patient.

In the context of a protein sequence or polypeptide, a "domain" refers to a functional and/or structural unit or region in a protein or polypeptide. Domains are usually responsible for a particular function or interaction, contributing to the overall role of a protein. A domain may vary in length but is preferably between 25 amino acids to up to 500 amino acids long.

The term "affibody" refers to a protein that is derived from the Z domain of protein A and that has been engineered to bind to a specific target (see Frejd & Kim, 2017. Exp Mol Med. 49(3): e306).

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies.

By "capable of binding to human CD7" is meant that the CD7 targeting moiety has binding affinity for human CD7. Preferably, the binding affinity is specific, i.e., the CD7 is capable of specifically binding to the human CD7 (which is its target molecule) in a manner which distinguishes from the binding to non-target molecules, i.e., from other molecules that are not CD7. Thus, the CD7 targeting moiety either does not bind to non-target molecules or exhibits negligible or substantially-reduced (as compared to the target) e. g. background, binding to nontarget molecules. The CD7 targeting moiety specifically recognises CD7. Specific binding between the CD7 targeting moiety and the human CD7 can be assessed by numerous techniques, such as surface plasmon resonance, flow cytometry, or ELISA assays.

A typical type of antibody comprises at least two heavy chains ("HC") and two light chains ("LC") interconnected by disulfide bonds.

Each "heavy chain" comprises a "heavy chain variable domain" (abbreviated herein as "VH") and a "heavy chain constant domain" (abbreviated herein as "CH"). The heavy chain constant domain typically comprises three constant domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW"). In molecular biology, a framework region is a subdivision of the variable region (Fab) of the antibody. The variable region is composed of seven regions, four of which are framework regions and three of which are hypervariable regions. The framework region makes up about 85% of the variable region and are responsible for acting as a scaffold for the CDR regions. These CDRs are in direct contact with the antigen and are involved in binding antigen, while the framework regions support the binding of the CDR to the antigen and may aid in maintaining the overall structure of the four variable domains on the antibody.

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme).

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

The term "humanized antibody" refers to forms of antibodies that contain sequences from non-human (eg, murine) antibodies as well as human antibodies. A humanized antibody can include conservative amino acid substitutions or non-natural residues from the same or different species that do not significantly alter its binding and/or biologic activity. Such antibodies are chimeric antibodies that contain minimal sequence derived from non-human immunoglobulins. For the most part, humanized antibodies are human immunoglobulins (recipient antibody) in which residues from a complementary-determining region (CDR) of the recipient are replaced by residues from a CDR of a non-human species (donor antibody) such as mouse, rat, camel, bovine, goat, or rabbit having the desired properties. Furthermore, humanized antibodies can comprise residues that are found neither in the recipient antibody nor in the imported CDR or framework sequences. These modifications are made to further refine and maximize antibody performance. Thus, in general, a humanized antibody can comprise all or substantially all of at least one, and in one aspect two, variable domains, in which all or substantially all of the hypervariable loops correspond to those of a non-human immunoglobulin and all or substantially all of the FR regions are those of a human immunoglobulin sequence. The humanized antibody optionally also can comprise at least a portion of an immunoglobulin constant region (Fc), or that of a human immunoglobulin. The process to humanize an antibody comprises a CDR-grafting technique. This technique implies the use of a "donor" antibody containing the antigen-specific CDRs and the "acceptor" antibody that will serve as the framework for the hybrid molecule. Usually, the "donor" antibody is the non-human derived antibody, such as the murine antibody, and the "acceptor" antibody is the human antibody. As a result of the humanization process, the resulting humanized antibody or hybrid antibody maintains the CDR sequences, in both the VL and VH domains, of the "donor" antibody, whereas the framework sequences are changed for those of the "acceptor" antibody.

An antibody can be of any of the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target (see Skerra, 2008. FEBS J. 275(11):2677-83).

The term "antigen-binding fragment" or "Fab" refers to an antibody fragment comprising one constant and one variable domain of each of the heavy and light chain. A Fab fragment may be obtained by digesting an intact monoclonal antibody with papain.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

The term "CD7" or "cluster of differentiation 7" refers to the T-lymphocyte surface antigen CD7, which is a protein that in humans is encoded by the CD7 gene. This gene encodes a transmembrane protein which is a member of the immunoglobulin superfamily. This protein is found on thymocytes and mature T cells. It plays an essential role in T-cell interactions and also in T-cell/B-cell interaction during early lymphoid development. CD7 may be overexpressed in T cell malignancies such as T-cell acute lymphoblastic leukemia (T-ALL).

"CD7-positive" cancer, including a "CD7-positive" cancerous disease, is one comprising cells, which have CD7 present at their cell surface. The term "CD7-positive" also refers to a cancer that produces sufficient levels of CD7 at the surface of cells thereof, such that a CAR-comprising cell of the present invention has a therapeutic effect, mediated by the binding of the CAR to CD7. In some embodiments, the CD7-positive cancer is T cell acute lymphoblastic leukemia (T-ALL).

The term "CD7-targeting moiety" refers to a substance that is able to bind CD7, preferably human CD7. Within the context of a CAR, a CD7-targeting moiety redirects T cells towards a CD7-positive cell, preferably a cancer cell. Within the context of a CAR, it is to be understood that the CD7-targeting moiety is genetically encodable.

The term "chimeric antigen receptor" or "CAR" refers to a synthetic receptor that targets T cells to a chosen antigen and reprograms T cell function, metabolism and persistence. Similarly, the term "CART" refers to a T cell that comprises a CAR.

A "complete response" or "complete remission" or "CR" indicates the disappearance of all target lesions as defined in the RECIST v1.1 guideline. This does not always mean the cancer has been cured.

The term "costimulatory signaling domain" refers to a signaling moiety that provides to T cells a signal which, in addition to the primary signal provided by, for instance, the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, ICOS, lymphocyte function-associated antigen-1 (LFA-1), CD2, CD7, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats T-ALL, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The term "fynomer" refers to a protein that is derived from the SH3 domain of human Fyn kinase that has been engineered to bind to a specific target (see Bertschinger et al., 2007. Protein Eng Des Sel. 20(2):57-68).

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a CD7-positive cancer.

"Infusion" or "infusing" refers to the introduction of a therapeutic agent-containing solution into the body through a vein for therapeutic purposes. Generally, this is achieved via an intravenous bag.

"Intracellular signaling domain" as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen- independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRy, CD3γ, CD3δ, CD3ε, CD5, CD22, CD79a, CD79b, and CD66d.

The term "monobody" refers to a protein that is derived from a fibronectin type III domain that has been engineered to bind to a specific target (see Koide et al., 2013. J Mol Biol. 415(2): 393-405).

The term "nanobody" refers to a protein comprising the soluble single antigen-binding V-domain of a heavy chain antibody, preferably a camelid heavy chain antibody (see Bannas et al., 2017. Front Immunol. 8:1603).

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

The term "peptide aptamer" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline and it is published in European Journal of Cancers 45 (2009) 228-247.

The term "repebody" refers to a protein that is derived from a leucine-rich repeat module and that been engineered to bind to a specific target (see Lee et al., 2012. PNAS. 109(9): 3299-3304).

The term "respond favorably" generally refers to causing a beneficial state in a subject. With respect to cancer treatment, the term refers to providing a therapeutic effect on the subject. Positive therapeutic effects in cancer can be measured in a number of ways. For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

"Percent (%) sequence identity" with respect to the proteins or nucleic acids described herein is defined as the percentage of amino acid residues or nucleobases, in a candidate sequence that are identical with the amino acid residues or nucleobases, respectively, in the reference sequence (i.e., the protein or polypeptide or nucleic acid from which it is derived), after aligning the sequences and introducing gaps, if necessary, to achieve the maximum percent sequence identity, and not considering any conservative substitutions as part of the sequence identity. Alignment for purposes of determining the percentage sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared.

Preferably, the "percentage of sequence identity" as used herein is decided in the context of a local alignment, i.e., it is based on the alignment of regions of local similarity between sequences, contrary to a global alignment, which aims to align two sequences across their entire span. Thus, in the context of the present invention, percentage of sequence identity is calculated preferably based on the local alignment comparison algorithm.

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv).

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrollment to disease progression. TTP is generally measured using the RECIST v1.1 criteria.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term "fratricide" refers to the process in which a CAR-T cell becomes the target of, and is killed by, another CAR-T cell comprising the same chimeric antigen receptor as the targeted CAR-T cell because the targeted CAR- T cell expresses the antigen specifically recognized by the chimeric antigen receptor on both T cells.

### DESCRIPTION OF THE EMBODIMENTS

### CD7 targeting moieties of the invention

On the one hand, the present invention is based on the design of three ScFvs directed against CD7, a 40 kDa type I transmembrane glycoprotein which is the primary marker for T cell malignancies, and which is highly expressed in all cases of T-ALL. As described herein, the anti-CD7 CAR induces T cells to exert specific cytotoxicity against T cell malignancies. On the other hand, also provided herein is a combination therapy of anti-CD7 CAR T cells used in combination with strategies to prevent or reduce fratricide during CAR-T cell manufacturing. These strategies include the elimination or reduction of CD7 expression in the T cell membrane, the selection of CD7 negative T cells, the pharmacological inhibition of CAR-mediated T cell activation during the manufacturing of the CD7-CAR-T cell product or the use of a short-term expansion protocol. These strategies allow greater T cell recovery after CAR expression as compared to cells that retained the target antigen (e.g., CD7), and a more effective cytotoxicity against T leukemia/lymphoma cells.

In view of the above, a **first aspect** of the present invention relates to a CD7 targeting moiety, also called herein **CD7_124.1D1,** comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of [RASQSIGTSIH] (SEQ ID NO: 4), or a sequence with at least 85%, 86%, 87%, 88%, 89%, 90%, 91%, 92%, 93%, 94%, 95%, 96%, 97%, 98%, 99% or 100% sequence identity to SEQ ID NO: 4;
- LCDR2 comprises, consists, or consists essentially of [YASESIS] (SEQ ID NO: 5), or a sequence with at least 85%, 86%, 87%, 88%, 89%, 90%, 91%, 92%, 93%, 94%, 95%, 96%, 97%, 98%, 99% or 100% sequence identity to SEQ ID NO: 5;
- LCDR3 comprises, consists, or consists essentially of [QQSISWPLT] (SEQ ID NO: 6), or a sequence with at least 85%, 86%, 87%, 88%, 89%, 90%, 91%, 92%, 93%, 94%, 95%, 96%, 97%, 98%, 99% or 100% sequence identity to SEQ ID NO: 6;
- HCDR1 comprises, consists, or consists essentially of [SDYAWN] (SEQ ID NO: 7), or a sequence with at least 85%, 86%, 87%, 88%, 89%, 90%, 91%, 92%, 93%, 94%, 95%, 96%, 97%, 98%, 99% or 100% sequence identity to SEQ ID NO: 7;
- HCDR2 comprises, consists, or consists essentially of [YISYSGSTSYNPSLKS] (SEQ ID NO: 8), or a sequence with at least 85%, 86%, 87%, 88%, 89%, 90%, 91%, 92%, 93%, 94%, 95%, 96%, 97%, 98%, 99% or 100% sequence identity to SEQ ID NO: 8; and
- HCDR3 comprises, consists, or consists essentially of [SQLGRWAMDY] (SEQ ID NO: 9), or a sequence with at least 85%, 86%, 87%, 88%, 89%, 90%, 91%, 92%, 93%, 94%, 95%, 96%, 97%, 98%, 99% or 100% sequence identity to SEQ ID NO: 9.

In some embodiments of the first aspect, the CD7-targeting moiety is an antibody, anticalin, repebody, monobody, scFv, Fab, scFab, affibody, fynomer, DARPin, nanobody, or peptide aptamer that specifically binds to CD7.

In some embodiments of the first aspect, the CD7-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9.

Preferably, the CD7 targeting moiety of the first aspect is an antibody, F(ab')2, Fab, preferably scFv, or scFab comprising a VL domain and a VH domain, wherein the VL domain comprises, consists, or consists essentially of SEQ ID NO: 1, or a sequence with at least 85%, 86%, 87%, 88%, 89%, 90%, 91%, 92%, 93%, 94%, 95%, 96%, 97%, 98%, 99% or 100% sequence identity to SEQ ID NO: 1; and wherein the VH domain comprises, consists, or consists essentially of SEQ ID NO: 2 , or a sequence with at least 85%, 86%, 87%, 88%, 89%, 90%, 91%, 92%, 93%, 94%, 95%, 96%, 97%, 98%, 99% or 100% sequence identity to SEQ ID NO: 2. In some embodiments, the CD7-targeting moiety of the first aspect is an antibody, scFv, Fab, or scFab comprising a VL domain and VH domain, wherein the VL domain comprises SEQ ID NO: 1 and the VH domain comprises SEQ ID NO: 2. In some embodiments, the CD7-targeting moiety of the first aspect is an antibody, scFv, Fab, or scFab comprising a VL domain and VH domain, wherein the VL domain consists of SEQ ID NO: 1 and the VH domain consists of SEQ ID NO: 2.

The VL and VH of the CD7 targeting moiety described herein in the first aspect, or any of its embodiments, may be humanized. In the humanization process, the framework regions of the variable region of a targeting moiety may be modified, whereas the CDRs are maintained constant. By "maintained constant" it is referred that, while the framework regions of the CD7 targeting moiety can vary (for instance, they can be different depending on whether it is a murine ScFv or a humanized ScFv), the sequences belonging to the CDRs are not changed or are invariable.

In the context of the present invention and in the context of the embodiments that refer to a percentage of identity over a sequence (e.g. VH or VL sequence) where some regions of said sequences (e.g., the CDRs) are maintained constant, the percentage of identity is obtained by aligning the sequences (e.g. VH or VL sequence), preferably using BLAST tool (or a similar local alignment algorithm) and, once the percentage of identity is obtained, it is checked (by, e.g. by visual inspection) whether the CDRs are maintained constant between both sequences. In other words, both sequences are aligned and only the mismatches in the framework regions, and not in the CDRs, are considered to obtain the % of sequence identity between both sequences.

Therefore, in some embodiments of the first aspect, the CD7 targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 1 or 54, preferably SEQ ID NO: 1, wherein the CDRs comprised in said VL are maintained constant (i.e., non variable) and consists of SEQ ID NOs: 4, 5, and 6; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 2 or 55, preferably SEQ ID NO: 2, wherein the CDRs comprised in said VH are maintained constant and consist of SEQ ID NOs: 7, 8, and 9. Preferably, the CD7 targeting moiety is scFv comprising a VL and a VH domains, wherein the VL domain comprises a sequence having at least 85% sequence identity to SEQ ID NO: 1 or 54, preferably SEQ ID NO: 1, wherein the CDRs comprised in said VL consists of SEQ ID NOs: 4, 5, and 6; and wherein the VH domain comprises a sequence having at least 85% sequence identity to SEQ ID NO: 2 or 55, preferably SEQ ID NO: 2, wherein the CDRs comprised in said VH consist of SEQ ID NOs: 7, 8, and 9.

Thus, in an embodiment of the first aspect, the CD7 targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 1 or 54, preferably SEQ ID NO: 1, and wherein said VL domain comprises CDR regions consisting of LCDR1 as set forth in SEQ ID NO: 4, LCDR2 as set forth in SEQ ID NO: 5, LCDR3 as set forth in SEQ ID NO: 6, and
- said VH domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 2 or 55, preferably SEQ ID NO: 2, and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9 and
preferably, wherein the CD7 targeting moiety is capable of binding to human CD7, preferably as measured by surface plasmon resonance (SPR).

In an embodiment of the first aspect, the CD7 targeting moiety is an antibody, F(ab')2, Fab, preferably scFv, or scFab, and is capable of binding to human CD7 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 1 or 54, preferably SEQ ID NO: 1, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 2 or 55, preferably SEQ ID NO: 1, and
wherein the LCDR1, 2, and 3 comprised in the VL domain consist of SEQ ID NO: 4, 5 and 6, respectively, and wherein the HCDR1, 2 and 3 comprised in said VH domain consists of SEQ ID NO: 7, 8 and 9, respectively.

Preferably, the CD7 targeting moiety of the first aspect is an antibody, F(ab')2, Fab, preferably scFv, or scFab comprising a VL domain and a VH domain, wherein the VL domain comprises, consists, or consists essentially of SEQ ID NO: 54, or a sequence with at least 85%, 86%, 87%, 88%, 89%, 90%, 91%, 92%, 93%, 94%, 95%, 96%, 97%, 98%, 99% or 100% sequence identity to SEQ ID NO: 54; and wherein the VH domain comprises, consists, or consists essentially of SEQ ID NO: 55, or a sequence with at least 85%, 86%, 87%, 88%, 89%, 90%, 91%, 92%, 93%, 94%, 95%, 96%, 97%, 98%, 99% or 100% sequence identity to SEQ ID NO: 55. In some embodiments, the CD7-targeting moiety of the first aspect is an antibody, scFv, Fab, or scFab comprising a VL domain and VH domain, wherein the VL domain comprises SEQ ID NO: 54 and the VH domain comprises SEQ ID NO: 55. In some embodiments, the CD7-targeting moiety of the first aspect is an antibody, scFv, Fab, or scFab comprising a VL domain and VH domain, wherein the VL domain consists of SEQ ID NO: 54 and the VH domain consists of SEQ ID NO: 55.

Preferably, the CD7 targeting moiety of the first aspect comprises, consists, or consists essentially of SEQ ID NO: 3 or 60, preferably SEQ ID NO: 3, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 3 or 60, preferably SEQ ID NO: 3. Most preferably, the CD7 targeting moiety comprises SEQ ID NO: 3 or 60, preferably SEQ ID NO: 3. Most preferably, the CD7 targeting moiety consists of SEQ ID NO: 3 or 60, preferably SEQ ID NO: 3.

In a **second aspect,** the present invention relates to a CD7 targeting moiety, also called herein **CD7_142.24,** comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of [RASQNIGTSIH] (SEQ ID NO: 14), or a sequence with at least 85%, 86%, 87%, 88%, 89%, 90%, 91%, 92%, 93%, 94%, 95%, 96%, 97%, 98%, 99% or 100% sequence identity to SEQ ID NO: 14;
- LCDR2 comprises, consists, or consists essentially of [YASESMS] (SEQ ID NO: 15), or a sequence with at least 85%, 86%, 87%, 88%, 89%, 90%, 91%, 92%, 93%, 94%, 95%, 96%, 97%, 98%, 99% or 100% sequence identity to SEQ ID NO: 15;
- LCDR3 comprises, consists, or consists essentially of [QQSNRWPYT] (SEQ ID NO: 16), or a sequence with at least 85%, 86%, 87%, 88%, 89%, 90%, 91%, 92%, 93%, 94%, 95%, 96%, 97%, 98%, 99% or 100% sequence identity to SEQ ID NO: 16;
- HCDR1 comprises, consists, or consists essentially of [HYGVN] (SEQ ID NO: 17), or a sequence with at least 85%, 86%, 87%, 88%, 89%, 90%, 91%, 92%, 93%, 94%, 95%, 96%, 97%, 98%, 99% or 100% sequence identity to SEQ ID NO: 17;
- HCDR2 comprises, consists, or consists essentially of [WINTNTGKPTYAEDFKG] (SEQ ID NO: 18), or a sequence with at least 85%, 86%, 87%, 88%, 89%, 90%, 91%, 92%, 93%, 94%, 95%, 96%, 97%, 98%, 99% or 100% sequence identity to SEQ ID NO: 18; and
- HCDR3 comprises, consists, or consists essentially of [WSTTVVAPYFDV] (SEQ ID NO: 19), or a sequence with at least 85%, 86%, 87%, 88%, 89%, 90%, 91%, 92%, 93%, 94%, 95%, 96%, 97%, 98%, 99% or 100% sequence identity to SEQ ID NO: 19.

In some embodiments of the second aspect, the CD7-targeting moiety is an antibody, anticalin, repebody, monobody, scFv, Fab, scFab, affibody, fynomer, DARPin, nanobody, or peptide aptamer that specifically binds to CD7.

In some embodiments of the second aspect, the CD7-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19.

Preferably, the CD7 targeting moiety of the second aspect is an antibody, F(ab')2, Fab, preferably scFv, or scFab comprising a VL domain and a VH domain, wherein the VL domain comprises, consists, or consists essentially of SEQ ID NO: 20, or a sequence with at least 80%, 81%, 82%, 83%, 84%, 85%, 86%, 87%, 88%, 89%,, 90%, 91%, 92%, 93%, 94%, 95%, 96%, 97%, 98%, 99% or 100% sequence identity to SEQ ID NO: 20; and wherein the VH domain comprises, consists, or consists essentially of SEQ ID NO: 21, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 21. In some embodiments, the CD7-targeting moiety of the second aspect is an antibody, scFv, Fab, or scFab comprising a VL domain and VH domain, wherein the VL domain comprises SEQ ID NO: 20 and the VH domain comprises SEQ ID NO: 21. In some embodiments, the CD7-targeting moiety of the second aspect is an antibody, scFv, Fab, or scFab comprising a VL domain and VH domain, wherein the VL domain consists of SEQ ID NO: 20 and the VH domain consists of SEQ ID NO: 21.

The VL and VH of the CD7 targeting moiety described herein in the second aspect, or any of its embodiments, may be humanized. In the humanization process, the framework regions of the variable region of a targeting moiety may be modified, whereas the CDRs are maintained constant or unchanged. Therefore, in some embodiments of the second aspect, the CD7 targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 20 or 56, preferably SEQ ID NO: 20, wherein the CDRs comprised in said VL are maintained constant and consist of SEQ ID NOs: 14, 15, and 16; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 21 or 57, preferably SEQ ID NO: 21, wherein the CDRs comprised in said VH are maintained constant and consist of SEQ ID NOs: 17, 18, and 19. Preferably, the CD7 targeting moiety is scFv comprising a VL and a VH domains, wherein the VL domain comprises a sequence having at least 85% sequence identity to SEQ ID NO: 20 or 56, preferably SEQ ID NO: 20, wherein the CDRs comprised in said VL consists of SEQ ID NOs: 14, 15, and 16; and wherein the VH domain comprises a sequence having at least 85% sequence identity to SEQ ID NO: 21 or 57, preferably SEQ ID NO: 21, wherein the CDRs comprised in said VH consist of SEQ ID NOs: 17, 18, and 19.

Thus, in an embodiment of the second aspect, the CD7 targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%,, 90%, 91%, 92%, 93%, 94%, 95%, 96%, 97%, 98%, or 99% sequence identity to SEQ ID NO: 20 or 56, preferably SEQ ID NO: 20, and wherein said VL domain comprises CDR regions consisting of LCDR1 as set forth in SEQ ID NO: 14, LCDR2 as set forth in SEQ ID NO: 15, LCDR3 as set forth in SEQ ID NO: 16, and
- said VH domain comprises a sequence with at least 80%, 81%, 82%, 83%, 84%, 85%, 86%, 87%, 88%, 89%,, 90%, 91%, 92%, 93%, 94%, 95%, 96%, 97%, 98%, or 99% sequence identity to SEQ ID NO: 21 or 57, preferably SEQ ID NO: 21, and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19 and
preferably, wherein the CD7 targeting moiety is capable of binding to human CD7, preferably as measured by surface plasmon resonance (SPR).

In an embodiment of the second aspect, the CD7 targeting moiety is an antibody, F(ab')2, Fab, preferably scFv, or scFab, and is capable of binding to human CD7 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 20 or 56, preferably SEQ ID NO: 20, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 21 or 57, preferably SEQ ID NO: 21, and
wherein the LCDR1, 2, and 3 comprised in the VL domain consist of SEQ ID NO: 14, 15 and 16, respectively, and wherein the HCDR1, 2 and 3 comprised in said VH domain consists of SEQ ID NO: 17, 18 and 19, respectively.

Thus, preferably, the CD7 targeting moiety of the second aspect is an antibody, F(ab')2, Fab, preferably scFv, or scFab comprising a VL domain and a VH domain, wherein the VL domain comprises, consists, or consists essentially of SEQ ID NO: 56, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 56; and wherein the VH domain comprises, consists, or consists essentially of SEQ ID NO: 57 , or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 57. In some embodiments, the CD7-targeting moiety of the first aspect is an antibody, scFv, Fab, or scFab comprising a VL domain and VH domain, wherein the VL domain comprises SEQ ID NO: 56 and the VH domain comprises SEQ ID NO: 57.In some embodiments, the CD7-targeting moiety of the first aspect is an antibody, scFv, Fab, or scFab comprising a VL domain and VH domain, wherein the VL domain consists of SEQ ID NO: 56 and the VH domain consists of SEQ ID NO: 57.

Preferably, the CD7 targeting moiety of the second aspect comprises, consists, or consists essentially of SEQ ID NO: 11 or 61, preferably SEQ ID NO: 11, or a sequence that has at least 80%, 81%, 82%, 83%, 84%, 85%, 86%, 87%, 88%, 89%,, 90%, 91%, 92%, 93%, 94%, 95%, 96%, 97%, 98%, 99% or 100% sequence identity to SEQ ID NO: 11 or 61, preferably SEQ ID NO: 11. Most preferably, the CD7 targeting moiety comprises SEQ ID NO: 11 or 61, preferably SEQ ID NO: 11. Most preferably, the CD7 targeting moiety consists of SEQ ID NO: 11 or 61, preferably SEQ ID NO: 11.

In a **third aspect,** the present invention relates to a CD7 targeting moiety, also called herein **CD7_142.9,** comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially SEQ ID NO: 42, or a sequence with at least 85%, 86%, 87%, 88%, 89%, 90%, 91%, 92%, 93%, 94%, 95%, 96%, 97%, 98%, 99% or 100% sequence identity to SEQ ID NO: 42;
- LCDR2 comprises, consists, or consists essentially of SEQ ID NO: 43, or a sequence with at least 85%, 86%, 87%, 88%, 89%, 90%, 91%, 92%, 93%, 94%, 95%, 96%, 97%, 98%, 99% or 100% sequence identity to SEQ ID NO: 43;
- LCDR3 comprises, consists, or consists essentially of SEQ ID NO: 44, or a sequence with at least 85%, 86%, 87%, 88%, 89%, 90%, 91%, 92%, 93%, 94%, 95%, 96%, 97%, 98%, 99% or 100% sequence identity to SEQ ID NO: 44;
- HCDR1 comprises, consists, or consists essentially of SEQ ID NO: 45, or a sequence with at least 85%, 86%, 87%, 88%, 89%, 90%, 91%, 92%, 93%, 94%, 95%, 96%, 97%, 98%, 99% or 100% sequence identity to SEQ ID NO: 45;
- HCDR2 comprises, consists, or consists essentially of SEQ ID NO: 46, or a sequence with at least 85%, 86%, 87%, 88%, 89%, 90%, 91%, 92%, 93%, 94%, 95%, 96%, 97%, 98%, 99% or 100% sequence identity to SEQ ID NO: 46; and
- HCDR3 comprises, consists, or consists essentially of SEQ ID NO: 47, or a sequence with at least 85%, 86%, 87%, 88%, 89%, 90%, 91%, 92%, 93%, 94%, 95%, 96%, 97%, 98%, 99% or 100% sequence identity to SEQ ID NO: 47.

In some embodiments of the second aspect, the CD7-targeting moiety is an antibody, anticalin, repebody, monobody, scFv, Fab, scFab, affibody, fynomer, DARPin, nanobody, or peptide aptamer that specifically binds to CD7.

In some embodiments of the second aspect, the CD7-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 42, LCDR2 consists of SEQ ID NO: 43, LCDR3 consists of SEQ ID NO: 44, HCDR1 consists of SEQ ID NO: 45, HCDR2 consists of SEQ ID NO: 46, and HCDR3 consists of SEQ ID NO: 47.

Preferably, the CD7 targeting moiety of the second aspect is an antibody, F(ab')2, Fab, preferably scFv, or scFab comprising a VL domain and a VH domain, wherein the VL domain comprises, consists, or consists essentially of SEQ ID NO: 48, or a sequence with at least 80%, 81%, 82%, 83%, 84%, 85%, 86%, 87%, 88%, 89%, , 90%, 91%, 92%, 93%, 94%, 95%, 96%, 97%, 98%, 99% or 100% sequence identity to SEQ ID NO: 48; and wherein the VH domain comprises, consists, or consists essentially of SEQ ID NO: 50, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 50. In some embodiments, the CD7-targeting moiety of the second aspect is an antibody, scFv, Fab, or scFab comprising a VL domain and VH domain, wherein the VL domain comprises SEQ ID NO: 48 and the VH domain comprises SEQ ID NO: 50. In some embodiments, the CD7-targeting moiety of the second aspect is an antibody, scFv, Fab, or scFab comprising a VL domain and VH domain, wherein the VL domain consists of SEQ ID NO: 48 and the VH domain consists of SEQ ID NO: 50.

The VL and VH of the CD7 targeting moiety described herein in the third aspect, or any of its embodiments, may be humanized as explained above. Therefore, in some embodiments of the third aspect, the CD7 targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 48 or 58, preferably SEQ ID NO: 48, wherein the CDRs comprised in said VL are maintained constant and consist of SEQ ID NOs: 42, 43, and 44; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 50 or 59, preferably SEQ ID NO: 50, wherein the CDRs comprised in said VH are maintained constant and consist of SEQ ID NOs: 45, 46, and 47. Preferably, the CD7 targeting moiety is an scFv comprising a VL and a VH domains, wherein the VL domain comprises a sequence having at least 85% sequence identity to SEQ ID NO: 48 or 58, preferably SEQ ID NO: 48, wherein the CDRs comprised in said VL consist of SEQ ID NOs: 42, 43, and 44; and wherein the VH domain comprises a sequence having at least 85% sequence identity to SEQ ID NO: 50 or 59, preferably SEQ ID NO: 50, wherein the CDRs comprised in said VH consist of SEQ ID NOs: 45, 46, and 47.

Thus, in an embodiment of the third aspect, the CD7 targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 48 or 58, preferably SEQ ID NO: 48, and wherein said VL domain comprises CDR regions consisting of LCDR1 as set forth in SEQ ID NO: 42, LCDR2 as set forth in SEQ ID NO: 43, LCDR3 as set forth in SEQ ID NO: 44, and
- said VH domain comprises a sequence with at least 80%, 81%, 82%, 83%, 84%, 85%, 86%, 87%, 88%, 89%,, 90%, 91%, 92%, 93%, 94%, 95%, 96%, 97%, 98%, or 99% sequence identity to SEQ ID NO: 50 or 59, preferably SEQ ID NO: 50, and wherein said VH domain comprises CDR regions consisting of HCDR1 as set forth in SEQ ID NO: 45, HCDR2 as set forth in SEQ ID NO: 46, and HCDR3 as set forth in SEQ ID NO: 47 and
preferably, wherein the CD7 targeting moiety is capable of binding to human CD7, preferably as measured by surface plasmon resonance (SPR).

In an embodiment of the third aspect, the CD7 targeting moiety is an antibody, F(ab')2, Fab, preferably scFv, or scFab, and is capable of binding to human CD7 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 48 or 58, preferably SEQ ID NO: 48, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 50 or 59, preferably SEQ ID NO: 50, and
wherein the LCDR1, 2, and 3 comprised in the VL domain consist of SEQ ID NO: 42, 43 and 44, respectively, and wherein the HCDR1, 2 and 3 comprised in said VH domain consists of SEQ ID NO: 45, 46 and 47, respectively.

Thus, preferably, the CD7 targeting moiety of the third aspect is an antibody, F(ab')2, Fab, preferably scFv, or scFab comprising a VL domain and a VH domain, wherein the VL domain comprises, consists, or consists essentially of SEQ ID NO: 58, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 58; and wherein the VH domain comprises, consists, or consists essentially of SEQ ID NO: 59, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 59. In some embodiments, the CD7-targeting moiety of the first aspect is an antibody, scFv, Fab, or scFab comprising a VL domain and VH domain, wherein the VL domain comprises SEQ ID NO: 58 and the VH domain comprises SEQ ID NO: 59. In some embodiments, the CD7-targeting moiety of the first aspect is an antibody, scFv, Fab, or scFab comprising a VL domain and VH domain, wherein the VL domain consists of SEQ ID NO: 58 and the VH domain consists of SEQ ID NO: 59.

Preferably, the CD7 targeting moiety of the third aspect comprises, consists, or consists essentially of SEQ ID NO: 52 or 62, preferably SEQ ID NO: 52, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 52 or 62, preferably SEQ ID NO: 52. Preferably, the CD7 targeting moiety comprises SEQ ID NO: 52 or 62, preferably SEQ ID NO: 52. Most preferably, the CD7 targeting moiety consists of SEQ ID NO: 52 or 62, preferably SEQ ID NO: 52.

In a preferred embodiment of the first, second and third aspects, the VL and the VH domains comprised in the CD7-targeting moieties are linked by a peptide linker. In some embodiments, the linker comprises at least 5 amino acids, preferably between 5 and 25, preferably between 10-20 amino acids, most preferably 20 amino acids. Preferably, the amino acid is G and/or S. Preferably, the peptide linker comprises, consists or consists essentially of SEQ ID NO: 10, or a sequence that has at least 85%, 86%, 87%, 88%, 89%, 90%, 91%, 92%, 93%, 94%, 95%, 96%, 97%, 98%, 99% or 100% sequence identity to SEQ ID NO: 10.

In a preferred embodiment of the first, second and third aspects, the CD7-targeting moieties further comprises a signal peptide that is placed preferably at the N-terminal region of the CD7-targeting moiety. Preferably, the signal peptide comprises, consists or consists essentially of SEQ ID NO: 27, or a sequence that has at least 85%, 86%, 87%, 88%, 89%, 90%, 91%, 92%, 93%, 94%, 95%, 96%, 97%, 98%, 99% or 100% sequence identity to SEQ ID NO: 27.

In an embodiment, the CD7-targeting moieties of the first or second aspects, or any of their embodiments, is comprised in a Chimeric antigen receptor (CAR) anti-CD7. In an embodiment, the CD7-targeting moieties of the first, second or third aspects, or any of their embodiments, is comprised in a CD7-Trap protein. Each of these constructs are further defined below:

### CAR anti-CD7 of the invention

The CD7 targeting moieties as defined above in both the first or second aspects, or any of their embodiments, can be part of a chimeric antigen receptor (CAR), also called herein the chimeric antigen receptor (CAR) anti-CD7 or the present invention. Thus, in a preferred embodiment of the first and second aspects, the present invention provides a CAR comprising:
a) an extracellular domain comprising a CD7 targeting moiety as defined in the first or second aspects or in any of the embodiments disclosed above,
b) a transmembrane domain; and
c) an intracellular signaling domain.

Each of the elements of the CAR according to this embodiment of the first, second and third aspects are further developed below:

### a) extracellular domain comprising a CD7 targeting moiety as defined in the first and second aspects or any of its embodiments.

In an embodiment the extracellular domain comprises at least one, preferably one, of the CD7 targeting moieties as defined above in the first or second aspects, or any of their embodiments. Thus, all the embodiments defined above in said aspects apply herein.

Preferably, the extracellular domain comprising a CD7 targeting moiety comprises a ScFv comprising a VL and a VH domains, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19. Preferably, the extracellular domain comprising a CD7 targeting moiety comprises a ScFv comprising a VL and a VH domains, wherein the VL domain comprises SEQ ID NO: 20 and the VL comprises SEQ ID NO: 21. Preferably, the extracellular domain comprising a CD7 targeting moiety comprises a ScFv comprising a VL and a VH domains, wherein the VL domain consists of SEQ ID NO: 20 and the VL consists of SEQ ID NO: 21.

Preferably, the extracellular domain comprising a CD7 targeting moiety is a ScFv comprising a VL and a VH domains, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9. Preferably, the extracellular domain comprising a CD7 targeting moiety comprises a ScFv comprising a VL and a VH domains, wherein the VL domain comprises SEQ ID NO: 1 and the VL comprises SEQ ID NO: 2. Preferably, the extracellular domain comprising a CD7 targeting moiety comprises a ScFv comprising a VL and a VH domains, wherein the VL domain consists of SEQ ID NO: 1 and the VL consists of SEQ ID NO: 2.

### b) transmembrane domain

The transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, CD154 or ICOS.

A transmembrane domain may be synthetic or a variant of a naturally occurring transmembrane domain. In some embodiments, synthetic or variant transmembrane domains comprise predominantly hydrophobic residues such as leucine and valine.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, ICOS or a variant thereof, wherein the variant thereof has a 95% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, ICOS or a variant thereof, wherein the variant thereof has a 98% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, or CD154.

In some embodiments, the transmembrane domain comprises the transmembrane domain of CD8 or a variant thereof, wherein the variant thereof has a 95% sequence identity.

In some embodiments, the transmembrane domain comprises the transmembrane domain of CD8 or a variant thereof. Preferably, the transmembrane domain consists or consists essentially of SEQ ID NO: 22, or a sequence that has at least 85%, 86%, 87%, 88%, 89%, 90%, 91%, 92%, 93%, 94%, 95%, 96%, 97%, 98%, 99% or 100% sequence identity to SEQ ID NO: 22.

In an embodiment, the transmembrane domain is linked to a hinge domain, wherein the hinge domain is preferably placed at the N-terminal of the transmembrane domain. Preferably, the hinge domain consists or consists essentially of SEQ ID NO: 23, or a sequence that has at least 85%, 86%, 87%, 88%, 89%, 90%, 91%, 92%, 93%, 94%, 95%, 96%, 97%, 98%, 99% or 100% sequence identity to SEQ ID NO: 23.

Therefore, in a preferred embodiment, the transmembrane domain linked to a hinge domain consists or consists essentially of SEQ ID NO: 24, or a sequence that has at least 85%, 86%, 87%, 88%, 89% 90%, 91%, 92%, 93%, 94%, 95%, 96%, 97%, 98%, 99% or 100% sequence identity to SEQ ID NO: 24.

### c) Intracellular signaling domain

The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In some embodiments, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has a 95% sequence identity. In some embodiments, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has a 98% sequence identity. In some embodiments, the intracellular signaling domain comprises the intracellular domain of CD3ζ, FcRy, CD3γ, CD3δ, CD3ε, CD5, CD22, CD79a, CD79b or CD66b.

In some embodiments, the intracellular signaling domain comprises the intracellular domain of CD3ζ or a variant thereof, wherein the variant thereof has a 95% sequence identity. In some embodiments, the intracellular signaling domain comprises the intracellular domain of CD3ζ or a variant thereof, wherein the variant thereof has a 98% sequence identity. In some embodiments, the intracellular signaling domain comprises the intracellular domain of CD3ζ.

In a preferred embodiment, the intracellular signalling domain consists or consists essentially of SEQ ID NO: 25, or a sequence that has at least 85%, 86%, 87%, 88%, 89% 90%, 91%, 92%, 93%, 94%, 95%, 96%, 97%, 98%, 99% or 100% sequence identity to SEQ ID NO: 25. In some embodiments, the intracellular signaling domain comprises SEQ ID NO: 25. In some embodiments, the intracellular signaling domain consists of SEQ ID NO: 25.

Optionally, at least a costimulatory signaling domain may also be present in the CAR according to the first and second aspects or any of its embodiments:

### d) Costimulatory signaling domain

In some embodiments, the CAR may further comprise a costimulatory signaling domain. In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276, ICOS or a variant thereof, wherein the variant thereof has a 95% sequence identity.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276, ICOS or a variant thereof, wherein the variant thereof has a 98% sequence identity.

In some embodiments, the costimulatory signaling domain comprises the intracellular domain of CD27, CD28, CD137 or 4-1 BB, CD134, CD30, CD40, lymphocyte function-associated antigen-1 (LFA-1), CD2, CD7, LIGHT, NKG2C, CD276 or ICOS.

In some embodiments, the costimulatory signaling domain comprises the intracellular domain of CD137 or a variant thereof, wherein the variant thereof has a 95% sequence identity. In some embodiments, the costimulatory signaling domain comprises the intracellular domain of CD137 or 4-1BB or a variant thereof, wherein the variant thereof has a 98% sequence identity. In some embodiments, the costimulatory signaling domain comprises the intracellular domain of CD137 or 4-1BB.

In a preferred embodiment, the costimulatory signaling domain consists or consists essentially of SEQ ID NO: 26, or a sequence that has at least 85%, 86%, 87%, 88%, 89% 90%, 91%, 92%, 93%, 94%, 95%, 96%, 97%, 98%, 99% or 100% sequence identity to SEQ ID NO: 26. In some embodiments, the costimulatory signaling domain comprises SEQ ID NO: 26. In some embodiments, the costimulatory signaling domain consists of SEQ ID NO: 26.

### Full CARs sequence according to the first and second aspects of the present invention

In some embodiments, the CAR comprises:
(a) an extracellular domain comprising a CD7 targeting moiety that is a:
   - scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9, or
   - scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19; and
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a CD7 targeting moiety that is a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9; and
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19, and
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a:
   - scFv comprising a VL domain and VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, or
   - scFv comprising a VL domain and VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a CD7 targeting moiety that is a scFv comprising a VL domain and VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19; and
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising a VL domain and VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a:
   - scFv comprising a VL domain and VH domain, wherein the VL domain comprises SEQ ID NO: 1, and the VH domain comprises SEQ ID NO: 2 or
   - scFv comprising a VL domain and VH domain, wherein the VL domain comprises SEQ ID NO: 54, and the VH domain comprises SEQ ID NO: 55,or
   - scFv comprising a VL domain and VH domain, wherein the VL domain comprises SEQ ID NO: 20, and the VH domain comprises SEQ ID NO: 21, or
   - scFv comprising a VL domain and VH domain, wherein the VL domain comprises SEQ ID NO: 56, and the VH domain comprises SEQ ID NO: 57; and
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a CD7 targeting moiety that is a:
   - scFv comprising a VL domain and VH domain, wherein the VL domain consists of SEQ ID NO: 1, and the VH domain consists of SEQ ID NO: 2 or
   - scFv comprising a VL domain and VH domain, wherein the VL domain consists of SEQ ID NO: 54, and the VH domain consists of SEQ ID NO: 55,or
   - scFv comprising a VL domain and VH domain, wherein the VL domain consists of SEQ ID NO: 20, and the VH domain consists of SEQ ID NO: 21, or
   - scFv comprising a VL domain and VH domain, wherein the VL domain consists of SEQ ID NO: 56, and the VH domain consists of SEQ ID NO: 57; and
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a CD7 targeting moiety that is a scFv comprising a VL domain and VH domain, wherein the VL domain comprises SEQ ID NO: 20, and the VH domain comprises SEQ ID NO: 21;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising a VL domain and VH domain, wherein the VL domain consists of SEQ ID NO: 20, and the VH domain consists of SEQ ID NO: 21;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising a VL domain and VH domain, wherein the VL domain comprises SEQ ID NO: 1, and the VH domain comprises SEQ ID NO: 2;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising a VL domain and VH domain, wherein the VL domain consists of SEQ ID NO: 1, and the VH domain consists of SEQ ID NO: 2;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising SEQ ID NOs: 3, 60, 11, or 61, preferably SEQ ID NO: 11;
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a CD7 targeting moiety that is a scFv consisting of SEQ ID NOs: 3, 60, 11, or 61, preferably SEQ ID NO: 11;
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a CD7 targeting moiety that is a scFv comprising SEQ ID NOs: 3 or 11, preferably 11;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv consisting of SEQ ID NOs: 3 or 11, preferably 11;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising SEQ ID NOs: 60 or 61, preferably 61;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv consisting of SEQ ID NOs: 60 or 61, preferably 61;
(b) the transmembrane domain of CD8;
(c) the intracellular domain of CD3ζ; and
(d) the intra-cellular domain of CD137.

In some embodiments, the CAR comprises:
(a) an extracellular domain comprising a CD7 targeting moiety that is a scFv comprising SEQ ID NOs: 3 or 11;
(b) a transmembrane domain comprising SEQ ID NO: 24;
(c) an intracellular signaling domain comprising SEQ ID NO: 25; and
(d) a costimulatory signaling domain comprising SEQ ID NO: 26.

In some embodiments, the CAR comprises:
(a) an extracellular domain comprising a CD7 targeting moiety that is a scFv consisting of SEQ ID NOs: 3 or 11;
(b) a transmembrane domain comprising SEQ ID NO: 24;
(c) an intracellular signaling domain comprising SEQ ID NO: 25; and
(d) a costimulatory signaling domain comprising SEQ ID NO: 26.

In some embodiments, the CAR comprises:
(a) an extracellular domain comprising a CD7 targeting moiety that is a scFv comprising or consisting of SEQ ID NOs: 3 or 11;
(b) a transmembrane domain consisting of SEQ ID NO: 24;
(c) an intracellular signaling domain consisting of SEQ ID NO: 25; and
(d) a costimulatory signaling domain consisting of SEQ ID NO: 26.

In some embodiments, the CAR comprises or consists of SEQ ID NO: 13 **(CD7 CAR 124.1D1),** or a sequence that has at least 85%, 86%, 87%, 88%, 89% 90%, 91%, 92%, 93%, 94%, 95%, 96%, 97%, 98%, 99% or 100% sequence identity to SEQ ID NO: 13. In some embodiments, the CAR comprises or consists of SEQ ID NO: 13. In some embodiments, the CAR comprises or consists of SEQ ID NO: 13.

In some embodiments, the CAR comprises or consists of SEQ ID NO: 65 **(CD7 CAR 124.1D1),** or a sequence that has at least 85%, 86%, 87%, 88%, 89% 90%, 91%, 92%, 93%, 94%, 95%, 96%, 97%, 98%, 99% or 100% sequence identity to SEQ ID NO: 65. In some embodiments, the CAR comprises or consists of SEQ ID NO: 65. In some embodiments, the CAR comprises or consists of SEQ ID NO: 65.

In some embodiments, the CAR comprises or consists of SEQ ID NO: 12 **(CD7 CAR 142.24),** or a sequence that has at least 85%, 86%, 87%, 88%, 89% 90%, 91%, 92%, 93%, 94%, 95%, 96%, 97%, 98%, 99% or 100% sequence identity to SEQ ID NO: 12. In some embodiments, the CAR comprises or consists of SEQ ID NO: 12. In some embodiments, the CAR comprises or consists of SEQ ID NO: 12.

In some embodiments, the CAR comprises or consists of SEQ ID NO: 66 **(CD7 CAR 142.24),** or a sequence that has at least 85%, 86%, 87%, 88%, 89% 90%, 91%, 92%, 93%, 94%, 95%, 96%, 97%, 98%, 99% or 100% sequence identity to SEQ ID NO: 66. In some embodiments, the CAR comprises or consists of SEQ ID NO: 66. In some embodiments, the CAR comprises or consists of SEQ ID NO: 66.

### CD7-trap protein of the invention

As explained above, the CD7 targeting moieties of the first, second, or third aspect, or any of their embodiments, may be comprised in a CD7-trap protein. The term "CD7-trap" or "CD7-trap protein" or "the CD7-trap of the invention" refers to a protein or polypeptide that comprises a first region comprising a CD7-targeting moiety and a second region comprising an amino acid sequence that avoids the surface location of the human CD7 protein. Thus, the CD7-trap protein is capable of binding to intracellular CD7 via its first region and causing a downregulation of the expression of human CD7 in the surface of the cell via its second region. By "capable of downregulate" is referred herein as capable of reducing or eliminating the expression of CD7 molecule in the surface of a cell in comparison with a control cell that does not comprise said CD7-Trap. In some embodiments, the CD7-trap is a protein expression blocker that avoids or reduces the location or expression of the human CD7 on the surface of the cell, preferably of a T cell. In the context of the present invention, "CD7-trap" is synonymous to "CD7-retention domain", and thus both terms are used interchangeably.

In an embodiment, the first region of the CD7-trap comprises the CD7-targeting moiety of the first, second or third aspects, or any of their embodiments, and the second region of the CD7-trap comprises an endoplasmic reticulum (ER) or Golgi retention sequence. Said retention sequence can direct the CD7-targeting moiety to a specific cellular compartment, such as the Endoplasmic reticulum or the Golgi. Preferably, the first region is located in the N-terminal region, preferably N-terminal end, of the CD7-trap protein, and the second region is located in the C-terminal region, preferably C-terminal end, of the CD7-trap protein.

In some embodiments, the retention sequence comprises, consists or consists essentially of SEQ ID NO: 30 [AEKDEL], or a sequence that has at least 50%, 60%, 66.6%, 80%, 83.3%, 85%, 86%, 87%, 88%, 89%, 90%, 91%, 92%, 93%, 94%, 95%, 96%, 97%, 98%, 99% or 100% sequence identity to SEQ ID NO: 30.

Preferably, the first and the second regions of the CD7-trap protein of the present invention are connected by means of a linker. Preferably, the linker is a peptide linker and comprises, consists or consists essentially of SEQ ID NO: 10, or a sequence that has at least 85%, 86%, 87%, 88%, 89%, 90%, 91%, 92%, 93%, 94%, 95%, 96%, 97%, 98%, 99% or 100% sequence identity to SEQ ID NO: 10.

Preferably, the first region of the CD7-trap protein comprises in its N-terminal end a signal peptide. Preferably, the signal peptide comprises, consists or consists essentially of SEQ ID NO: 27, or a sequence that has at least 85%, 86%, 87%, 88%, 89%, 90%, 91%, 92%, 93%, 94%, 95%, 96%, 97%, 98%, 99% or 100% sequence identity to SEQ ID NO: 27.

Thus, preferably, the CD-7 trap protein comprises in the N-terminal to C-terminal direction:
- a signal peptide, preferably comprising or consisting of SEQ ID NO: 27,
- first region comprising a CD7-targeting moiety, preferably the CD7-targeting moieties as defined in the first, second or third aspects, or any of their embodiments,
- a linker, preferably comprising or consisting of SEQ ID NO: 10, and
- a second region comprising a sequence, preferably an amino acid sequence, that avoids the surface location of the CD7 protein, most preferably comprising or consisting of SEQ ID NO: 30.

In a preferred embodiment, the CD7-trap comprises a first region comprising the CD7-targeting moiety of the first aspect or any of its embodiments and a second region comprising an ER retention sequence. Preferably, the CD7-trap of the present invention comprises, preferably in the N-terminal to C-terminal direction:
(i) the CD7-targeting moiety of the first aspect or any of its embodiments **(CD7 124.1D1)** comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
   - LCDR1 comprises or consists of SEQ ID NO: 4;
   - LCDR2 comprises or consists of SEQ ID NO: 5;
   - LCDR3 comprises or consists of SEQ ID NO: 6;
   - HCDR1 comprises or consists of SEQ ID NO: 7;
   - HCDR2 comprises or consists of SEQ ID NO: 8;
   - HCDR3 comprises or consists of SEQ ID NO: 9, and
(ii) an ER retention sequence, wherein the retention sequence comprises, consists or consists essentially of SEQ ID NO: 30 [AEKDEL], or a sequence that has at least 60%, 66.6%, 80%, 83.3%, 90% or 100% sequence identity to SEQ ID NO: 30.

In an embodiment, the CD7-trap comprises:
(i) a ScFv **(CD7 124.1D1)** comprising a VL and a VH domains, wherein the VL domain comprises a sequence having at least 85% sequence identity to SEQ ID NO: 1, wherein the CDRs comprised in said VL are maintained constant (i.e., invariable) and consists of SEQ ID NOs: 4, 5, and 6; and wherein the VH domain comprises a sequence having at least 85% sequence identity to SEQ ID NO: 2, wherein the CDRs comprised in said VH are maintained constant and consist of SEQ ID NOs: 7, 8, and 9, and
(ii) a retention sequence that comprises, consists or consists essentially of SEQ ID NO: 30 [AEKDEL], or a sequence that has at least 60%, 66.6%, 80%, 83.3%, 90% or 100% sequence identity to SEQ ID NO: 30;
(iii) optionally, a linker to connect i) and ii), preferably wherein the linker comprises, consists or consists essentially of SEQ ID NO: 10, or a sequence that has at least 85%, 86%, 87%, 88%, 89%, 90%, 91%, 92%, 93%, 94%, 95%, 96%, 97%, 98%, 99% or 100% sequence identity to SEQ ID NO: 10; and
(iv) optionally, a signal peptide before (i) (i.e., in the N-terminal region of the ScFv), wherein, preferably, the signal peptide comprises, consists or consists essentially of SEQ ID NO: 27, or a sequence that has at least 85%, 86%, 87%, 88%, 89%, 90%, 91%, 92%, 93%, 94%, 95%, 96%, 97%, 98%, 99% or 100% sequence identity to SEQ ID NO: 27.

Preferably, the CD7-trap comprises, preferably in the N-terminal to C-terminal direction, a VL domain, a VH domain and a retention sequence, wherein the VL domain comprises SEQ ID NO: 1, wherein the VH domain comprises SEQ ID NO: 2, and wherein the retention sequence comprises SEQ ID NO: 30 [AEKDEL], Preferably, the CD7-trap comprises, in the N-terminal to C-terminal direction, a VL domain, a VH domain and a retention sequence, wherein the VL domain consists of SEQ ID NO: 1, wherein the VH domain consists of SEQ ID NO: 2, and wherein the retention sequence consists of SEQ ID NO: 30 [AEKDEL],

Preferably, the CD7-trap comprises, preferably in the N-terminal to C-terminal direction, a VL domain, a VH domain and a retention sequence, wherein the VL domain comprises SEQ ID NO: 54, wherein the VH domain comprises SEQ ID NO: 55, and wherein the retention sequence comprises SEQ ID NO: 30 [AEKDEL], Preferably, the CD7-trap comprises, in the N-terminal to C-terminal direction, a VL domain, a VH domain and a retention sequence, wherein the VL domain consists of SEQ ID NO: 54, wherein the VH domain consists of SEQ ID NO: 55, and wherein the retention sequence consists of SEQ ID NO: 30 [AEKDEL],

Preferably, the CD7-trap comprises, preferably in the N-terminal to C-terminal direction, a CD7-targeting moiety and a retention sequence, wherein the CD7-targeting moiety comprises, consists, or consists essentially of SEQ ID NO: 3 or 60, preferably SEQ ID NO: 3 **(CD7_124.1D1),** or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 3 or 60, preferably SEQ ID NO: 3, and wherein the retention sequence comprises, consists or consists essentially of SEQ ID NO: 30 [AEKDEL], or a sequence that has at least 60%, 66.6%, 80%, 83.3%, 90% or 100% sequence identity to SEQ ID NO: 30. Preferably, the CD7-trap comprises, in the N-terminal to C-terminal direction, a CD7-targeting moiety and a retention sequence, wherein the CD7-targeting moiety consists of SEQ ID NO: 3 or 60, preferably SEQ ID NO: 3 **(CD7_124.1D1),** and the retention sequence consists of SEQ ID NO: 30.

In some embodiments, the CD7-trap comprises or consists of SEQ ID NO: 31 **(CD7 trap 124.1D1),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 31. In some embodiments, the CD7-trap comprises or consists of SEQ ID NO: 67 **(CD7 trap 124.1D1),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 67. In some embodiments, the nucleotide sequence encoding for the CD7-trap comprises or consists of SEQ ID NO: 33, or a sequence that has at least 85%, 86%, 87%, 88%, 89%, 90%, 91%, 92%, 93%, 94%, 95%, 96%, 97%, 98%, 99% or 100% sequence identity to SEQ ID NO: 33 **(CD7 trap 124.1D1).**

In some embodiments, the CD7-trap comprises a first region comprising the CD7-moiety of the second aspect or any of its embodiments and a second region comprising an ER retention sequence. Preferably, the CD7-trap comprises, preferably in the N-terminal to C-terminal direction:
(i) the CD7-targeting moiety of the second aspect or any of its embodiments **(CD7_142.24)** comprising a VL domain and a VH domains, wherein said VL domain comprises LCDR1, LCDR2 and LCDR3 polypeptides and said VH domain comprises HCDR1, HCDR2 and HCDR3 polypeptides, and wherein:
   - LCDR1 comprises or consists of SEQ ID NO: 14;
   - LCDR2 comprises or consists of SEQ ID NO: 15;
   - LCDR3 comprises or consists of SEQ ID NO: 16;
   - HCDR1 comprises or consists of SEQ ID NO: 17;
   - HCDR2 comprises or consists of SEQ ID NO: 18;
   - HCDR3 comprises or consists of SEQ ID NO: 19, and
(ii) an ER retention sequence, wherein the retention sequence comprises, consists or consists essentially of SEQ ID NO: 30 [AEKDEL], or a sequence that has at least 60%, 66.6%, 80%, 83.3%, 90% or 100% sequence identity to SEQ ID NO: 30.

In an embodiment, the CD7-trap comprises:
(i) a ScFv **(CD7_142.24)** comprising a VL and a VH domains, wherein the VL domain comprises a sequence having at least 85% sequence identity to SEQ ID NO: 20, wherein the CDRs comprised in said VL are maintained constant (i.e., non-variable) and consists of SEQ ID NOs: 14, 15, and 16; and wherein the VH domain comprises a sequence having at least 85% sequence identity to SEQ ID NO: 21, wherein the CDRs comprised in said VH are maintained constant and consist of SEQ ID NOs: 17, 18, and 19, and
(ii) a retention sequence that comprises, consists or consists essentially of SEQ ID NO: 30 [AEKDEL], or a sequence that has at least 60%, 66.6%, 80%, 83.3%, 90% or 100% sequence identity to SEQ ID NO: 3;
(iii) optionally, a linker to connect i) and ii), preferably wherein the linker comprises, consists or consists essentially of SEQ ID NO: 10, or a sequence that has at least 85%, 86%, 87%, 88%, 89%, 90%, 91%, 92%, 93%, 94%, 95%, 96%, 97%, 98%, 99% or 100% sequence identity to SEQ ID NO: 10, and
(iv) optionally, a signal peptide before (i) (i.e., in the N-terminal region of the ScFv), wherein, preferably, the signal peptide comprises, consists or consists essentially of SEQ ID NO: 27, or a sequence that has at least 85%, 86%, 87%, 88%, 89%, 90%, 91%, 92%, 93%, 94%, 95%, 96%, 97%, 98%, 99% or 100% sequence identity to SEQ ID NO: 27.

Preferably, the CD7-trap comprises, preferably in the N-terminal to C-terminal direction, a VL domain, a VH domain and a retention sequence, wherein the VL domain comprises SEQ ID NO: 20, wherein the VH domain comprises SEQ ID NO: 21, and wherein the retention sequence comprises SEQ ID NO: 30 [AEKDEL], Preferably, the CD7-trap comprises, in the N-terminal to C-terminal direction, a VL domain, a VH domain and a retention sequence, wherein the VL domain consists of SEQ ID NO: 20, wherein the VH domain consists of SEQ ID NO: 21, and wherein the retention sequence consists of SEQ ID NO: 30 [AEKDEL],

Preferably, the CD7-trap comprises, preferably in the N-terminal to C-terminal direction, a VL domain, a VH domain and a retention sequence, wherein the VL domain comprises SEQ ID NO: 56, wherein the VH domain comprises SEQ ID NO: 57, and wherein the retention sequence comprises SEQ ID NO: 30 [AEKDEL], Preferably, the CD7-trap comprises, in the N-terminal to C-terminal direction, a VL domain, a VH domain and a retention sequence, wherein the VL domain consists of SEQ ID NO: 56, wherein the VH domain consists of SEQ ID NO: 57, and wherein the retention sequence consists of SEQ ID NO: 30 [AEKDEL],

Preferably, the CD7-trap comprises, preferably in the N-terminal to C-terminal direction, a CD7-targeting moiety and a retention sequence, wherein the CD7-targeting moiety comprises, consists, or consists essentially of SEQ ID NO: 11 or 61, preferably SEQ ID NO: 11 **(CD7_142.24),** or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11 or 61, preferably SEQ ID NO: 11, and wherein the retention sequence comprises, consists or consists essentially of SEQ ID NO: 30 [AEKDEL], or a sequence that has at least 60%, 66.6%, 80%, 83.3%, 90% or 100% sequence identity to SEQ ID NO: 30. Preferably, the CD7-trap comprises, in the N-terminal to C-terminal direction, a CD7-targeting moiety and a retention sequence, wherein the CD7-targeting moiety consists of SEQ ID NO: 11 or 61, preferably SEQ ID NO: 11, and the retention sequence consists of SEQ ID NO: 30.

In some embodiments, the CD7-trap comprises or consists of SEQ ID NO: 32 **(CD7 trap 142.24),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 32. In some embodiments, the CD7-trap comprises or consists of SEQ ID NO: 68 **(CD7 trap 142.24),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 68. In some embodiments, the nucleotide sequence encoding for the CD7-trap comprises or consists of SEQ ID NO: 34 **(CD7 trap 142.24),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 34.

In some embodiments, the CD7-trap comprises a first region comprising the CD7-moiety of the third aspect or any of its embodiments and a second region comprising an ER retention sequence. Preferably, the CD7-trap comprises, preferably in the N-terminal to C-terminal direction:
(i) the CD7-targeting moiety of the third aspect or any of its embodiments **(CD7_142.9)** comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
   - LCDR1 comprises or consists of SEQ ID NO: 42;
   - LCDR2 comprises or consists of SEQ ID NO: 43;
   - LCDR3 comprises or consists of SEQ ID NO: 44;
   - HCDR1 comprises or consists of SEQ ID NO: 45;
   - HCDR2 comprises or consists of SEQ ID NO: 46;
   - HCDR3 comprises or consists of SEQ ID NO: 47, and
(ii) an ER retention sequence, wherein the retention sequence comprises, consists or consists essentially of SEQ ID NO: 30 [AEKDEL], or a sequence that has at least 60%, 66.6%, 80%, 83.3%, 90% or 100% sequence identity to SEQ ID NO: 30.

In an embodiment, the CD7-trap comprises:
(i) a ScFv **(CD7_142.9)** comprising a VL and a VH domains, wherein the VL domain comprises a sequence having at least 85% sequence identity to SEQ ID NO: 48, wherein the CDRs comprised in said VL are maintained constant (i.e., non-variable) and consists of SEQ ID NOs: 42, 43, and 44; and wherein the VH domain comprises a sequence having at least 85% sequence identity to SEQ ID NO: 50, wherein the CDRs comprised in said VH are maintained constant and consist of SEQ ID NOs: 45, 46, and 47, and
(ii) a retention sequence that comprises, consists or consists essentially of SEQ ID NO: 30 [AEKDEL], or a sequence that has at least 60%, 66.6%, 80%, 83.3%, 90% or 100% sequence identity to SEQ ID NO: 30;
(iii) optionally, a linker to connect i) and ii), preferably wherein the linker comprises, consists or consists essentially of SEQ ID NO: 10, or a sequence that has at least 85%, 86%, 87%, 88%, 89%, 90%, 91%, 92%, 93%, 94%, 95%, 96%, 97%, 98%, 99% or 100% sequence identity to SEQ ID NO: 10, and
(iv) optionally, a signal peptide before (i) (i.e., in the N-terminal region of the ScFv), wherein, preferably, the signal peptide comprises, consists or consists essentially of SEQ ID NO: 27, or a sequence that has at least 85%, 86%, 87%, 88%, 89%, 90%, 91%, 92%, 93%, 94%, 95%, 96%, 97%, 98%, 99% or 100% sequence identity to SEQ ID NO: 27.

Preferably, the CD7-trap comprises, preferably in the N-terminal to C-terminal direction, a VL domain, a VH domain and a retention sequence, wherein the VL domain comprises SEQ ID NO: 48, wherein the VH domain comprises SEQ ID NO: 50, and wherein the retention sequence comprises SEQ ID NO: 30 [AEKDEL]. Preferably, the CD7-trap comprises, in the N-terminal to C-terminal direction, a VL domain, a VH domain and a retention sequence, wherein the VL domain consists of SEQ ID NO: 48, wherein the VH domain consists of SEQ ID NO: 50, and wherein the retention sequence consists of SEQ ID NO: 30 [AEKDEL].

Preferably, the CD7-trap comprises, preferably in the N-terminal to C-terminal direction, a VL domain, a VH domain and a retention sequence, wherein the VL domain comprises SEQ ID NO: 58 wherein the VH domain comprises SEQ ID NO: 59, and wherein the retention sequence comprises SEQ ID NO: 30 [AEKDEL]. Preferably, the CD7-trap comprises, in the N-terminal to C-terminal direction, a VL domain, a VH domain and a retention sequence, wherein the VL domain consists of SEQ ID NO: 58, wherein the VH domain consists of SEQ ID NO: 59, and wherein the retention sequence consists of SEQ ID NO: 30 [AEKDEL].

Preferably, the CD7-trap comprises, preferably in the N-terminal to C-terminal direction, a CD7-targeting and a retention sequence, wherein the CD7-targeting moiety comprises, consists, or consists essentially of SEQ ID NO: 52 or 62, preferably SEQ ID NO: 52, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 52 or 62, preferably SEQ ID NO: 52, and wherein the retention sequence comprises, consists or consists essentially of SEQ ID NO: 30 [AEKDEL], or a sequence that has at least 60%, 66.6%, 80%, 83.3%, 90% or 100% sequence identity to SEQ ID NO: 30. Preferably, the CD7-trap comprises, in the N-terminal to C-terminal direction, a CD7-targeting moiety and a retention sequence, wherein the CD7-targeting moiety consists of SEQ ID NO: 52 or 62, preferably SEQ ID NO: 52, and the retention sequence consists of SEQ ID NO: 30.

In some embodiments, the CD7-trap comprises or consists of SEQ ID NO: 63 (**CD7 trap 142.9),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 63. In some embodiments, the CD7-trap comprises or consists of SEQ ID NO: 69 (**CD7 trap 142.9),** or a sequence that has at least 85%, 86%, 87%, 88%, 89%, 90%, 91%, 92%, 93%, 94%, 95%, 96%, 97%, 98%, 99% or 100% sequence identity to SEQ ID NO: 69. In some embodiments, the nucleotide sequence encoding for the CD7-trap comprises or consists of SEQ ID NO: 64 (**CD7 trap 142.9**), or a sequence that has at least 85%, 86%, 87%, 88%, 89%, 90%, 91%, 92%, 93%, 94%, 95%, 96%, 97%, 98%, 99% or 100% sequence identity to SEQ ID NO: 64.

### Nucleic acid molecules of the invention

In **a fourth aspect**, the present invention provides a nucleic acid, also called herein the nucleic acid of the present invention, encoding one or more of the CD7 targeting moieties defined above in the first, second or third aspects, including any one of the CAR anti-CD7 and CD7-traps disclosed above in the first, second or third aspects, or any of their embodiments. The nucleic acid sequence that encodes the chimeric receptor links together a number of modular components that can be excised and replaced with other components in order to customize the chimeric receptor for efficient T cell activation and recognition of CD7.

In a preferred embodiment, the nucleic acid comprises, consists or consists essentially of SEQ ID NO: 53 **(CD7 CAR 124.1D1),** or a sequence that has at least 85%, 86%, 87%, 88%, 89% 90%, 91%, 92%, 93%, 94%, 95%, 96%, 97%, 98%, 99% or 100% sequence identity to SEQ ID NO: 53. In some embodiments, the nucleic acid comprises or consists of SEQ ID NO: 53.

In a preferred embodiment, the nucleic acid comprises, consists or consists essentially of SEQ ID NO: 28 **(CD7 CAR 142.24),** or a sequence that has at least 85%, 86%, 87%, 88%, 89% 90%, 91%, 92%, 93%, 94%, 95%, 96%, 97%, 98%, 99% or 100% sequence identity to SEQ ID NO: 28. In some embodiments, the nucleic acid comprises or consists of SEQ ID NO: 28.

In a preferred embodiment, the nucleic acid comprises, consists or consists essentially of SEQ ID NO: 33 **(CD7 Trap 124.1D1),** or a sequence that has at least 85%, 86%, 87%, 88%, 89% 90%, 91%, 92%, 93%, 94%, 95%, 96%, 97%, 98%, 99% or 100% sequence identity to SEQ ID NO: 33. In some embodiments, the nucleic acid comprises or consists of SEQ ID NO: 33.

In a preferred embodiment, the nucleic acid comprises, consists or consists essentially of SEQ ID NO: 34 **(CD7 Trap 142.24),** or a sequence that has at least 85%, 86%, 87%, 88%, 89% 90%, 91%, 92%, 93%, 94%, 95%, 96%, 97%, 98%, 99% or 100% sequence identity to SEQ ID NO: 34. In some embodiments, the nucleic acid comprises or consists of SEQ ID NO: 34.

In a preferred embodiment, the nucleic acid comprises, consists or consists essentially of SEQ ID NO: 64 **(CD7 Trap 142.9),** or a sequence that has at least 85%, 86%, 87%, 88%, 89% 90%, 91%, 92%, 93%, 94%, 95%, 96%, 97%, 98%, 99% or 100% sequence identity to SEQ ID NO: 64. In some embodiments, the nucleic acid comprises or consists of SEQ ID NO: 64.

### Bicistronic CARCD7Trap nucleic acid molecule of the invention

In some embodiments, the nucleic acid molecule of the present invention is a bicistronic nucleic acid and it encodes, in the same ORF, for two or more of the CARs and CD7-trap proteins as defined above.

Hence, two or more of the CD7-targeting moieties as defined above in the first, second and third aspects, and any of their embodiments, can be encoded by a bicistronic nucleic acid molecule, which is also referred herein as the "bicistronic CARCD7Trap nucleic acid molecule of the invention". By "bicistronic nucleic acid molecule" is referred herein to a polynucleotide comprising at least two distinct genes of interest encoding for two therapeutic proteins, preferably one of them being a CAR against CD7 and the other being a CD7 trap as defined herein.

Preferably, the two therapeutic genes are under the control of a single promoter. Thus, in an embodiment, the bicistronic nucleic acid molecule, as referred herein, is a nucleic acid molecule that allows the simultaneous expression of at least two therapeutic proteins (a CAR and a CD7-trap protein) that are derived or originated from the same RNA transcript.

Preferably, the bicistronic CARCD7Trap nucleic acid molecule of the invention comprises a least:
- a first nucleotide sequence encoding for the CAR anti-CD7 of the invention as defined above, and
- a second nucleotide sequence encoding for the CD7-trap protein of the invention as defined above,
wherein both nucleotide sequences are placed in tandem, under the control of the same promoter. In some embodiments, a nucleotide sequence encoding for a 2A peptide cleavage site is placed between the first and second nucleotide sequences. By "2 A cleavage peptide" is referred herein to an auto-catalytic peptide, preferably selected from the group comprising T2A element (SEQ ID NO: 29), a P2A cleavage peptide (SEQ ID NO: 35), a E2A cleavage peptide (SEQ ID NO: 36), or an F2A cleavage peptide (SEQ ID NO: 37).

In some other embodiments, an internal ribosome entry site (IRES) is placed between the first and second nucleotide sequences. Preferably, the IRES is derived from a picornavirus IRES sequence; the IRES sequence is selected from the group consisting of an enterovirus, rhinovirus, cardiovirus, and aphthovirus IRES sequence; the IRES is selected from the group consisting of a hepatitis A virus IRES sequence, an hepatitis B virus sequence and an hepatitis C virus IRES sequence.

Preferably, the bicistronic CARCD7Trap nucleic acid molecule comprises, preferably in the 5' to 3' direction:
- a first nucleotide sequence encoding for CAR that comprises an extracellular domain comprising the CD7 targeting moiety of the first aspect or any of its embodiments **(CD7_124.1D1),** and
- a second nucleotide sequence encoding for a CD7-trap protein that comprises the CD7-targeting moiety of the first aspect or any of its embodiments **(CD7_124.1D1),**
preferably wherein the first and second nucleotide sequences are connected by a sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for CAR that comprises an extracellular domain comprising the CD7 targeting moiety of the first aspect or any of its embodiments **(CD7_124.1D1),** and
- a second nucleotide sequence encoding for a CD7-trap protein that comprises the CD7-targeting moiety of the second aspect or any of its embodiments **(CD7_142.24),**
preferably wherein the first and second nucleotide sequences are connected by a sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for CAR that comprises an extracellular domain comprising the CD7 targeting moiety of the first aspect or any of its embodiments **(CD7_124.1D1),** and
- a second nucleotide sequence encoding for a CD7-trap protein that comprises the CD7-targeting moiety of the third aspect or any of its embodiments **(CD7_142.9),**
preferably wherein the first and second nucleotide sequences are connected by a sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for CAR that comprises an extracellular domain comprising the CD7 targeting moiety of the second aspect or any of its embodiments **(CD7_142.24),** and
- a second nucleotide sequence encoding for a CD7-trap protein that comprises the CD7-targeting moiety of the second aspect or any of its embodiments **(CD7_142.24),**
preferably wherein the first and second nucleotide sequences are connected by a sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for CAR that comprises an extracellular domain comprising the CD7 targeting moiety of the second aspect or any of its embodiments **(CD7_142.24),** and
- a second nucleotide sequence encoding for a CD7-trap protein that comprises the CD7-targeting moiety of the first aspect or any of its embodiments **(CD7_124.1D1),**
preferably wherein the first and second nucleotide sequences are connected by a sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for CAR that comprises an extracellular domain comprising the CD7 targeting moiety of the second aspect or any of its embodiments **(CD7_142.24),** and
- a second nucleotide sequence encoding for a CD7-trap protein that comprises the CD7-targeting moiety of the third aspect or any of its embodiments **(CD7_142.9),**
preferably wherein the first and second nucleotide sequences are connected by a sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising a VL and a VH domain, wherein the VL domain comprises a sequence having at least 85% sequence identity to SEQ ID NO: 20, wherein the CDRs comprised in said VL are maintained constant and consist of SEQ ID NOs: 14, 15, and 16; and wherein the VH domain comprises a sequence having at least 85% sequence identity to SEQ ID NO: 21, wherein the CDRs comprised in said VH are maintained constant and consist of SEQ ID NOs: 17, 18, and 19;
   (b) the transmembrane domain of CD8;
   (c) the intracellular domain of CD3ζ; and
   (d) the intra-cellular domain of CD137; and
- a second nucleotide sequence encoding for a CD7-trap that comprises:
   (a) a ScFv **(CD7_124.1D1)** comprising a VL and a VH domains, wherein the VL domain comprises a sequence having at least 85% sequence identity to SEQ ID NO: 1, wherein the CDRs comprised in said VL are maintained constant (i.e., non variable) and consists of SEQ ID NOs: 4, 5, and 6; and wherein the VH domain comprises a sequence having at least 85% sequence identity to SEQ ID NO: 2, wherein the CDRs comprised in said VH are maintained constant and consist of SEQ ID NOs: 7, 8, and 9, and
   (b) a retention sequence that comprises SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising a VL and a VH domain, wherein the VL domain comprises a sequence having at least 85% sequence identity to SEQ ID NO: 56, wherein the CDRs comprised in said VL are maintained constant and consist of SEQ ID NOs: 14, 15, and 16; and wherein the VH domain comprises a sequence having at least 85% sequence identity to SEQ ID NO: 57, wherein the CDRs comprised in said VH are maintained constant and consist of SEQ ID NOs: 17, 18, and 19;
   (b) the transmembrane domain of CD8;
   (c) the intracellular domain of CD3ζ; and
   (d) the intra-cellular domain of CD137; and
- a second nucleotide sequence encoding for a CD7-trap that comprises:
   (a) a ScFv **(CD7_124.1D1)** comprising a VL and a VH domains, wherein the VL domain comprises a sequence having at least 85% sequence identity to SEQ ID NO: 1, wherein the CDRs comprised in said VL are maintained constant (i.e., non variable) and consists of SEQ ID NOs: 4, 5, and 6; and wherein the VH domain comprises a sequence having at least 85% sequence identity to SEQ ID NO: 2, wherein the CDRs comprised in said VH are maintained constant and consist of SEQ ID NOs: 7, 8, and 9, and
   (b) a retention sequence that comprises SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising a VL and a VH domain, wherein the VL domain comprises a sequence having at least 85% sequence identity to SEQ ID NO: 20, wherein the CDRs comprised in said VL are maintained constant and consist of SEQ ID NOs: 14, 15, and 16; and wherein the VH domain comprises a sequence having at least 85% sequence identity to SEQ ID NO: 21, wherein the CDRs comprised in said VH are maintained constant and consist of SEQ ID NOs: 17, 18, and 19;
   (b) the transmembrane domain of CD8;
   (c) the intracellular domain of CD3ζ; and
   (d) the intra-cellular domain of CD137; and
- a second nucleotide sequence encoding for a CD7-trap that comprises:
   (a) a ScFv **(CD7_142.9)** comprising a VL and a VH domains, wherein the VL domain comprises a sequence having at least 85% sequence identity to SEQ ID NO: 48, wherein the CDRs comprised in said VL are maintained constant (i.e., non variable) and consists of SEQ ID NOs: 42, 43, and 44; and wherein the VH domain comprises a sequence having at least 85% sequence identity to SEQ ID NO: 50, wherein the CDRs comprised in said VH are maintained constant and consist of SEQ ID NOs: 45, 46, and 47, and
   (b) a retention sequence that comprises SEQ ID NO: 30 [AEKDEL],
   preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising a VL and a VH domain, wherein the VL domain comprises a sequence having at least 85% sequence identity to SEQ ID NO: 56, wherein the CDRs comprised in said VL are maintained constant and consist of SEQ ID NOs: 14, 15, and 16; and wherein the VH domain comprises a sequence having at least 85% sequence identity to SEQ ID NO: 57, wherein the CDRs comprised in said VH are maintained constant and consist of SEQ ID NOs: 17, 18, and 19;
   (b) the transmembrane domain of CD8;
   (c) the intracellular domain of CD3ζ; and
   (d) the intra-cellular domain of CD137; and
- a second nucleotide sequence encoding for a CD7-trap that comprises:
   (a) a ScFv **(CD7_142.9)** comprising a VL and a VH domains, wherein the VL domain comprises a sequence having at least 85% sequence identity to SEQ ID NO: 48, wherein the CDRs comprised in said VL are maintained constant (i.e., non variable) and consists of SEQ ID NOs: 42, 43, and 44; and wherein the VH domain comprises a sequence having at least 85% sequence identity to SEQ ID NO: 50, wherein the CDRs comprised in said VH are maintained constant and consist of SEQ ID NOs: 45, 46, and 47, and
   (b) a retention sequence that comprises SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv ScFv **(CD7_142.24)** comprising a VL and a VH domains, wherein the VL domain consists of SEQ ID NO: 20, and wherein the VH domain consists of SEQ ID NO: 21,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26; and
- a second nucleotide sequence encoding for a CD7-trap that comprises:
   (a) a ScFv **(CD7_142.9)** comprising a VL and a VH domains, wherein the VL domain consists of SEQ ID NO: 48, and wherein the VH domain consists of SEQ ID NO: 50, and
   (b) a retention sequence comprising SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv ScFv **(CD7_142.24)** comprising a VL and a VH domains, wherein the VL domain consists of SEQ ID NO: 56, and wherein the VH domain consists of SEQ ID NO: 57,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26; and
- a second nucleotide sequence encoding for a CD7-trap that comprises:
   (a) a ScFv **(CD7_142.9)** comprising a VL and a VH domains, wherein the VL domain consists of SEQ ID NO: 48, and wherein the VH domain consists of SEQ ID NO: 50, and
   (b) a retention sequence comprising SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising a VL and a VH domain, wherein the VL domain consists of SEQ ID NO: 20, and wherein the VH domain consists of SEQ ID NO: 21,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26, and
- a second nucleotide sequence encoding for a CD7-trap that comprises:
   (a) a ScFv **(CD7_124.1D1)** comprising a VL and a VH domains, wherein the VL domain consists of SEQ ID NO: 1, and wherein the VH domain consists of SEQ ID NO: 2, and
   (b) a retention sequence that comprises SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising a VL and a VH domain, wherein the VL domain consists of SEQ ID NO: 56, and wherein the VH domain consists of SEQ ID NO: 57,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26, and
- a second nucleotide sequence encoding for a CD7-trap that comprises:
   (a) a ScFv **(CD7_124.1D1)** comprising a VL and a VH domains, wherein the VL domain consists of SEQ ID NO: 1, and wherein the VH domain consists of SEQ ID NO: 2, and
   (b) a retention sequence that comprises SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising or consisting of SEQ ID NO: 11 or 61, preferably SEQ ID NO: 11,
   (d) a transmembrane domain comprising SEQ ID NO: 24,
   (c) an intracellular signaling domain comprising SEQ ID NO: 25, and
   (d) a costimulatory signaling domain comprising SEQ ID NO: 26; and
- a second nucleotide sequence encoding for a CD7-trap that comprises:
   (a) a ScFv **(CD7_142.9)** comprising or consisting of SEQ ID NO: 52, and
   (b) a retention sequence comprising SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising or consisting of SEQ ID NO: 11 or 61, preferably SEQ ID NO: 11,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26; and
- a second nucleotide sequence encoding for a CD7-trap that comprises:
   (a) a ScFv **(CD7_124.1D1)** comprising or consisting of SEQ ID NO: 3, and
   (b) a retention sequence comprising SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising or consisting of SEQ ID NO: 11 or 61, preferably SEQ ID NO: 11,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26; and
- a second nucleotide sequence encoding for a CD7-trap that comprises:
   (a) a ScFv **(CD7_142.9)** comprising or consisting of SEQ ID NO: 62, and
   (b) a retention sequence comprising SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises:
   (a) an extracellular domain comprising a CD7 targeting moiety that is a ScFv **(CD7_142.24)** comprising or consisting of SEQ ID NO: 11 or 61, preferably SEQ ID NO: 11,
   (b) a transmembrane domain comprising SEQ ID NO: 24,
   (c) an intracellular signaling domain comprising SEQ ID NO: 25, and
   (d) a costimulatory signaling domain comprising SEQ ID NO: 26; and
- a second nucleotide sequence encoding for a CD7-trap that comprises:
   (a) a ScFv **(CD7_124.1D1)** comprising or consisting of SEQ ID NO: 60, and
   (b) a retention sequence comprising SEQ ID NO: 30 [AEKDEL],
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises, consists or consists essentially of SEQ ID NO: 12 or 66 (**CD7 142.24**), or a sequence that has at least 85%, 86%, 87%, 88%, 89% 90%, 91%, 92%, 93%, 94%, 95%, 96%, 97%, 98%, 99% or 100% sequence identity to SEQ ID NO: 12 or 66, and
- a second nucleotide sequence encoding for a CD7-trap protein that comprises, consists or consists essentially of SEQ ID NO: 63 **(CD7_142.9),** or a sequence that has at least 85%, 86%, 87%, 88%, 89% 90%, 91%, 92%, 93%, 94%, 95%, 96%, 97%, 98%, 99% or 100% sequence identity to SEQ ID NO: 63,
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises, consists or consists essentially of SEQ ID NO: 12 or 66 **(CD7 142.24**), or a sequence that has at least 85%, 86%, 87%, 88%, 89% 90%, 91%, 92%, 93%, 94%, 95%, 96%, 97%, 98%, 99% or 100% sequence identity to SEQ ID NO: 12 or 66, and
- a second nucleotide sequence encoding for a CD7-trap protein that comprises, consists or consists essentially of SEQ ID NO: 31 (**CD7_124.1 D1**), or a sequence that has at least 85%, 86%, 87%, 88%, 89% 90%, 91%, 92%, 93%, 94%, 95%, 96%, 97%, 98%, 99% or 100% sequence identity to SEQ ID NO: 31,
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises, consists or consists essentially of SEQ ID NO: 12 or 66 **(CD7 142.24),** or a sequence that has at least 85%, 86%, 87%, 88%, 89% 90%, 91%, 92%, 93%, 94%, 95%, 96%, 97%, 98%, 99% or 100% sequence identity to SEQ ID NO: 12 or 66, and
- a second nucleotide sequence encoding for a CD7-trap protein that comprises, consists or consists essentially of SEQ ID NO: 69 **(CD7_142.9),** or a sequence that has at least 85%, 86%, 87%, 88%, 89% 90%, 91%, 92%, 93%, 94%, 95%, 96%, 97%, 98%, 99% or 100% sequence identity to SEQ ID NO: 69,
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 that comprises, consists or consists essentially of SEQ ID NO: 12 or 66 **(CD7 142.24),** or a sequence that has at least 85%, 86%, 87%, 88%, 89% 90%, 91%, 92%, 93%, 94%, 95%, 96%, 97%, 98%, 99% or 100% sequence identity to SEQ ID NO: 12 or 66, and
- a second nucleotide sequence encoding for a CD7-trap protein that comprises, consists or consists essentially of SEQ ID NO: 67 **(CD7_124.1D1),** or a sequence that has at least 85%, 86%, 87%, 88%, 89% 90%, 91%, 92%, 93%, 94%, 95%, 96%, 97%, 98%, 99% or 100% sequence identity to SEQ ID NO: 67,
preferably wherein the first and second nucleotide sequences are connected by a nucleic acid sequence encoding for a 2A cleavage peptide, preferably a T2A cleavage peptide.

Preferably, the bicistronic CARCD7Trap nucleic acid molecule comprises, preferably in the 5' to 3' direction:
- a first nucleotide sequence encoding for a CAR anti-CD7 **(CD7_142.24)** that comprises SEQ ID NO: 12 or 66, preferably SEQ ID NO: 12, and
- a second nucleotide sequence encoding for a CD7-trap **(CD7_142.9)** that comprises SEQ ID NO: 63.

Preferably, the bicistronic CARCD7Trap nucleic acid molecule comprises, preferably in the 5' to 3' direction:
- a first nucleotide sequence encoding for a CAR anti-CD7 **(CD7_142.24)** that comprises SEQ ID NO: 12 or 66, preferably SEQ ID NO: 12, and
- a second nucleotide sequence encoding for a CD7-trap **(CD7_124.1D1)** that comprises SEQ ID NO: 31.

Preferably, the bicistronic CARCD7Trap nucleic acid molecule comprises, preferably in the 5' to 3' direction:
- a first nucleotide sequence encoding for a CAR anti-CD7 **(CD7_142.24)** that consists of SEQ ID NO: 12 or 66, preferably SEQ ID NO: 12, and
- a second nucleotide sequence encoding for a CD7-trap **(CD7_142.9)** that consists of SEQ ID NO: 63.

Preferably, the bicistronic CARCD7Trap nucleic acid molecule comprises, preferably in the 5' to 3' direction:
- a first nucleotide sequence encoding for a CAR anti-CD7 **(CD7_142.24)** that consists of SEQ ID NO: 12 or 66, preferably SEQ ID NO: 12, and
- a second nucleotide sequence encoding for a CD7-trap **(CD7_124.1D1)** that consists of SEQ ID NO: 31.

Preferably, the bicistronic CARCD7Trap nucleic acid molecule comprises or consists of SEQ ID NO: 72 (CARCD7 142.24trap) or a sequence that has at least 85%, 86%, 87%, 88%, 89% 90%, 91%, 92%, 93%, 94%, 95%, 96%, 97%, 98%, 99% or 100% sequence identity to SEQ ID NO: 72. Preferably, the bicistronic CARCD7Trap nucleic acid molecule encodes for a protein that comprises or consists of SEQ ID NO: 73 (CARCD7 142.24trap) or a sequence that has at least 85%, 86%, 87%, 88%, 89% 90%, 91%, 92%, 93%, 94%, 95%, 96%, 97%, 98%, 99% or 100% sequence identity to SEQ ID NO: 73.

Preferably, the bicistronic CARCD7Trap nucleic acid comprises or consists of SEQ ID NO: 74 (CARCD7 142.24trap) or a sequence that has at least 85%, 86%, 87%, 88%, 89% 90%, 91%, 92%, 93%, 94%, 95%, 96%, 97%, 98%, 99% or 100% sequence identity to SEQ ID NO: 74. Preferably, the bicistronic CARCD7Trap nucleic acid molecule encodes for a protein that comprises or consists of SEQ ID NO: 75 (CARCD7 142.24trap) or a sequence that has at least 85%, 86%, 87%, 88%, 89% 90%, 91%, 92%, 93%, 94%, 95%, 96%, 97%, 98%, 99% or 100% sequence identity to SEQ ID NO: 75.

In some embodiments, the nucleic acid molecule, including the bicistronic CARCD7Trap nucleic acid molecule, is suitable for transducing a cell. In some embodiments, the nucleic acid, including the bicistronic nucleic acid molecule is suitable for transducing a T cell or a NK cell for use in adoptive immunotherapy.

In some embodiments, the nucleic acid molecule, including the bicistronic CARCD7Trap nucleic acid molecule, is codon optimized for expression in mammalian cells. Codon optimization methods are known in the art.

The nucleic acid of the present invention molecule, including the bicistronic CARCD7Trap nucleic acid molecule, may be comprised in a γ-retroviral or lentiviral vector which can be used to transduce or transform a T cell. The nucleic acid molecule may also be inserted into a cell through the use of DNA transposons, RNA transfection or genome editing techniques such as TALEN, ZFN and CRISPR/Cas9.

### Cells of the invention

In a **fifth aspect**, the present invention provides a cell or a plurality of cells, also called herein the cell or cells of the present invention, comprising and/or expressing one or more of the CD7 targeting moieties, the CARs, the CD7-traps of the first, second and third aspect; the nucleic acid molecule, including the bicistronic CARCD7Trap nucleic acid molecule, of the fourth aspect, or any of their embodiments. In some embodiments, the cell is a natural killer cell (NK), a NK/T cell, a monocyte, a macrophage, or a dendritic cell.

Preferably, the cell is a T-cell. In some embodiments, the cell is a naive T cell, memory stem T cell or central memory T cell. It is currently thought that these cells are better suited for adaptive immunotherapy. Preferably, the cell is a T cell and comprises and/or expresses the CARs defined above (also called herein CAR-T cell).

In some embodiments, the cell is an autologous T cell. The term "autologous cell" refers to a cell obtained from the same patient that is to be treated using any one of the methods of the present invention.

In some embodiments, the cell is an allo-tolerant T cell. The term "allo-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art.

In some embodiments, the T cell is a CD3-positive (CD3+) T cell. In some embodiments, the T cell is T a CD8+ T cell. In some instances, the cell is a human cell. In some embodiments, the cell is a lymphoid precursor, embryonic stem cell or an induced pluripotent stem cell with the capacity to differentiate into a mature T cell. In some embodiments, the cell is a CD34-positive (CD34+) cell, preferably selected from the list consisting of HPCs or cord blood cells.

The cells encompassed by the present invention are preferably deficient in an antigen to which the chimeric antigen receptor specifically binds and are therefore fratricide-resistant. In some embodiments, the CD7 protein of the cell, preferably T cell, is modified such that the anti-CD7 CAR of the present invention no longer specifically binds the modified antigen. For example, the epitope of the CD7 protein recognized by the anti-CD7 CAR of the present invention may be modified by one or more amino acid changes (e.g., substitutions or deletions) or the epitope may be deleted from the CD7 protein. In other embodiments, expression of the CD7 protein is reduced in the cell, preferably T cell, by at least 50%, at least 60%, at least 70%, at least 80%, at least 90% or more in comparison to a wild-type cell. Methods for decreasing the expression of CD7 protein are known in the art and include, but are not limited to, modifying or replacing the promoter operably linked to the nucleic acid sequence encoding the CD7 protein. In still other embodiments, the cell, preferably T cell, is modified such that the CD7 protein is not expressed, e.g., by deletion or disruption of the gene encoding the CD7 protein. Thus, in some embodiments, the T cell is a naturally occurring CD7-negative T cell. In some embodiments, the T cell is a genetically modified to avoid CD7 expression in the T cell surface. In some embodiments, the T cell is CD7- negative and CD3-positive.

Methods for genetically modifying a cell to be deficient in a protein are well known in art. In an exemplary embodiment, CRISPR/cas9 gene editing can be used to modify a T cell to be deficient in CD7 protein. In an embodiment, the cell is a T cell that is modified to reduce or remove the expression of CD7 endogenous protein, and subsequently is transduced or transfected with one or more of the CARs defined above.

Thus, in some embodiments, the cell, preferably the T cell, is characterized by having a downregulation of CD7 expression in its surface, which can be achieved according to a variety of other known methods including, for example, gene editing methods with meganucleases, TALEN, CRISPR/Cas9, and zinc finger nucleases. Thus, in certain embodiments, the cell further comprises a modified CD7 gene, which modification renders the human CD7 gene or protein non-functional. By way of example, the cell of the present invention further comprises a modified (e.g., nonfunctional) CD7 gene (modified using, e.g., meganucleases, TALEN, CRISPR/Cas9, zinc finger nucleases, Base-editors or Prime-editors) that prevents or reduces expression of CD7, and/or otherwise impairs (e.g., structurally) the CD7 protein from being recognized by an anti-CD7 CAR. Methods of modifying gene expression using such methods are readily available and well-known in the art. Preferably, the cell, preferably the T cell, is knocked out for CD7 expression using genome editing techniques, such as Cas9/CRISPR.

Preferably, the cell, preferably the T cell, comprises the CD7-trap protein of the present invention, and thus has a downregulated expression of human CD7 in its surface. Preferably, the cell is a CD34+ cell and comprises the CD7-trap protein defined above, and thus has a downregulated expression of human CD7 in its surface. Preferably, the cell is a CD3+ cell and comprises the CD7-trap protein defined above, and thus has a downregulated expression of human CD7 in its surface.

Preferably, the cell comprises and/or expresses the CD7-trap of the invention and/or the CAR anti-CD7 of the invention as defined in any of the embodiments above. In an embodiment, the cell comprises and/or expresses the CD7-trap of the invention and/or the CAR anti-CD7 of the invention as defined in any of the embodiments above.

In some embodiments, the cell is a T cell comprising i) a nucleic acid encoding for a CD7-targeting moiety, preferably a CAR anti-CD7, as defined above, and/or ii) a nucleic acid encoding for the CD7-trap as defined above. In some embodiments, the cell is a T cell and it expresses i) a CD7-targeting moiety, preferably a CAR anti-CD7, as defined in the first or second aspects, and ii) the CD7-trap as defined above. In some embodiments, the cell is a T cell and it expresses the bicistronic CARCD7Trap nucleic acid molecule of the invention.

Preferably, the cell is a T cell comprising i) a nucleic acid encoding for SEQ ID NO: 12 **(CD7 CAR 142.24)** and/or ii) a nucleic acid encoding for SEQ ID NO: 31 **(CD7 Trap 124.1D1).** Preferably, the cell is a T cell comprising i) a nucleic acid encoding for SEQ ID NO: 12 **(CD7 CAR 142.24)** and/or ii) a nucleic acid encoding for SEQ ID NO: 63 **(CD7 Trap 142.9).**

Preferably, the cell is a T cell comprising i) a nucleic acid encoding for SEQ ID NO: 66 **(CD7 CAR 142.24)** and/or ii) a nucleic acid encoding for SEQ ID NO: 31 **(CD7 Trap 124.1D1).** Preferably, the cell is a T cell comprising i) a nucleic acid encoding for SEQ ID NO: 66 **(CD7 CAR 142.24)** and/or ii) a nucleic acid encoding for SEQ ID NO: 63 **(CD7 Trap 142.9).**

The invention thus also provides in the fifth aspect a cell, preferably a T cell, comprising a chimeric antigen receptor that specifically binds CD7 antigen according to the first aspect or any of its embodiments, wherein the T cell is deficient in CD7 surface expression. The deficiency in CD7 surface expression may be resulted from (a) modification of CD7 expressed by the cell such that the chimeric antigen receptor no longer specifically binds the modified CD7, (b) modification of the cell such that expression of the CD7 protein in the cell's surface is reduced in the T cell by at least 50%, at least 60%, at least 70%, at least 80%, at least 90% or more, (c) modification of the cell such that CD7 is not expressed (e.g., by deletion or disruption of the gene encoding CD7), or (d) selection of T cells that do not express CD7 at the time of apheresis. In (b) the modification of the cell is achieved by comprising in the cell the CD7-trap according to the first aspect or any of its embodiments.

### Pharmaceutical composition of the invention

In **a sixth aspect,** the present invention provides a pharmaceutical composition, also called herein the pharmaceutical composition of the invention, comprising a plurality of cells of the present invention and a pharmaceutically acceptable carrier or diluent.

A pharmaceutical composition as described herein may also contain other substances. These substances include, but are not limited to, cryoprotectants, surfactants, antioxidants, and stabilizing agents. The term "cryoprotectant" as used herein, includes agents which provide stability to the CARTs against freezing-induced stresses. Nonlimiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In some embodiments, the pharmaceutical composition comprises other substances, such as cancer growth blockers, that improve the therapeutic effect of composition.

Preferably, the pharmaceutical composition further comprises Dasatinib. Dasatinib is a compoung described in WO Patent Publication No. 00/62778, U.S. Patent Application No. 2005/0215795 and U.S. Patent No. 6,596,746, and it is chemically defined as N-(2-chloro-6-methylphenyl)-2-[[6-[4-(2-hydroxyethyl)-1-piperazinyl]-2-methyl-4-pyrimidinyl]amino]-5-thiazolecarboxamide. Addition of Dasatinib into the manufacturing product inhibits CAR-mediated activation and prevents or reduces CAR-T cell fratricide.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum or other human serum components.

### Combination therapy of the invention

In a **seventh aspect,** the present invention provides a combination therapy. "Combination therapy", "in combination with" or "in conjunction with" as used herein denotes any form of concurrent, parallel, simultaneous, sequential or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations or unit dosage forms) or separately (e.g., on the same day or on different days and in any order as according to an appropriate dosing protocol for the separate compositions, formulations or unit dosage forms) in a manner and dosing regimen prescribed by a medical care taker or according to a regulatory agency. In general, each treatment modality will be administered at a dose and/or on a time schedule determined for that treatment modality. Optionally, three or more modalities may be used in a combination therapy.

In an embodiment, the combination therapy comprises administration of at least two different CD7-targeting moieties, preferably the CD7-targeting moiety of the second aspect, or any of its embodiments, combined with the CD7-targeting moieties of the first or third aspects, or any of their embodiments. More preferably, the combination therapy comprises administering:
i) the CAR-anti CD7 of the invention, or a nucleic acid encoding thereof, and
ii) the CD7-trap protein of the invention, or a nucleic acid encoding thereof.

More preferably, the combination therapy comprises administering:
i) a CAR that comprises an extracellular domain comprising the CD7 targeting moiety of the second aspect or any of its embodiments **(CD7_142.24),** or a nucleic acid encoding thereof, and
ii) a CD7-trap protein that comprises the CD7-targeting moiety of the first aspect or any of its embodiments **(CD7_124.1D1),** or a nucleic acid encoding thereof.

Preferably, the combination therapy comprises administering:
i) a CAR anti-CD7, or a nucleic acid encoding thereof, that comprises:
   (a) an extracellular domain comprising a CD7 targeting moiety that is a ScFv **(CD7_142.24)** comprising a VL and a VH domain, wherein the VL domain comprises a sequence having at least 85% sequence identity to SEQ ID NO: 20, wherein the CDRs comprised in said VL are maintained constant and consist of SEQ ID NOs: 14, 15, and 16; and wherein the VH domain comprises a sequence having at least 85% sequence identity to SEQ ID NO: 21, wherein the CDRs comprised in said VH are maintained constant and consist of SEQ ID NOs: 17, 18, and 19,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_124.1D1)** comprising a VL and a VH domains, wherein the VL domain comprises a sequence having at least 85% sequence identity to SEQ ID NO: 1, wherein the CDRs comprised in said VL are maintained constant (i.e., non variable) and consists of SEQ ID NOs: 4, 5, and 6; and wherein the VH domain comprises a sequence having at least 85% sequence identity to SEQ ID NO: 2, wherein the CDRs comprised in said VH are maintained constant and consist of SEQ ID NOs: 7, 8, and 9, and
   (b) a retention sequence that comprises SEQ ID NO: 30 [AEKDEL].

Preferably, the combination therapy comprises administering:
i) a CAR anti-CD7, or a nucleic acid encoding thereof, that comprises:
   (a) an extracellular domain comprising a CD7 targeting moiety that is a ScFv ScFv **(CD7_142.24)** comprising a VL and a VH domains, wherein the VL domain comprises SEQ ID NO: 20, and wherein the VH domain comprises SEQ ID NO: 21,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_124.1D1)** comprising a VL and a VH domains, wherein the VL domain comprises SEQ ID NO: 1, and wherein the VH domain comprises SEQ ID NO: 2, and
   (b) a retention sequence that comprises, consists or consists essentially of SEQ ID NO: 30 [AEKDEL].

Preferably, the combination therapy comprises administering:
i) a CAR anti-CD7, or a nucleic acid encoding thereof, that comprises:
   (a) an extracellular domain comprising a CD7 targeting moiety that is a ScFv ScFv **(CD7_142.24)** comprising a VL and a VH domains, wherein the VL domain consists of SEQ ID NO: 20, and wherein the VH domain consists of SEQ ID NO: 21,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_124.1D1)** comprising a VL and a VH domains, wherein the VL domain consists of SEQ ID NO: 1, and wherein the VH domain consists of SEQ ID NO: 2, and
   (b) a retention sequence that comprises, consists or consists essentially of SEQ ID NO: 30 [AEKDEL].

Preferably, the combination therapy comprises administering:
i) a CAR anti-CD7, or a nucleic acid encoding thereof, that comprises:
   (a) an extracellular domain comprising a CD7 targeting moiety that is a ScFv **(CD7_142.24)** comprising or consisting of SEQ ID NO: 11 or 61, preferably SEQ ID NO: 11,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv comprising or consisting of SEQ ID NO: 3 **(CD7_124.1D1),** and
   (b) a retention sequence that comprises SEQ ID NO: 30 [AEKDEL].

Preferably, the combination therapy comprises administering:
i) a CAR comprising SEQ ID NOs: 12 or 66 **(CD7_142.24),** or a nucleic acid encoding thereof, and
ii) a CD7-trap protein comprising SEQ ID NO: 31 **(CD7_124.1D1),** or a nucleic acid encoding thereof.

Preferably, the combination therapy comprises administering:
i) a CAR consisting of SEQ ID NOs: 12 or 66 **(CD7_142.24),** or a nucleic acid encoding thereof, and
ii) a CD7-trap protein consisting of SEQ ID NO: 31 **(CD7_124.1D1),** or a nucleic acid encoding thereof.

More preferably, the combination therapy comprises administering:
i) a CAR that comprises an extracellular domain comprising the CD7 targeting moiety of the second aspect or any of its embodiments **(CD7_142.24),** or a nucleic acid encoding thereof, and
ii) a CD7-trap protein that comprises the CD7-targeting moiety of the third aspect or any of its embodiments **(CD7_142.9),** or a nucleic acid encoding thereof.

Preferably, the combination therapy comprises administering:
i) a CAR anti-CD7, or a nucleic acid encoding thereof, that comprises:
   (a) an extracellular domain comprising a CD7 targeting moiety that is a ScFv **(CD7_142.24)** comprising a VL and a VH domain, wherein the VL domain comprises a sequence having at least 85% sequence identity to SEQ ID NO: 20, wherein the CDRs comprised in said VL are maintained constant and consist of SEQ ID NOs: 14, 15, and 16; and wherein the VH domain comprises a sequence having at least 85% sequence identity to SEQ ID NO: 21, wherein the CDRs comprised in said VH are maintained constant and consist of SEQ ID NOs: 17, 18, and 19,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_142.9)** comprising a VL and a VH domains, wherein the VL domain comprises a sequence having at least 85% sequence identity to SEQ ID NO: 48, wherein the CDRs comprised in said VL are maintained constant (i.e., non variable) and consists of SEQ ID NOs: 42, 43, and 44; and wherein the VH domain comprises a sequence having at least 85% sequence identity to SEQ ID NO: 50, wherein the CDRs comprised in said VH are maintained constant and consist of SEQ ID NOs: 45, 46, and 47, and
   (b) a retention sequence that comprises SEQ ID NO: 30 [AEKDEL].

Preferably, the combination therapy comprises administering:
i) a CAR anti-CD7, or a nucleic acid encoding thereof, that comprises:
   (a) an extracellular domain comprising a CD7 targeting moiety that is a ScFv ScFv **(CD7_142.24)** comprising a VL and a VH domains, wherein the VL domain comprises SEQ ID NO: 20, and wherein the VH domain comprises SEQ ID NO: 21,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_142.9)** comprising a VL and a VH domains, wherein the VL domain comprises SEQ ID NO: 48, and wherein the VH domain comprises SEQ ID NO: 50, and
   (b) a retention sequence that comprises SEQ ID NO: 30 [AEKDEL].

Preferably, the combination therapy comprises administering:
i) a CAR anti-CD7, or a nucleic acid encoding thereof, that comprises:
   (a) an extracellular domain comprising a CD7 targeting moiety that is a ScFv ScFv **(CD7_142.24)** comprising a VL and a VH domains, wherein the VL domain consists of SEQ ID NO: 20, and wherein the VH domain consists of SEQ ID NO: 21,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_142.9)** comprising a VL and a VH domains, wherein the VL domain consists of SEQ ID NO: 48, and wherein the VH domain consists of SEQ ID NO: 50, and
   (b) a retention sequence that comprises SEQ ID NO: 30 [AEKDEL].

Preferably, the combination therapy comprises administering:
i) a CAR anti-CD7, or a nucleic acid encoding thereof, that comprises:
   (a) an extracellular domain comprising a CD7 targeting moiety **(CD7_142.24)** that is a ScFv comprising or consisting of SEQ ID NO: 11 or 61, preferably SEQ ID NO: 11,
   (b) the transmembrane domain of CD8;
   (c) the intracellular domain of CD3ζ; and
   (d) the intra-cellular domain of CD137; and
ii) a CD7-trap protein, or a nucleic acid encoding thereof, that comprises:
   (a) a ScFv **(CD7_142.9)** comprising or consisting of SEQ ID NO: 52, and
   (b) a retention sequence that comprises SEQ ID NO: 30 [AEKDEL].

Preferably, the combination therapy comprises administering:
i) a CAR comprising SEQ ID NOs: 12 or 66 **(CD7_142.24),** or a nucleic acid encoding thereof, and
ii) a CD7-trap protein **(CD7_142.9)** comprising SEQ ID NO: 63, or a nucleic acid encoding thereof.

Preferably, the combination therapy comprises administering:
i) a CAR consisting of SEQ ID NOs: 12 or 66 **(CD7_142.24),** or a nucleic acid encoding thereof, and
ii) a CD7-trap protein **(CD7_142.9)** consisting of SEQ ID NO: 63, or a nucleic acid encoding thereof.

Preferably, the combination therapy comprises administering the nucleic acid according to the fourth aspect, or any of its embodiments, including bicistronic CARCD7Trap nucleic acid molecule of the invention. Preferably, the combination therapy comprises administering the cell or cells according to the fifth aspect, or any of its embodiments. Preferably, the combination therapy comprises administering a cell or a plurality of cells, that comprise:
i) the CAR-anti CD7 of the invention, or a nucleic acid encoding thereof, and
ii) the CD7-trap protein of the invention, or a nucleic acid encoding thereof.

Preferably, the combination therapy comprises administering the composition of the sixth aspect, or any of its embodiments.

In the combination therapy, items i) or ii) may be administered simultaneously or sequentially. However, it is preferred that i) and ii) are administered simultaneously to the subject in need thereof. Preferably, items i) and ii) in the combination therapy defined herein are administered comprised or vehiculized in a cell.

In one embodiment, the combination therapy comprises administering to the subject in need thereof a CAR anti-CD7, and subsequently or simultaneously administering a CD7-trap, both directed to target human CD7 antigen. This combination of CAR anti-CD7 with CD7-trap is particularly useful to treat a CD7-positive cancer while reducing fratricide.

### Methods of treatment

In an **eighth aspect,** the present invention provides a CD7 targeting moiety and/or the CAR and/or the CD7-trap according to the first, second or third aspects, a nucleic acid according to the fourth aspect (including the bicistronic CARCR7trap nucleic acid molecule of the invention), a cell according to the fifth aspect, and/or a pharmaceutical composition according to the sixth aspect, and the combination therapy according to the seventh aspect, for use as a medicament or in therapy. In a preferred embodiment, the use is in a method of treating a CD7-positive cancer comprising administering the CD7 targeting moiety and/or the CAR and/or the CD7-trap of the first, second or third aspects, the nucleic acid of the fourth aspect, the cell of the fifth aspect and/or the pharmaceutical composition of the sixth aspect to a patient in need thereof. In some embodiments, the CD7-positive cancer is a T-cell malignancy, such as lymphoma or leukemia. Preferably, the CD7-positive cancer is T cell leukemia or T cell lymphoma, such a T-cell acute lymphoblastic leukemia, T-cell prolymphocytic leukemia, T- cell large granular lymphocytic leukemia, enteropathy-associated T-cell lymphoma, hepatosplenic T-cell lymphoma, subcutaneous panniculitis-like T-cell lymphoma, mycosis fungoides, Sezary syndrome, primary cutaneous gamma-delta T-cell lymphoma, peripheral T- cell lymphoma not otherwise specified, angioimmunoblastic T-cell lymphoma, anaplastic large cell lymphoma. In certain embodiments, the T cell malignancy is early T-cell progenitor acute lymphoblastic leukemia (ETP-ALL). Most preferably, the CD7-positive cancer is cortical T-cell acute lymphoblastic leukemia (T-ALL). In some embodiments, the CD7-positive cancer is relapsed/refractory cortical T-cell acute lymphoblastic leukemia.

In general, the relapse of leukemia can manifest several months or years after the initial remission; however, most relapses occur within two years after the initial treatment. Refractoriness is a term that implies that the patient has no longer responded to at least one therapy strategy after a relapse. There is a broad consensus in first-line trials for ALL, specifically in adults that a relapse is defined as "detection of more than 5% of blast cells in the bone marrow after a previous achievement of complete remission (CR) or unequivocal demonstration of extramedullary leukemia participation" (see Gökbuget (2017)). The European Working Group on Adult ALL (EWALL) has documented this statement in a consensus recommendation, (see Dohner (2010)) with the additional explanation that "in the case of 5 to 20% of cell blasts at some stage during the intensive treatment phase and / or during regeneration, the evaluation of the bone marrow should be repeated one week later to distinguish among bone marrow relapse and regeneration phenomenon. The cited definition is based on international recommendations for outcome parameters in acute myeloid leukemia (see Cheson (2003) and Chantepie (213)); that has been extrapolated to several subtypes of ALL, as in the case of T-ALL.

The CAR-T cells and/or the cells expressing or comprising the CD7-Trap protein of the present invention may be used to target malignant T-cells that overexpress CD7. The cells, preferably T cells, expressing or comprising the CD7-Trap protein of the present invention may be used to reduce fraticide. Thus, disclosed are methods of treating T cell acute lymphoblastic leukemia (T-ALL) comprising administering an effective amount of a CD7 targeting moiety, a nucleic acid, cell (preferably T cell) genetically modified to express one or more of the disclosed CAR anti-CD7 and/or CD7-traps polypeptides, or pharmaceutical composition, to a subject in need thereof. For example, disclosed are methods of treating T cell acute lymphoblastic leukemia (T-ALL) comprising administering an effective amount of a T cell genetically modified to express a CAR polypeptide comprising a CD7 targeting binding domain, a hinge and transmembrane domain, and an intracellular signaling domain, as described above. For example, disclosed are methods of treating T cell acute lymphoblastic leukemia (T-ALL) comprising administering an effective amount of a T cell genetically modified to express a CAR polypeptide comprising a CD7-trap protein, as described above.

In some embodiments, the CD7 targeting moiety, the CARs, the cell expressing CD7-traps, the nucleic acid, the cell and/or pharmaceutical composition of the invention is administered intravenously, intraperitoneally, into the bone marrow, into the lymph node, and /or into cerebrospinal fluid.

In another embodiment, the present invention provides a method of killing a malignant T cell, the method comprising contacting the malignant T cell with an effective amount of a T cell comprising a chimeric antigen receptor (CAR-T cell), wherein the CAR-T cell is deficient in an antigen to which the chimeric antigen receptor specifically binds, and wherein the chimeric antigen receptor specifically binds an antigen expressed on a malignant cell. In various embodiments, the malignant cell is a malignant T cell. In further embodiments, the antigen is CD7.

Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject. Hence, the method and uses thereof also include the administration of two (or more) different treatments that are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered. Preferably, the method comprises administering the CD7 targeting moieties, the CARs, the CD7-traps, the nucleic acid, the cells or pharmaceutical composition of the invention

In an embodiment, the CD7-targeting moiety and/or the CAR-expressing cell described herein is administered with at least one additional therapeutic agent simultaneously, in the same or in separate compositions, or sequentially. For sequential administration, the CD7 targeting moiety and/or the CAR-expressing cell described herein can be administered first, and the additional agent can be administered second, or the order of administration can be reversed. In some embodiments, the additional agent is Dasatinib.

In an embodiment, the uses or methods of treatment comprises a first step of a) providing a population of blood cells, b) isolating CD7-negative (CD7-) cells from said population of blood cells, c) modifying the CD7- cells to express the CARs of the present invention, and d) administering said CD7- cells expressing the CARs of the present invention to a patient in need thereof. Preferably, the population of blood cells is obtained from the same patient to which the cells are administered in step d) (autologous therapy). Preferably, the patient in need thereof is a human, most preferably a human having or suffering from a CD7-positive cancer.

In some embodiments, the CD7 positive cancer is relapsed/refractory T cell acute lymphoblastic leukemia (T-ALL). In general, the relapse of T-ALL can manifest several months or years after the initial remission; however, most relapses occur within two years after the initial treatment. Refractoriness is a term that implies that the patient has no longer responded to at least one therapy strategy after a relapse.

In some embodiments, the patient to be treated with the method of the present invention is in complete or near-complete remission after treatment with another therapy. It may be preferable desirable to decrease the tumor burden before using the methods of the present invention because since there are several alternative effector T-cells in cases of patients with highly active relapsed/refractory cancer. In some embodiments, the patient to be treated with the method of the present invention has previously been treated with another therapy which resulted in a partial response, complete response, stable disease, decrease in progressive disease, reduced time to tumor progression or any combination thereof.

In some embodiments, the method comprises further administering an immune checkpoint. In a further embodiment, the method comprises further administering an immune checkpoint inhibitor and/or an IAP inhibitor. In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely. In some embodiments, the therapeutic agent can be, but is not limited to, conventional chemotherapy including but not limited to alkylating agents, antimetabolites, anti -microtubule agents, topoisomerase inhibitors, and cytotoxic antibiotics; high- dose chemotherapy including but not limited to high-dose Melphalan chemotherapy with or without stem cell transplant; proteasome inhibitors such as, but not limited to, bortezomib, ixazomib, and carfilzomib; immunomodulatory agents (IMiDS) such as, but not limited to, thalidomide, lenalidomide, and pomalidomide; histone deacetylase (HDAC) inhibitors such as, but not limited to panobinostat; monoclonal antibodies such as, but not limited to, daratumumab, isatuximab, or elotuzumab; bispecific antibodies; and immune checkpoint inhibitors such as, but not limited to, ipilimumab, nivolumab, and pembrolizumab.

### Kits

In a **ninth aspect,** the present invention provides kits and their use according to any of the previous aspects. The materials described above as well as other materials can be packaged together in any suitable combination as a kit useful for performing, or aiding in the performance of, the disclosed method. It is useful if the kit components in a given kit are designed and adapted for use together in the disclosed method. Also disclosed are kits comprising one or more of the vectors disclosed herein.

### SEQUENCE LISTING

### ScFv CD7 124.1D1:

- CD7 124.1D1 VL CDRs:
   LCDR1: SEQ ID NO: 4: RASQSIGTSIH
   LCDR2: SEQ ID NO: 5: YASESIS
   LCDR3: SEQ ID NO: 6: QQSISWPLT
- CD7 124.1D1 VH CDRs:
   HCDR1: SEQ ID NO: 7 SDYAWN
   HCDR2: SEQ ID NO: 8 YISYSGSTSYNPSLKS
   HCDR3: SEQ ID NO: 9 SQLGRWAMDY
- SEQ ID NO: 1: CD7 124.1D1 VL amino acid sequence (murine): 
DILLTQSPAILSVSPGERVSFSCRASQSIGTSIHWYQQRTNDSPRLLIKYASESISGIPS
RFSGSGSGTDFTLNINSVESEDIADYYCQQSISWPLTFGPGTKLELK
- SEQ ID NO: 54: CD7 124.1D1 VL amino acid sequence (humanized): 
EIVLTQSPAILSLSPGERVTFSCRASQSIGTSIHWYQQKTNDSPRLLIKYASESISGIPSR
FSGSGSGTDFTLNISSLESEDIADYYCQQSISWPLTFGPGTKLEIK
- SEQ ID NO: 38: CD7 124.1D1 VL nucleotide sequence (murine): 
GACATTCTGCTTACACAGAGTCCCGCCATCCTGAGCGTCTCTCCAGGCGAGCGG
GTCTCCTTCAGTTGTCGCGCTAGTCAGAGCATTGGAACCTCCATCCATTGGTATCA
GCAGAGAACGAATGATAGCCCCCGGCTGCTGATAAAGTATGCATCCGAGTCCATA
TCAGGCATCCCTTCTAGGTTTTCAGGAAGCGGATCTGGCACTGACTTTACGTTGA
ACATCAACAGCGTGGAATCCGAGGACATCGCTGATTATTATTGCCAACAGTCTATC
TCCTGGCCGCTTACATTCGGCCCAGGAACAAAGTTAGAGCTGAAG
- SEQ ID NO: 2: CD7 124.1D1 VH amino acid sequence (murine) 
DVQLQESGPGLVKPSQSLSLTCTVTGYSITSDYAWNWIRQFPGNKLEWLGYISYSGS
TSYNPSLKSRISITRDTSKNQFFLQLNSVTTEDTATYYCAKSQLGRWAMDYWGQGTS
VTVSS
- SEQ ID NO: 55: CD7 124.1D1 VH amino acid sequence (humanized): 
EVQLLEQSGPGLVKPSQSLSLTCSVTGYSITSDYAWNWIRQFPGNKLEWIGYISYSGS
TSYNPSLKSRVTITRDTSQNQYYLQLNSVTTEDTATYYCAKSQLGRWAMDYWGQGT
TVTVSS
- SEQ ID NO: 39: CD7 124.1D1 VH nucleotide sequence (murine) 
GATGTGCAGCTGCAAGAAAGTGGCCCAGGTCTTGTGAAGCCTAGTCAGAGCCTG
AGCCTAACCTGTACAGTGACCGGTTACAGCATTACTTCGGATTACGCATGGAACT
GGATTCGTCAATTCCCCGGTAACAAACTGGAGTGGCTCGGATACATCAGCTATTC
CGGGTCCACATCCTACAATCCCTCTTTGAAATCCAGAATATCTATTACACGAGACA
CCAGCAAGAATCAATTCTTTCTGCAGTTGAATTCAGTGACCACCGAAGACACCGC
 
CACTTACTACTGCGCGAAATCACAGCTGGGGAGATGGGCCATGGATTACTGGGG
GCAGGGGACTAGCGTTACTGTTTCTTCA
- SEQ ID NO: 3: CD7 124.1D1 Signal peptide + VL + linker + VH aa sequence (murine) 
MALPVTGLLLSLGLLLHAARPDILLTQSPAILSVSPGERVSFSCRASQSIGTSIHWYQQ
RTNDSPRLLIKYASESISGIPSRFSGSGSGTDFTLNINSVESEDIADYYCQQSISWPLTF
GPGTKLELKGGGGSGGGGSGGGGSGGGGSDVQLQESGPGLVKPSQSLSLTCTVTG
YSITSDYAWNWIRQFPGNKLEWLGYISYSGSTSYNPSLKSRISITRDTSKNQFFLQLNS
VTTEDTATYYCAKSQLGRWAMDYWGQGTSVTVSS
- SEQ ID NO: 60: CD7 124.1D1 Signal peptide + VL + linker + VH aa sequence (humanized)

MALPVTGLLLSLGLLLHAARPEIVLTQSPAILSLSPGERVTFSCRASQSIGTSIHWYQQK
TNDSPRLLIKYASESISGIPSRFSGSGSGTDFTLNISSLESEDIADYYCQQSISWPLTFG
PGTKLEIKGGGGSGGGGSGGGGSGGGGSEVQLLEQSGPGLVKPSQSLSLTCSVTG
YSITSDYAWNWIRQFPGNKLEWIGYISYSGSTSYNPSLKSRVTITRDTSQNQYYLQLN
SVTTEDTATYYCAKSQLGRWAMDYWGQGTTVTVSS

### ScFv CD7 142.24:

- CD7 142.24 VL CDRs:
   LCDR1: SEQ ID NO: 14: RASQNIGTSIH
   LCDR2: SEQ ID NO: 15: YASESMS
   LCDR3: SEQ ID NO: 16: QQSNRWPYT
- CD7 142.24 VH CDRs:
   HCDR1: SEQ ID NO: 17: HYGVN
   HCDR2: SEQ ID NO: 18: WINTNTGKPTYAEDFKG
   HCDR3: SEQ ID NO: 19: WSTTVVAPYFDV
- SEQ ID NO: 20: CD7 142.24 VL amino acid sequence (murine): 
DILLTQSPAILSVSPGERVSFSCRASQNIGTSIHWFQQRSNGSPSLLIKYASESMSGIP
SRFSGSGSGTDFTLSINSVEPEDIADYYCQQSNRWPYTFGGGTKLEIK
- SEQ ID NO: 56: CD7 142.24 VL amino acid sequence (humanized): 
EIVLTQSPAILSVSPGERVSFSCRASQNIGTSIHWYQQKTNGSPSLLIKYASESMSGIP
SRFSGSGSGTDFTLTINSLQPEDIADYYCQQSNRWPYTFGGGTKLELK
- SEQ ID NO: 40: CD7 142.24 VL nucleotide sequence (murine): 
GACATTCTGCTGACTCAGTCACCAGCCATCCTCTCAGTGTCACCTGGCGAGCGTG
TTTCATTCAGCTGTAGGGCTTCGCAAAATATCGGGACCAGCATCCACTGGTTCCA
GCAGAGATCTAACGGCTCGCCAAGCTTACTCATCAAATATGCGAGTGAGTCCATG
TCCGGCATCCCTTCACGCTTTTCCGGCTCTGGTTCTGGAACTGATTTTACCCTTAG
CATAAATAGTGTGGAGCCTGAAGATATAGCCGACTACTACTGCCAACAATCCAAC
CGGTGGCCCTACACATTTGGCGGTGGCACCAAACTTGAGATTAAG
- SEQ ID NO: 21: CD7 142.24 VH amino acid sequence (murine): 
QLVQSGPEVKKPGETVKISCKASGYSFTHYGVNWVKQAPGKGLKWMGWINTNTGKP
TYAEDFKGRFAFSLETSARTAYLQINNLKNEDTATYFCARWSTTVVAPYFDVWGAGT
TVTVSS
- SEQ ID NO: 57: CD7 142.24 VH amino acid sequence (humanized): 
QLVQSGPELKKPGETVKISCKASGYSFTHYGVNWVRQAPGQGLEWMGWINTNTGKP
TYAEDFKGRFAFSLDTSARTAYLQISSLKNEDTATYYCARWSTTVVAPYFDVWGAGT
SVTVSS
- SEQ ID NO: 41: CD7 142.24 VH nucleotide sequence (murine): 
CAGCTAGTGCAGTCTGGACCCGAAGTGAAAAAGCCTGGAGAGACAGTTAAGATTT
CCTGCAAAGCAAGTGGATATAGCTTCACACACTACGGTGTAAATTGGGTGAAGCA
GGCACCAGGCAAAGGGCTTAAGTGGATGGGGTGGATCAACACAAATACGGGCAA
GCCCACTTATGCCGAAGACTTTAAGGGGAGATTCGCCTTCTCTCTGGAAACGAGT
GCACGAACAGCTTACCTCCAGATTAACAATCTGAAAAACGAGGATACTGCTACATA
TTTCTGTGCTAGGTGGTCAACCACCGTCGTGGCCCCCTACTTTGACGTCTGGGGT
GCGGGGACTACCGTAACCGTCTCCTCC
- SEQ ID NO: 11: CD7 142.24 Signal peptide + VL + linker + VH aa sequence murine): 
MALPVTGLLLSLGLLLHAARPDILLTQSPAILSVSPGERVSFSCRASQNIGTSIHWFQQ
RSNGSPSLLIKYASESMSGIPSRFSGSGSGTDFTLSINSVEPEDIADYYCQQSNRWPY
 
TFGGGTKLEIKGGGGSGGGGSGGGGSGGGGSQLVQSGPEVKKPGETVKISCKASG
YSFTHYGVNWVKQAPGKGLKWMGWINTNTGKPTYAEDFKGRFAFSLETSARTAYLQI
NNLKNEDTATYFCARWSTTVVAPYFDVWGAGTTVTVSS
- SEQ ID NO: 61: CD7 142.24 Signal peptide + VL + linker + VH aa sequence (humanized):

MALPVTGLLLSLGLLLHAARPEIVLTQSPAILSVSPGERVSFSCRASQNIGTSIHWYQQ
KTNGSPSLLIKYASESMSGIPSRFSGSGSGTDFTLTINSLQPEDIADYYCQQSNRWPY
TFGGGTKLELKGGGGSGGGGSGGGGSGGGGSQLVQSGPELKKPGETVKISCKASG
YSFTHYGVNWVRQAPGQGLEWMGWINTNTGKPTYAEDFKGRFAFSLDTSARTAYLQ
ISSLKNEDTATYYCARWSTTVVAPYFDVWGAGTSVTVSS

### ScFv CD7 142.9

- CD7 142.9 VL CDRs:
   LCDR1: SEQ ID NO: 42: SARSSVSYMN
   LCDR2: SEQ ID NO: 43: GISNLAS
   LCDR3: SEQ ID NO: 44: QLRSSYSRT
- CD7 142.9 VH CDRs:
   HCDR1: SEQ ID NO: 45: NYWLG
   HCDR2: SEQ ID NO: 46: DFYPGSIYTNYNENFQG
   HCDR3: SEQ ID NO: 47: EGDYYGSGHFDY
- SEQ ID NO: 48: CD7 142.9 VL amino acid sequence (murine): 
EILLTQSPAIIAASPGEKVTITCSARSSVSYMNWYQQKSGSSPKIWIYGISNLASGVPAR
FSGSGSGTSFSFTINSMEAEDVAYYYCQLRSSYSRTFGGGTKLEIK
- SEQ ID NO: 58: CD7 142.9 VL amino acid sequence (humanized): 
EIVLTQSPAILSASPGDRVTLSCSARSSVSYMNWYQQKSGSSPRIWIYGISNLASGIPA
RFSGSGSGTSFTFTINSMEAEDVAYYYCQLRSSYSRTFGGGTKVEIK
- SEQ ID NO: 49: CD7 142.9 VL nucleotide sequence (murine): 
GAGATTCTGTTAACTCAGTCACCCGCTATTATTGCCGCATCTCCAGGCGAAAAGG
TGACAATCACCTGCTCAGCCCGCAGCTCCGTATCTTACATGAACTGGTATCAGCA
GAAATCAGGTTCATCCCCTAAGATATGGATCTATGGAATATCTAACTTGGCTTCAG
GCGTGCCTGCAAGGTTCTCTGGGTCCGGTAGTGGCACTTCCTTTAGTTTCACAAT
CAATTCCATGGAAGCCGAGGATGTCGCATACTATTACTGTCAACTCAGAAGTTCCT
ACTCCCGTACATTCGGCGGCGGCACAAAACTTGAGATCAAG
- SEQ ID NO: 50: CD7 142.9 VH amino acid sequence (murine): 
QVQLQQSGAELVRPGTSVKISCKASGYTFTNYWLGWVKQRPGHGLEWIGDFYPGSI
YTNYNENFQGKATLTADTSSSTAYMQLSSLTSEDSAVYFCAREGDYYGSGHFDYWG
QGTTLTVSS
- SEQ ID NO: 59: CD7 142.9 VH amino acid sequence (humanized) 
QVQLQQSGAELVKPGTSVKISCKASGYTFTNYWLGWVKQRPGHGLEWIGDFYPGSI
YTNYNENFQGKATLTADTSSSTAYMQLNSLTSEDTAVYFCAREGDYYGSGHFDYWG
QGTTVTVSS
- SEQ ID NO: 51: CD7 142.9 VH nucleotide sequence (murine) 
CAGGTGCAGCTCCAGCAGTCCGGTGCCGAGCTGGTTCGGCCCGGCACATCCGT
GAAGATCTCCTGCAAGGCTTCGGGCTATACTTTCACCAACTATTGGCTGGGATGG
GTGAAACAAAGGCCCGGTCACGGGTTGGAGTGGATCGGGGATTTCTACCCTGGA
TCAATTTATACCAATTATAACGAGAATTTTCAAGGGAAAGCTACACTGACCGCCGA
CACTAGCAGCAGCACCGCCTACATGCAGCTCAGCTCTCTTACGAGCGAGGACTC
GGCCGTCTACTTTTGTGCACGAGAAGGGGACTACTACGGCAGTGGTCATTTTGAT
TACTGGGGGCAGGGTACCACCCTCACGGTCAGCTCT
- SEQ ID NO: 52: CD7 142.9 Signal peptide + VL + linker + VH aa sequence (murine) 
MALPVTGLLLSLGLLLHAARPEILLTQSPAIIAASPGEKVTITCSARSSVSYMNWYQQK
SGSSPKIWIYGISNLASGVPARFSGSGSGTSFSFTINSMEAEDVAYYYCQLRSSYSRT
FGGGTKLEIKGGGGSGGGGSGGGGSGGGGSQVQLQQSGAELVRPGTSVKISCKAS
GYTFTNYWLGWVKQRPGHGLEWIGDFYPGSIYTNYNENFQGKATLTADTSSSTAYM
QLSSLTSEDSAVYFCAREGDYYGSGHFDYWGQGTTLTVSS
- SEQ ID NO: 62: CD7 142.9 Signal peptide + VL + linker + VH aa sequence (humanized)

MALPVTGLLLSLGLLLHAARPEIVLTQSPAILSASPGDRVTLSCSARSSVSYMNWYQQ
KSGSSPRIWIYGISNLASGIPARFSGSGSGTSFTFTINSMEAEDVAYYYCQLRSSYSRT
FGGGTKVEIKGGGGSGGGGSGGGGSGGGGSQVQLQQSGAELVKPGTSVKISCKAS
GYTFTNYWLGWVKQRPGHGLEWIGDFYPGSIYTNYNENFQGKATLTADTSSSTAYM
QLNSLTSEDTAVYFCAREGDYYGSGHFDYWGQGTTVTVSS

### CARS Backbone:

- SEQ ID NO: 22: CD8 TM : IYIWAPLAGTCGVLLLSLVITLYC
- SEQ ID NO: 23: CD8 hinge:
   TTTPAPRPPTPAPTIASQPLSLRPEACRPAAGGAVHTRGLDFACD
- SEQ ID NO: 24: CD8 hinge + TM: 
TTTPAPRPPTPAPTIASQPLSLRPEACRPAAGGAVHTRGLDFACDIYIWAPLAG
TCGVLLLSLVITLYC
- SEQ ID NO: 25: CD3z: 
RVKFSRSADAPAYQQGQNQLYNELNLGRREEYDVLDKRRGRDPEMGGKPQ
RRKNPQEGLYNELQKDKMAEAYSEIGMKGERRRGKGHDGLYQGLSTATKDT
YDALHMQALPPR
- SEQ ID NO: 26: 4-1BB:
   KRGRKKLLYIFKQPFMRPVQTTQEEDGCSCRFPEEEEGGCEL

### CAR full sequence:

- SEQ ID NO: 13: **CD7 CAR 124.1D1** - Signal peptide + VL + linker + VH + CD8 hinge + CD8 TM + 4-1BB + CD3z amino acid sequence (murine): 
MALPVTGLLLSLGLLLHAARPDILLTQSPAILSVSPGERVSFSCRASQSIGTSIHWYQQ
RTNDSPRLLIKYASESISGIPSRFSGSGSGTDFTLNINSVESEDIADYYCQQSISWPLTF
GPGTKLELKGGGGSGGGGSGGGGSGGGGSDVQLQESGPGLVKPSQSLSLTCTVTG
YSITSDYAWNWIRQFPGNKLEWLGYISYSGSTSYNPSLKSRISITRDTSKNQFFLQLNS
VTTEDTATYYCAKSQLGRWAMDYWGQGTSVTVSSTTTPAPRPPTPAPTIASQPLSLR
PEACRPAAGGAVHTRGLDFACDIYIWAPLAGTCGVLLLSLVITLYCKRGRKKLLYIFKQ
PFMRPVQTTQEEDGCSCRFPEEEEGGCELRVKFSRSADAPAYQQGQNQLYNELNL
 
GRREEYDVLDKRRGRDPEMGGKPQRRKNPQEGLYNELQKDKMAEAYSEIGMKGER
RRGKGHDGLYQGLSTATKDTYDALHMQALPPR*
- SEQ ID NO: 65: **CD7 CAR 124.1D1** - Signal peptide + VL + linker + VH+ CD8 hinge + CD8 TM + 4-1BB + CD3z amino acid sequence (humanized): 
MALPVTGLLLSLGLLLHAARPEIVLTQSPAILSLSPGERVTFSCRASQSIGTSIHWYQQK
TNDSPRLLIKYASESISGIPSRFSGSGSGTDFTLNISSLESEDIADYYCQQSISWPLTFG
PGTKLEIKGGGGSGGGGSGGGGSGGGGSEVQLLEQSGPGLVKPSQSLSLTCSVTG
YSITSDYAWNWIRQFPGNKLEWIGYISYSGSTSYNPSLKSRVTITRDTSQNQYYLQLN
SVTTEDTATYYCAKSQLGRWAMDYWGQGTTVTVSSTTTPAPRPPTPAPTIASQPLSL
RPEACRPAAGGAVHTRGLDFACDIYIWAPLAGTCGVLLLSLVITLYCKRGRKKLLYIFK
QPFMRPVQTTQEEDGCSCRFPEEEEGGCELRVKFSRSADAPAYQQGQNQLYNELN
LGRREEYDVLDKRRGRDPEMGGKPQRRKNPQEGLYNELQKDKMAEAYSEIGMKGE
RRRGKGHDGLYQGLSTATKDTYDALHMQALPPR*
- SEQ ID NO: 53: **CD7 CAR 124.1D1-** Signal peptide + VH + linker + VL + CD8 hinge + CD8 TM + 4-1BB + CD3z nucleic acid sequence (murine): 
ATGGCTCTGCCTGTAACCGGACTCTTACTCTCTTTGGGGCTACTCCTTCACGCAG
CCAGGCCTGACATTCTGCTTACACAGAGTCCCGCCATCCTGAGCGTCTCTCCAGG
CGAGCGGGTCTCCTTCAGTTGTCGCGCTAGTCAGAGCATTGGAACCTCCATCCAT
TGGTATCAGCAGAGAACGAATGATAGCCCCCGGCTGCTGATAAAGTATGCATCCG
AGTCCATATCAGGCATCCCTTCTAGGTTTTCAGGAAGCGGATCTGGCACTGACTTT
ACGTTGAACATCAACAGCGTGGAATCCGAGGACATCGCTGATTATTATTGCCAAC
AGTCTATCTCCTGGCCGCTTACATTCGGCCCAGGAACAAAGTTAGAGCTGAAGGG
TGGGGGGGGTAGCGGAGGAGGCGGATCAGGAGGGGGCGGCAGTGGAGGCGGG
GGCTCCGATGTGCAGCTGCAAGAAAGTGGCCCAGGTCTTGTGAAGCCTAGTCAG
AGCCTGAGCCTAACCTGTACAGTGACCGGTTACAGCATTACTTCGGATTACGCAT
GGAACTGGATTCGTCAATTCCCCGGTAACAAACTGGAGTGGCTCGGATACATCAG
CTATTCCGGGTCCACATCCTACAATCCCTCTTTGAAATCCAGAATATCTATTACAC
GAGACACCAGCAAGAATCAATTCTTTCTGCAGTTGAATTCAGTGACCACCGAAGA
CACCGCCACTTACTACTGCGCGAAATCACAGCTGGGGAGATGGGCCATGGATTA
CTGGGGGCAGGGGACTAGCGTTACTGTTTCTTCAACAACTACGCCGGCGCCGAG
ACCACCTACACCTGCACCAACTATTGCCTCTCAGCCACTGAGTCTGCGCCCCGAG
GCATGTCGACCTGCCGCTGGCGGGGCTGTGCACACCAGGGGCCTAGACTTCGC
CTGCGATATCTATATTTGGGCTCCACTGGCAGGAACCTGTGGCGTGCTGCTGCTG
TCTCTGGTCATCACACTGTACTGCAAAAGAGGCAGGAAGAAACTGCTGTATATTTT
 
CAAGCAGCCCTTTATGAGACCTGTGCAGACAACTCAGGAGGAAGACGGGTGCAG
CTGTAGGTTCCCTGAGGAAGAGGAAGGAGGCTGTGAGCTGCGCGTGAAATTTTCT
CGGAGTGCAGATGCCCCAGCTTACCAGCAGGGCCAGAACCAGCTGTATAACGAG
CTGAATCTGGGGCGGAGAGAGGAATACGACGTGCTGGATAAGAGGCGCGGGCG
AGATCCAGAAATGGGAGGAAAACCCCAGCGACGGAAGAACCCTCAGGAGGGACT
GTACAATGAACTGCAGAAGGACAAAATGGCAGAGGCCTATTCCGAAATCGGGATG
AAAGGAGAAAGAAGGCGCGGCAAGGGGCATGATGGCCTGTATCAGGGACTGTCA
ACCGCAACAAAAGATACTTATGATGCTCTGCACATGCAGGCTCTGCCCCCGCGGT
AA
- SEQ ID NO: 12: **CD7 CAR 142.24** - Signal peptide + VL + linker + VH+ CD8 hinge + CD8 TM + 4-1BB + CD3z amino acid sequence (murine): 
MALPVTGLLLSLGLLLHAARPDILLTQSPAILSVSPGERVSFSCRASQNIGTSIHWFQQ
RSNGSPSLLIKYASESMSGIPSRFSGSGSGTDFTLSINSVEPEDIADYYCQQSNRWPY
TFGGGTKLEIKGGGGSGGGGSGGGGSGGGGSQLVQSGPEVKKPGETVKISCKASG
YSFTHYGVNWVKQAPGKGLKWMGWINTNTGKPTYAEDFKGRFAFSLETSARTAYLQI
NNLKNEDTATYFCARWSTTVVAPYFDVWGAGTTVTVSSTTTPAPRPPTPAPTIASQPL
SLRPEACRPAAGGAVHTRGLDFACDIYIWAPLAGTCGVLLLSLVITLYCKRGRKKLLYI
FKQPFMRPVQTTQEEDGCSCRFPEEEEGGCELRVKFSRSADAPAYQQGQNQLYNE
LNLGRREEYDVLDKRRGRDPEMGGKPQRRKNPQEGLYNELQKDKMAEAYSEIGMK
GERRRGKGHDGLYQGLSTATKDTYDALHMQALPPR*
- SEQ ID NO: 66: **CD7 CAR 142.24** - Signal peptide + VL + linker + VH+ CD8 hinge + CD8 TM + 4-1BB + CD3z amino acid sequence (humanized): 
MALPVTGLLLSLGLLLHAARPEIVLTQSPAILSVSPGERVSFSCRASQNIGTSIHWYQQ
KTNGSPSLLIKYASESMSGIPSRFSGSGSGTDFTLTINSLQPEDIADYYCQQSNRWPY
TFGGGTKLELKGGGGSGGGGSGGGGSGGGGSQLVQSGPELKKPGETVKISCKASG
YSFTHYGVNWVRQAPGQGLEWMGWINTNTGKPTYAEDFKGRFAFSLDTSARTAYLQ
ISSLKNEDTATYYCARWSTTVVAPYFDVWGAGTSVTVSSTTTPAPRPPTPAPTIASQP
LSLRPEACRPAAGGAVHTRGLDFACDIYIWAPLAGTCGVLLLSLVITLYCKRGRKKLLYI
FKQPFMRPVQTTQEEDGCSCRFPEEEEGGCELRVKFSRSADAPAYQQGQNQLYNE
LNLGRREEYDVLDKRRGRDPEMGGKPQRRKNPQEGLYNELQKDKMAEAYSEIGMK
GERRRGKGHDGLYQGLSTATKDTYDALHMQALPPR*
- SEQ ID NO: 28: **CD7 CAR 142.24** Signal peptide + VL + linker + VH+ CD8 hinge + CD8 TM + 4-1BB + CD3z nucleic acid sequence (murine): 
ATGGCTCTGCCTGTAACCGGACTCTTACTCTCTTTGGGGCTACTCCTTCACGCAG
CCAGGCCTGACATTCTGCTGACTCAGTCACCAGCCATCCTCTCAGTGTCACCTGG
CGAGCGTGTTTCATTCAGCTGTAGGGCTTCGCAAAATATCGGGACCAGCATCCAC
TGGTTCCAGCAGAGATCTAACGGCTCGCCAAGCTTACTCATCAAATATGCGAGTG
AGTCCATGTCCGGCATCCCTTCACGCTTTTCCGGCTCTGGTTCTGGAACTGATTTT
ACCCTTAGCATAAATAGTGTGGAGCCTGAAGATATAGCCGACTACTACTGCCAAC
AATCCAACCGGTGGCCCTACACATTTGGCGGTGGCACCAAACTTGAGATTAAGGG
TGGGGGGGGTAGCGGAGGAGGCGGATCAGGAGGGGGCGGCAGTGGAGGCGGG
GGCTCCCAGCTAGTGCAGTCTGGACCCGAAGTGAAAAAGCCTGGAGAGACAGTT
AAGATTTCCTGCAAAGCAAGTGGATATAGCTTCACACACTACGGTGTAAATTGGGT
GAAGCAGGCACCAGGCAAAGGGCTTAAGTGGATGGGGTGGATCAACACAAATAC
GGGCAAGCCCACTTATGCCGAAGACTTTAAGGGGAGATTCGCCTTCTCTCTGGAA
ACGAGTGCACGAACAGCTTACCTCCAGATTAACAATCTGAAAAACGAGGATACTG
CTACATATTTCTGTGCTAGGTGGTCAACCACCGTCGTGGCCCCCTACTTTGACGT
CTGGGGTGCGGGGACTACCGTAACCGTCTCCTCCACAACTACGCCGGCGCCGAG
ACCACCTACACCTGCACCAACTATTGCCTCTCAGCCACTGAGTCTGCGCCCCGAG
GCATGTCGACCTGCCGCTGGCGGGGCTGTGCACACCAGGGGCCTAGACTTCGC
CTGCGATATCTATATTTGGGCTCCACTGGCAGGAACCTGTGGCGTGCTGCTGCTG
TCTCTGGTCATCACACTGTACTGCAAAAGAGGCAGGAAGAAACTGCTGTATATTTT
CAAGCAGCCCTTTATGAGACCTGTGCAGACAACTCAGGAGGAAGACGGGTGCAG
CTGTAGGTTCCCTGAGGAAGAGGAAGGAGGCTGTGAGCTGCGCGTGAAATTTTCT
CGGAGTGCAGATGCCCCAGCTTACCAGCAGGGCCAGAACCAGCTGTATAACGAG
CTGAATCTGGGGCGGAGAGAGGAATACGACGTGCTGGATAAGAGGCGCGGGCG
AGATCCAGAAATGGGAGGAAAACCCCAGCGACGGAAGAACCCTCAGGAGGGACT
GTACAATGAACTGCAGAAGGACAAAATGGCAGAGGCCTATTCCGAAATCGGGATG
AAAGGAGAAAGAAGGCGCGGCAAGGGGCATGATGGCCTGTATCAGGGACTGTCA
ACCGCAACAAAAGATACTTATGATGCTCTGCACATGCAGGCTCTGCCCCCGCGGT
AA
- SEQ ID NO: 70: **CD7 CAR 142.9 humanized** - Signal peptide + VL + linker + VH+ CD8 hinge + CD8 TM + 4-1BB + CD3z amino acid sequence (humanized):

MALPVTGLLLSLGLLLHAARPEIVLTQSPAILSASPGDRVTLSCSARSSVSYMNWYQQ
KSGSSPRIWIYGISNLASGIPARFSGSGSGTSFTFTINSMEAEDVAYYYCQLRSSYSRT
FGGGTKVEIKGGGGSGGGGSGGGGSGGGGSQVQLQQSGAELVKPGTSVKISCKAS
GYTFTNYWLGWVKQRPGHGLEWIGDFYPGSIYTNYNENFQGKATLTADTSSSTAYM
QLNSLTSEDTAVYFCAREGDYYGSGHFDYWGQGTTVTVSSTTTPAPRPPTPAPTIAS

QPLSLRPEACRPAAGGAVHTRGLDFACDIYIWAPLAGTCGVLLLSLVITLYCKRGRKKL
LYIFKQPFMRPVQTTQEEDGCSCRFPEEEEGGCELRVKFSRSADAPAYQQGQNQLY
NELNLGRREEYDVLDKRRGRDPEMGGKPQRRKNPQEGLYNELQKDKMAEAYSEIG
MKGERRRGKGHDGLYQGLSTATKDTYDALHMQALPPR

### CD7-TRAP:

- SEQ ID NO: 30 ER retention sequence: AEKDEL
- SEQ ID NO: 31 CD7 trap 124.1D1 amino acid sequence (murine): 
MALPVTGLLLSLGLLLHAARPDILLTQSPAILSVSPGERVSFSCRASQSIGTSIHWYQQ
RTNDSPRLLIKYASESISGIPSRFSGSGSGTDFTLNINSVESEDIADYYCQQSISWPLTF
GPGTKLELKGGGGSGGGGSGGGGSGGGGSDVQLQESGPGLVKPSQSLSLTCTVTG
YSITSDYAWNWIRQFPGNKLEWLGYISYSGSTSYNPSLKSRISITRDTSKNQFFLQLNS
VTTEDTATYYCAKSQLGRWAMDYWGQGTSVTVSSGGGGSGGGGSGGGGSGGGG
SAEKDEL*
- SEQ ID NO: 67: CD7 trap 124.1D1 amino acid sequence (humanized): 
MALPVTGLLLSLGLLLHAARPEIVLTQSPAILSLSPGERVTFSCRASQSIGTSIHWYQQK
TNDSPRLLIKYASESISGIPSRFSGSGSGTDFTLNISSLESEDIADYYCQQSISWPLTFG
PGTKLEIKGGGGSGGGGSGGGGSGGGGSEVQLLEQSGPGLVKPSQSLSLTCSVTG
YSITSDYAWNWIRQFPGNKLEWIGYISYSGSTSYNPSLKSRVTITRDTSQNQYYLQLN
SVTTEDTATYYCAKSQLGRWAMDYWGQGTTVTVSSGGGGSGGGGSGGGGSGGG
GSAEKDEL*
- SEQ ID NO: 32: CD7 trap 142.24 amino acid sequence (murine): 
MALPVTGLLLSLGLLLHAARPDILLTQSPAILSVSPGERVSFSCRASQNIGTSIHWFQQ
RSNGSPSLLIKYASESMSGIPSRFSGSGSGTDFTLSINSVEPEDIADYYCQQSNRWPY
TFGGGTKLEIKGGGGSGGGGSGGGGSGGGGSQLVQSGPEVKKPGETVKISCKASG
YSFTHYGVNWVKQAPGKGLKWMGWINTNTGKPTYAEDFKGRFAFSLETSARTAYLQI
NNLKNEDTATYFCARWSTTVVAPYFDVWGAGTTVTVSSGGGGSGGGGSGGGGSG
GGGSAEKDEL*
- SEQ ID NO: 68: CD7 trap 142.24 amino acid sequence (humanized): 
MALPVTGLLLSLGLLLHAARPEIVLTQSPAILSVSPGERVSFSCRASQNIGTSIHWYQQ
KTNGSPSLLIKYASESMSGIPSRFSGSGSGTDFTLTINSLQPEDIADYYCQQSNRWPY
 
TFGGGTKLELKGGGGSGGGGSGGGGSGGGGSQLVQSGPELKKPGETVKISCKASG
YSFTHYGVNWVRQAPGQGLEWMGWINTNTGKPTYAEDFKGRFAFSLDTSARTAYLQ
ISSLKNEDTATYYCARWSTTVVAPYFDVWGAGTSVTVSSGGGGSGGGGSGGGGSG
GGGSAEKDEL*
- SEQ ID NO: 63: CD7 trap 142.9 amino acid sequence (murine): 
MALPVTGLLLSLGLLLHAARPEILLTQSPAIIAASPGEKVTITCSARSSVSYMNWYQQK
SGSSPKIWIYGISNLASGVPARFSGSGSGTSFSFTINSMEAEDVAYYYCQLRSSYSRT
FGGGTKLEIKGGGGSGGGGSGGGGSGGGGSQVQLQQSGAELVRPGTSVKISCKAS
GYTFTNYWLGWVKQRPGHGLEWIGDFYPGSIYTNYNENFQGKATLTADTSSSTAYM
QLSSLTSEDSAVYFCAREGDYYGSGHFDYWGQGTTLTVSSGGGGSGGGGSGGGG
SGGGGSAEKDEL*
- SEQ ID NO: 69 CD7 trap 142.9 amino acid sequence (humanized): 
MALPVTGLLLSLGLLLHAARPEIVLTQSPAILSASPGDRVTLSCSARSSVSYMNWYQQ
KSGSSPRIWIYGISNLASGIPARFSGSGSGTSFTFTINSMEAEDVAYYYCQLRSSYSRT
FGGGTKVEIKGGGGSGGGGSGGGGSGGGGSQVQLQQSGAELVKPGTSVKISCKAS
GYTFTNYWLGWVKQRPGHGLEWIGDFYPGSIYTNYNENFQGKATLTADTSSSTAYM
QLNSLTSEDTAVYFCAREGDYYGSGHFDYWGQGTTVTVSSGGGGSGGGGSGGGG
SGGGGSAEKDEL*
- SEQ ID NO: 33 CD7 trap 124.1D1 nucleic acid sequence (murine): 
ATGGCTCTGCCTGTAACCGGACTCTTACTCTCTTTGGGGCTACTCCTTCACGCAG
CCAGGCCTGACATTCTGCTTACACAGAGTCCCGCCATCCTGAGCGTCTCTCCAGG
CGAGCGGGTCTCCTTCAGTTGTCGCGCTAGTCAGAGCATTGGAACCTCCATCCAT
TGGTATCAGCAGAGAACGAATGATAGCCCCCGGCTGCTGATAAAGTATGCATCCG
AGTCCATATCAGGCATCCCTTCTAGGTTTTCAGGAAGCGGATCTGGCACTGACTTT
ACGTTGAACATCAACAGCGTGGAATCCGAGGACATCGCTGATTATTATTGCCAAC
AGTCTATCTCCTGGCCGCTTACATTCGGCCCAGGAACAAAGTTAGAGCTGAAGGG
TGGGGGGGGTAGCGGAGGAGGCGGATCAGGAGGGGGCGGCAGTGGAGGCGGG
GGCTCCGATGTGCAGCTGCAAGAAAGTGGCCCAGGTCTTGTGAAGCCTAGTCAG
AGCCTGAGCCTAACCTGTACAGTGACCGGTTACAGCATTACTTCGGATTACGCAT
GGAACTGGATTCGTCAATTCCCCGGTAACAAACTGGAGTGGCTCGGATACATCAG
CTATTCCGGGTCCACATCCTACAATCCCTCTTTGAAATCCAGAATATCTATTACAC
GAGACACCAGCAAGAATCAATTCTTTCTGCAGTTGAATTCAGTGACCACCGAAGA
 
CACCGCCACTTACTACTGCGCGAAATCACAGCTGGGGAGATGGGCCATGGATTA
CTGGGGGCAGGGGACTAGCGTTACTGTTTCTTCAGGCGGTGGCGGATCAGGTGG
GGGCGGGTCTGGGGGCGGTGGCTCGGGCGGCGGGGGAAGTGCCGAGAAAGAC
GAACTCTAA
- SEQ ID NO: 34 CD7 trap 142.24 nucleic acid sequence (murine): 
ATGGCTCTGCCTGTAACCGGACTCTTACTCTCTTTGGGGCTACTCCTTCACGCAG
CCAGGCCTGACATTCTGCTGACTCAGTCACCAGCCATCCTCTCAGTGTCACCTGG
CGAGCGTGTTTCATTCAGCTGTAGGGCTTCGCAAAATATCGGGACCAGCATCCAC
TGGTTCCAGCAGAGATCTAACGGCTCGCCAAGCTTACTCATCAAATATGCGAGTG
AGTCCATGTCCGGCATCCCTTCACGCTTTTCCGGCTCTGGTTCTGGAACTGATTTT
ACCCTTAGCATAAATAGTGTGGAGCCTGAAGATATAGCCGACTACTACTGCCAAC
AATCCAACCGGTGGCCCTACACATTTGGCGGTGGCACCAAACTTGAGATTAAGGG
TGGGGGGGGTAGCGGAGGAGGCGGATCAGGAGGGGGCGGCAGTGGAGGCGGG
GGCTCCCAGCTAGTGCAGTCTGGACCCGAAGTGAAAAAGCCTGGAGAGACAGTT
AAGATTTCCTGCAAAGCAAGTGGATATAGCTTCACACACTACGGTGTAAATTGGGT
GAAGCAGGCACCAGGCAAAGGGCTTAAGTGGATGGGGTGGATCAACACAAATAC
GGGCAAGCCCACTTATGCCGAAGACTTTAAGGGGAGATTCGCCTTCTCTCTGGAA
ACGAGTGCACGAACAGCTTACCTCCAGATTAACAATCTGAAAAACGAGGATACTG
CTACATATTTCTGTGCTAGGTGGTCAACCACCGTCGTGGCCCCCTACTTTGACGT
CTGGGGTGCGGGGACTACCGTAACCGTCTCCTCCGGCGGTGGCGGATCAGGTG
GGGGCGGGTCTGGGGGCGGTGGCTCGGGCGGCGGGGGAAGTGCCGAGAAAGA
CGAACTCTAA
- SEQ ID NO: 64: CD7 trap 142.9 nucleic acid sequence (murine):

ATGGCTCTGCCTGTAACCGGACTCTTACTCTCTTTGGGGCTACTCCTTCACGCAG
CCAGGCCTGAGATTCTGTTAACTCAGTCACCCGCTATTATTGCCGCATCTCCAGG
CGAAAAGGTGACAATCACCTGCTCAGCCCGCAGCTCCGTATCTTACATGAACTGG
TATCAGCAGAAATCAGGTTCATCCCCTAAGATATGGATCTATGGAATATCTAACTT
GGCTTCAGGCGTGCCTGCAAGGTTCTCTGGGTCCGGTAGTGGCACTTCCTTTAGT
TTCACAATCAATTCCATGGAAGCCGAGGATGTCGCATACTATTACTGTCAACTCAG
AAGTTCCTACTCCCGTACATTCGGCGGCGGCACAAAACTTGAGATCAAGGGTGGG
GGGGGTAGCGGAGGAGGCGGATCAGGAGGGGGCGGCAGTGGAGGCGGGGGCT
CCCAGGTGCAGCTCCAGCAGTCCGGTGCCGAGCTGGTTCGGCCCGGCACATCC
GTGAAGATCTCCTGCAAGGCTTCGGGCTATACTTTCACCAACTATTGGCTGGGAT

GGGTGAAACAAAGGCCCGGTCACGGGTTGGAGTGGATCGGGGATTTCTACCCTG
GATCAATTTATACCAATTATAACGAGAATTTTCAAGGGAAAGCTACACTGACCGCC
GACACTAGCAGCAGCACCGCCTACATGCAGCTCAGCTCTCTTACGAGCGAGGAC
TCGGCCGTCTACTTTTGTGCACGAGAAGGGGACTACTACGGCAGTGGTCATTTTG
ATTACTGGGGGCAGGGTACCACCCTCACGGTCAGCTCTGGCGGTGGCGGATCAG
GTGGGGGCGGGTCTGGGGGCGGTGGCTCGGGCGGCGGGGGAAGTGCCGAGAA
AGACGAACTCTAA

### Bicistronic constructs:

- SEQ ID NO: 72: CD7 CAR 142.24trap humanized: Signal peptide + VL- 142.24 humanized + linker + VH 142.24 humanized+ CD8 hinge + CD8 TM + 4-1BB + CD3z amino acid sequence + T2A + Signal peptide + VL 124.1D1 humanized + linker + VH 124.1D1 humanized + Linker + ER retention nucleotide sequence: 
ATGGCTCTGCCTGTAACCGGACTCTTACTCTCTTTGGGGCTACTCCTTCACGCAG
CCAGGCCTGAGATCGTTCTTACACAGTCGCCAGCCATCCTGTCTGTGTCCCCTGG
AGAGCGCGTCTCATTTAGTTGCCGTGCTTCTCAGAACATCGGCACCAGCATACAT
TGGTATCAGCAGAAAACCAACGGCTCCCCCAGTTTACTGATTAAATATGCTTCCGA
GTCAATGTCTGGTATACCTTCCAGGTTCTCAGGTTCAGGAAGCGGCACAGATTTTA
CTCTTACAATCAACTCTTTGCAGCCAGAAGACATTGCAGACTACTACTGTCAACAG
TCTAATAGATGGCCCTATACGTTCGGTGGAGGCACTAAGTTGGAGCTCAAGGGGG
GCGGAGGTAGTGGAGGCGGCGGCAGCGGAGGCGGGGGGTCGGGGGGAGGAG
GTAGCCAACTAGTGCAATCCGGGCCAGAACTCAAGAAGCCGGGCGAAACCGTGA
AGATTTCCTGTAAAGCCAGTGGATACAGCTTCACTCACTACGGCGTCAATTGGGT
GCGGCAGGCCCCTGGGCAGGGGCTGGAGTGGATGGGGTGGATTAATACCAATA
CAGGCAAACCCACCTATGCTGAAGACTTTAAAGGGAGGTTCGCATTTTCCCTGGA
CACTTCTGCCCGAACCGCCTACCTCCAGATCAGCAGCCTGAAGAACGAGGATACT
GCCACATACTATTGCGCGAGATGGAGCACAACCGTAGTCGCACCCTACTTCGATG
TGTGGGGTGCTGGCACCTCAGTGACGGTTAGTTCCACAACTACGCCGGCGCCGA
GACCACCTACACCTGCACCAACTATTGCCTCTCAGCCACTGAGTCTGCGCCCCGA
GGCATGTCGACCTGCCGCTGGCGGGGCTGTGCACACCAGGGGCCTAGACTTCG
CCTGCGATATCTATATTTGGGCTCCACTGGCAGGAACCTGTGGCGTGCTGCTGCT
GTCTCTGGTCATCACACTGTACTGCAAAAGAGGCAGGAAGAAACTGCTGTATATTT
TCAAGCAGCCCTTTATGAGACCTGTGCAGACAACTCAGGAGGAAGACGGGTGCA
GCTGTAGGTTCCCTGAGGAAGAGGAAGGAGGCTGTGAGCTGCGCGTGAAATTTT
CTCGGAGTGCAGATGCCCCAGCTTACCAGCAGGGCCAGAACCAGCTGTATAACG
AGCTGAATCTGGGGCGGAGAGAGGAATACGACGTGCTGGATAAGAGGCGCGGG
 
CGAGATCCAGAAATGGGAGGAAAACCCCAGCGACGGAAGAACCCTCAGGAGGGA
CTGTACAATGAACTGCAGAAGGACAAAATGGCAGAGGCCTATTCCGAAATCGGGA
TGAAAGGAGAAAGAAGGCGCGGCAAGGGGCATGATGGCCTGTATCAGGGACTGT
CAACCGCAACAAAAGATACTTATGATGCTCTGCACATGCAGGCTCTGCCCCCGCG
GTCTAGAGAGGGACGTGGAAGTCTGCTAACATGCGGTGACGTCGAGGAGAATCC
CGGCCCTACGCGTATGGCTCTGCCTGTAACCGGACTCTTACTCTCTTTGGGGCTA
CTCCTTCACGCAGCCAGGCCTGAAATAGTACTGACACAGTCCCCAGCGATTCTCA
GCCTGAGCCCTGGCGAGAGAGTGACTTTTTCTTGTCGCGCCTCTCAATCAATCGG
AACTTCAATCCACTGGTACCAGCAGAAAACCAATGATTCCCCTAGGTTGTTAATAA
AGTATGCATCCGAGTCTATCTCTGGAATCCCCTCGAGGTTCAGTGGGAGCGGCAG
CGGTACCGATTTTACGTTGAACATTTCTTCCCTGGAGAGTGAAGACATAGCTGATT
ACTACTGCCAGCAGAGTATTAGTTGGCCCCTTACCTTCGGCCCGGGGACGAAGCT
GGAGATCAAGGGAGGGGGAGGTTCAGGCGGAGGTGGGTCAGGCGGCGGGGGA
TCCGGGGGTGGCGGGTCTGAAGTTCAGCTGCTGGAACAGTCGGGCCCCGGTCTT
GTCAAGCCTAGCCAAAGTCTCTCACTGACTTGTAGCGTGACCGGTTATAGCATCA
CAAGTGACTATGCCTGGAATTGGATCCGTCAGTTCCCAGGCAATAAACTGGAGTG
GATTGGATATATTAGCTATAGCGGCTCAACATCCTACAACCCATCCTTAAAGTCCC
GGGTGACTATTACTAGAGACACTTCACAGAACCAGTACTACCTACAACTCAACAGC
GTGACCACAGAGGACACCGCAACATACTACTGCGCCAAATCTCAACTCGGGCGAT
GGGCTATGGATTATTGGGGACAGGGCACCACAGTCACCGTTTCTTCCGGCGGTG
GCGGATCAGGTGGGGGCGGGTCTGGGGGCGGTGGCTCGGGCGGCGGGGGAAG
TGCCGAGAAAGACGAACTCTAA
- SEQ ID NO: 73: CD7 CAR 142.24trap humanized: Signal peptide + VL- 142.24 humanized + linker + VH 142.24 humanized+ CD8 hinge + CD8 TM + 4-1BB + CD3z amino acid sequence + T2A + Signal peptide + VL 124.1D1 humanized + linker + VH 124.1D1 humanized + Linker + ER retention amino acid sequence: 
MALPVTGLLLSLGLLLHAARPEIVLTQSPAILSVSPGERVSFSCRASQNIGTSIHWYQQ
KTNGSPSLLIKYASESMSGIPSRFSGSGSGTDFTLTINSLQPEDIADYYCQQSNRWPY
TFGGGTKLELKGGGGSGGGGSGGGGSGGGGSQLVQSGPELKKPGETVKISCKASG
YSFTHYGVNWVRQAPGQGLEWMGWINTNTGKPTYAEDFKGRFAFSLDTSARTAYLQ
ISSLKNEDTATYYCARWSTTVVAPYFDVWGAGTSVTVSSTTTPAPRPPTPAPTIASQP
LSLRPEACRPAAGGAVHTRGLDFACDIYIWAPLAGTCGVLLLSLVITLYCKRGRKKLLYI
FKQPFMRPVQTTQEEDGCSCRFPEEEEGGCELRVKFSRSADAPAYQQGQNQLYNE
LNLGRREEYDVLDKRRGRDPEMGGKPQRRKNPQEGLYNELQKDKMAEAYSEIGMK
GERRRGKGHDGLYQGLSTATKDTYDALHMQALPPRSREGRGSLLTCGDVEENPGPT
 
RMALPVTGLLLSLGLLLHAARPEIVLTQSPAILSLSPGERVTFSCRASQSIGTSIHWYQ
QKTNDSPRLLIKYASESISGIPSRFSGSGSGTDFTLNISSLESEDIADYYCQQSISWPLT
FGPGTKLEIKGGGGSGGGGSGGGGSGGGGSEVQLLEQSGPGLVKPSQSLSLTCSV
TGYSITSDYAWNWIRQFPGNKLEWIGYISYSGSTSYNPSLKSRVTITRDTSQNQYYLQ
LNSVTTEDTATYYCAKSQLGRWAMDYWGQGTTVTVSSGGGGSGGGGSGGGGSGG
GGSAEKDEL*
- SEQ ID NO: 74: CD7 CAR 142.24Trap Signal peptide + VL- 142.24 murine + linker + VH 142.24 murine+ CD8 hinge + CD8 TM + 4-1BB + CD3z amino acid sequence + T2A + Signal peptide + VL 124.1D1 murine + linker + VH 124.1D1 murine + Linker + ER retention nucleotide sequence: 
ATGGCTCTGCCTGTAACCGGACTCTTACTCTCTTTGGGGCTACTCCTTCACGCAG
CCAGGCCTGACATTCTGCTGACTCAGTCACCAGCCATCCTCTCAGTGTCACCTGG
CGAGCGTGTTTCATTCAGCTGTAGGGCTTCGCAAAATATCGGGACCAGCATCCAC
TGGTTCCAGCAGAGATCTAACGGCTCGCCAAGCTTACTCATCAAATATGCGAGTG
AGTCCATGTCCGGCATCCCTTCACGCTTTTCCGGCTCTGGTTCTGGAACTGATTTT
ACCCTTAGCATAAATAGTGTGGAGCCTGAAGATATAGCCGACTACTACTGCCAAC
AATCCAACCGGTGGCCCTACACATTTGGCGGTGGCACCAAACTTGAGATTAAGGG
TGGGGGGGGTAGCGGAGGAGGCGGATCAGGAGGGGGCGGCAGTGGAGGCGGG
GGCTCCCAGCTAGTGCAGTCTGGACCCGAAGTGAAAAAGCCTGGAGAGACAGTT
AAGATTTCCTGCAAAGCAAGTGGATATAGCTTCACACACTACGGTGTAAATTGGGT
GAAGCAGGCACCAGGCAAAGGGCTTAAGTGGATGGGGTGGATCAACACAAATAC
GGGCAAGCCCACTTATGCCGAAGACTTTAAGGGGAGATTCGCCTTCTCTCTGGAA
ACGAGTGCACGAACAGCTTACCTCCAGATTAACAATCTGAAAAACGAGGATACTG
CTACATATTTCTGTGCTAGGTGGTCAACCACCGTCGTGGCCCCCTACTTTGACGT
CTGGGGTGCGGGGACTACCGTAACCGTCTCCTCCACAACTACGCCGGCGCCGAG
ACCACCTACACCTGCACCAACTATTGCCTCTCAGCCACTGAGTCTGCGCCCCGAG
GCATGTCGACCTGCCGCTGGCGGGGCTGTGCACACCAGGGGCCTAGACTTCGC
CTGCGATATCTATATTTGGGCTCCACTGGCAGGAACCTGTGGCGTGCTGCTGCTG
TCTCTGGTCATCACACTGTACTGCAAAAGAGGCAGGAAGAAACTGCTGTATATTTT
CAAGCAGCCCTTTATGAGACCTGTGCAGACAACTCAGGAGGAAGACGGGTGCAG
CTGTAGGTTCCCTGAGGAAGAGGAAGGAGGCTGTGAGCTGCGCGTGAAATTTTCT
CGGAGTGCAGATGCCCCAGCTTACCAGCAGGGCCAGAACCAGCTGTATAACGAG
CTGAATCTGGGGCGGAGAGAGGAATACGACGTGCTGGATAAGAGGCGCGGGCG
AGATCCAGAAATGGGAGGAAAACCCCAGCGACGGAAGAACCCTCAGGAGGGACT
GTACAATGAACTGCAGAAGGACAAAATGGCAGAGGCCTATTCCGAAATCGGGATG
 
AAAGGAGAAAGAAGGCGCGGCAAGGGGCATGATGGCCTGTATCAGGGACTGTCA
ACCGCAACAAAAGATACTTATGATGCTCTGCACATGCAGGCTCTGCCCCCGCGGT
CTAGAGAGGGACGTGGAAGTCTGCTAACATGCGGTGACGTCGAGGAGAATCCCG
GCCCTACGCGTATGGCTCTGCCTGTAACCGGACTCTTACTCTCTTTGGGGCTACT
CCTTCACGCAGCCAGGCCTGACATTCTGCTTACACAGAGTCCCGCCATCCTGAGC
GTCTCTCCAGGCGAGCGGGTCTCCTTCAGTTGTCGCGCTAGTCAGAGCATTGGAA
CCTCCATCCATTGGTATCAGCAGAGAACGAATGATAGCCCCCGGCTGCTGATAAA
GTATGCATCCGAGTCCATATCAGGCATCCCTTCTAGGTTTTCAGGAAGCGGATCT
GGCACTGACTTTACGTTGAACATCAACAGCGTGGAATCCGAGGACATCGCTGATT
ATTATTGCCAACAGTCTATCTCCTGGCCGCTTACATTCGGCCCAGGAACAAAGTTA
GAGCTGAAGGGTGGGGGGGGTAGCGGAGGAGGCGGATCAGGAGGGGGCGGCA
GTGGAGGCGGGGGCTCCGATGTGCAGCTGCAAGAAAGTGGCCCAGGTCTTGTGA
AGCCTAGTCAGAGCCTGAGCCTAACCTGTACAGTGACCGGTTACAGCATTACTTC
GGATTACGCATGGAACTGGATTCGTCAATTCCCCGGTAACAAACTGGAGTGGCTC
GGATACATCAGCTATTCCGGGTCCACATCCTACAATCCCTCTTTGAAATCCAGAAT
ATCTATTACACGAGACACCAGCAAGAATCAATTCTTTCTGCAGTTGAATTCAGTGA
CCACCGAAGACACCGCCACTTACTACTGCGCGAAATCACAGCTGGGGAGATGGG
CCATGGATTACTGGGGGCAGGGGACTAGCGTTACTGTTTCTTCAGGCGGTGGCG
GATCAGGTGGGGGCGGGTCTGGGGGCGGTGGCTCGGGCGGCGGGGGAAGTGC
CGAGAAAGACGAACTCTAA
- SEQ ID NO: 75: CD7 CAR 142.24Trap Signal peptide + VL- 142.24 murine + linker + VH 142.24 murine+ CD8 hinge + CD8 TM + 4-1BB + CD3z amino acid sequence + T2A + Signal peptide + VL 124.1D1 murine + linker + VH 124.1D1 murine + Linker + ER retention amino acid sequence: 
MALPVTGLLLSLGLLLHAARPDILLTQSPAILSVSPGERVSFSCRASQNIGTSIHWFQQ
RSNGSPSLLIKYASESMSGIPSRFSGSGSGTDFTLSINSVEPEDIADYYCQQSNRWPY
TFGGGTKLEIKGGGGSGGGGSGGGGSGGGGSQLVQSGPEVKKPGETVKISCKASG
YSFTHYGVNWVKQAPGKGLKWMGWINTNTGKPTYAEDFKGRFAFSLETSARTAYLQI
NNLKNEDTATYFCARWSTTVVAPYFDVWGAGTTVTVSSTTTPAPRPPTPAPTIASQPL
SLRPEACRPAAGGAVHTRGLDFACDIYIWAPLAGTCGVLLLSLVITLYCKRGRKKLLYI
FKQPFMRPVQTTQEEDGCSCRFPEEEEGGCELRVKFSRSADAPAYQQGQNQLYNE
LNLGRREEYDVLDKRRGRDPEMGGKPQRRKNPQEGLYNELQKDKMAEAYSEIGMK
GERRRGKGHDGLYQGLSTATKDTYDALHMQALPPRSREGRGSLLTCGDVEENPGPT
RMALPVTGLLLSLGLLLHAARPDILLTQSPAILSVSPGERVSFSCRASQSIGTSIHWYQ
QRTNDSPRLLIKYASESISGIPSRFSGSGSGTDFTLNINSVESEDIADYYCQQSISWPLT
 
FGPGTKLELKGGGGSGGGGSGGGGSGGGGSDVQLQESGPGLVKPSQSLSLTCTVT
GYSITSDYAWNWIRQFPGNKLEWLGYISYSGSTSYNPSLKSRISITRDTSKNQFFLQLN
SVTTEDTATYYCAKSQLGRWAMDYWGQGTSVTVSSGGGGSGGGGSGGGGSGGG
GSAEKDEL*
- SEQ ID NO: 76: CD7 CAR 142.9trap humanized - Signal peptide + VL 142.9 humanized + linker + VH 142.9 humanized + CD8 hinge + CD8 TM + 4-1BB + CD3z amino acid sequence + Signal peptide + VL 124.1D1 humanized + linker + VH 124.1D1 humanized + Linker + ER retention nucleotide sequence: 
ATGGCTCTGCCTGTAACCGGACTCTTACTCTCTTTGGGGCTACTCCTTCAC
GCAGCCAGGCCTGAGATCGTGCTGACCCAGAGCCCAGCTATCCTGAGTG
CCTCTCCAGGGGATAGAGTAACTCTTTCGTGTTCCGCCCGTAGTAGTGTCT
CTTACATGAACTGGTACCAGCAGAAATCAGGCAGCAGCCCCCGCATTTGG
ATTTACGGTATCAGCAACCTCGCCAGCGGTATTCCTGCCAGATTCAGCGG
ATCCGGGTCAGGGACTAGCTTCACATTCACAATCAATAGCATGGAGGCTG
AAGACGTGGCCTATTATTATTGTCAGTTAAGGTCCTCCTACTCGAGGACAT
TTGGGGGAGGCACCAAGGTGGAGATTAAGGGCGGCGGGGGCTCAGGGG
GCGGCGGTTCAGGCGGGGGCGGATCTGGAGGAGGAGGCAGTCAGGTGC
AGCTGCAGCAATCCGGCGCAGAACTCGTGAAGCCTGGCACATCTGTCAAG
ATAAGTTGCAAGGCTTCCGGATATACTTTTACCAATTATTGGTTGGGTTGG
GTGAAACAACGGCCGGGACATGGTCTAGAGTGGATCGGGGATTTTTACCC
CGGTAGTATATATACTAACTACAATGAAAACTTTCAGGGAAAAGCCACCCT
TACCGCTGACACTTCCTCCTCTACAGCATACATGCAGCTGAATTCACTGAC
GTCTGAAGACACCGCAGTCTACTTCTGCGCGCGAGAGGGGGATTACTATG
GGTCTGGCCACTTCGACTACTGGGGACAAGGCACAACCGTTACGGTTAGC
TCAACAACTACGCCGGCGCCGAGACCACCTACACCTGCACCAACTATTGC
CTCTCAGCCACTGAGTCTGCGCCCCGAGGCATGTCGACCTGCCGCTGGC
GGGGCTGTGCACACCAGGGGCCTAGACTTCGCCTGCGATATCTATATTTG
GGCTCCACTGGCAGGAACCTGTGGCGTGCTGCTGCTGTCTCTGGTCATCA
CACTGTACTGCAAAAGAGGCAGGAAGAAACTGCTGTATATTTTCAAGCAGC
CCTTTATGAGACCTGTGCAGACAACTCAGGAGGAAGACGGGTGCAGCTGT
AGGTTCCCTGAGGAAGAGGAAGGAGGCTGTGAGCTGCGCGTGAAATTTTC
TCGGAGTGCAGATGCCCCAGCTTACCAGCAGGGCCAGAACCAGCTGTATA
 
ACGAGCTGAATCTGGGGCGGAGAGAGGAATACGACGTGCTGGATAAGAG
GCGCGGGCGAGATCCAGAAATGGGAGGAAAACCCCAGCGACGGAAGAAC
CCTCAGGAGGGACTGTACAATGAACTGCAGAAGGACAAAATGGCAGAGGC
CTATTCCGAAATCGGGATGAAAGGAGAAAGAAGGCGCGGCAAGGGGCAT
GATGGCCTGTATCAGGGACTGTCAACCGCAACAAAAGATACTTATGATGCT
CTGCACATGCAGGCTCTGCCCCCGCGGTCTAGAGAGGGACGTGGAAGTC
TGCTAACATGCGGTGACGTCGAGGAGAATCCCGGCCCTACGCGTATGGCT
CTGCCTGTAACCGGACTCTTACTCTCTTTGGGGCTACTCCTTCACGCAGCC
AGGCCTGAAATAGTACTGACACAGTCCCCAGCGATTCTCAGCCTGAGCCC
TGGCGAGAGAGTGACTTTTTCTTGTCGCGCCTCTCAATCAATCGGAACTTC
AATCCACTGGTACCAGCAGAAAACCAATGATTCCCCTAGGTTGTTAATAAA
GTATGCATCCGAGTCTATCTCTGGAATCCCCTCGAGGTTCAGTGGGAGCG
GCAGCGGTACCGATTTTACGTTGAACATTTCTTCCCTGGAGAGTGAAGACA
TAGCTGATTACTACTGCCAGCAGAGTATTAGTTGGCCCCTTACCTTCGGCC
CGGGGACGAAGCTGGAGATCAAGGGAGGGGGAGGTTCAGGCGGAGGTG
GGTCAGGCGGCGGGGGATCCGGGGGTGGCGGGTCTGAAGTTCAGCTGC
TGGAACAGTCGGGCCCCGGTCTTGTCAAGCCTAGCCAAAGTCTCTCACTG
ACTTGTAGCGTGACCGGTTATAGCATCACAAGTGACTATGCCTGGAATTGG
ATCCGTCAGTTCCCAGGCAATAAACTGGAGTGGATTGGATATATTAGCTAT
AGCGGCTCAACATCCTACAACCCATCCTTAAAGTCCCGGGTGACTATTACT
AGAGACACTTCACAGAACCAGTACTACCTACAACTCAACAGCGTGACCACA
GAGGACACCGCAACATACTACTGCGCCAAATCTCAACTCGGGCGATGGGC
TATGGATTATTGGGGACAGGGCACCACAGTCACCGTTTCTTCCGGCGGTG
GCGGATCAGGTGGGGGCGGGTCTGGGGGCGGTGGCTCGGGCGGCGGG
GGAAGTGCCGAGAAAGACGAACTCTAA
- SEQ ID NO: 77: CD7 CAR 142.9trap humanized - Signal peptide + VL 142.9 humanized + linker + VH 142.9 humanized + CD8 hinge + CD8 TM + 4-1BB + CD3z amino acid sequence + Signal peptide + VL 124.1D1 humanized + linker + VH 124.1D1 humanized + Linker + ER retention amino acid sequence:

MALPVTGLLLSLGLLLHAARPEIVLTQSPAILSASPGDRVTLSCSARSSVSYMNWYQQ
KSGSSPRIWIYGISNLASGIPARFSGSGSGTSFTFTINSMEAEDVAYYYCQLRSSYSRT
FGGGTKVEIKGGGGSGGGGSGGGGSGGGGSQVQLQQSGAELVKPGTSVKISCKAS
GYTFTNYWLGWVKQRPGHGLEWIGDFYPGSIYTNYNENFQGKATLTADTSSSTAYM

QLNSLTSEDTAVYFCAREGDYYGSGHFDYWGQGTTVTVSSTTTPAPRPPTPAPTIAS
QPLSLRPEACRPAAGGAVHTRGLDFACDIYIWAPLAGTCGVLLLSLVITLYCKRGRKKL
LYIFKQPFMRPVQTTQEEDGCSCRFPEEEEGGCELRVKFSRSADAPAYQQGQNQLY
NELNLGRREEYDVLDKRRGRDPEMGGKPQRRKNPQEGLYNELQKDKMAEAYSEIG
MKGERRRGKGHDGLYQGLSTATKDTYDALHMQALPPRSREGRGSLLTCGDVEENP
GPTRMALPVTGLLLSLGLLLHAARPEIVLTQSPAILSLSPGERVTFSCRASQSIGTSIHW
YQQKTNDSPRLLIKYASESISGIPSRFSGSGSGTDFTLNISSLESEDIADYYCQQSISWP
LTFGPGTKLEIKGGGGSGGGGSGGGGSGGGGSEVQLLEQSGPGLVKPSQSLSLTCS
VTGYSITSDYAWNWIRQFPGNKLEWIGYISYSGSTSYNPSLKSRVTITRDTSQNQYYL
QLNSVTTEDTATYYCAKSQLGRWAMDYWGQGTTVTVSSGGGGSGGGGSGGGGSG
GGGSAEKDEL*

### Others:

- SEQ ID NO: 10 Linker: GGGGSGGGGSGGGGSGGGGS
- SEQ ID NO: 27 signal peptide: MALPVTGLLLSLGLLLHAARP
- SEQ ID NO: 29 T2A cleavage peptide: SREGRGSLLTCGDVEENPGP
- SEQ ID NO: 35 P2A cleavage peptide: ATNFSLLKQAGDVEENPGP
- SEQ ID NO: 36 E2A cleavage peptide: QCTNYALLKLAGDVESNPGP
- SEQ ID NO: 37 F2A cleavage peptide: VKQTLNFDLLKLAGDVESNPGP

The invention is described below by the following examples, which must be considered as merely illustrative and in no case limiting of the scope of the present invention.

### EXAMPLES

### Materials and methods

### Generation of anti-CD7 antibodies and derived anti-CD7 single chain variable fragments (scFv)

The 124.1D1, 142.24 and 142.9 murine anti-CD7 mAb were generated at the Department of Immunology (Hospital Clinic de Barcelona) using conventional moAb generation techniques and certified at the Fourth International Workshop on Human Leucocyte Differentiation Antigens (1).

The sequence corresponding to the variable light (VL) and variable heavy (VH) regions of 124.1D1, 142.24 and 142.9 antibodies was extracted from 124.1D1, 142.24 and 142.9 hybridoma cells using Mouse Ig-Primer Set (Novagen, 69831-3). We used the Integrated antibody sequence & structure analysis (abYsis) web-based platform to analyze the generated hybridomas sequences.

### Humanization of anti-CD7 scFv

Based on the murine sequences obtained from the hybridomas of the Immunology Department of the Hospital Clinic of Barcelona, a methodology was designed to obtain humanized sequences. These humanized sequences were constructed by CDR grafting, while retaining those murine framework residues that influence the antigen-binding activity and the structural configuration.

Briefly, the CDRs of both light and heavy chains were determined by several predictive algorithms like Kabat and Chothia numbering scheme, using Abysis and BLAST software. We also determined the most conserved positions of the sequences (>75% frequency) and identify the positions susceptible to post-translational modifications. These identified amino acids were maintained in the humanized sequence.

Next, a comparative search was made with the Position-Specific Iterated BLAST software to find the immunoglobulins of human origin with a closer identity to our sequence and will be used as a model to make the changes, but always respecting the essential positions identified above. Moreover, only synonymous amino acid changes were introduced. At this moment, a humanized sequence has been designed for the light and heavy chain of each murine scFv.

### Microfluidic antibody-affinity profiling (MAAP)

Recombinant CD7 protein was purchased from ACRO biosystems (Cat No CD-H52H7) and was labeled with Alexa FluorTM 647 NHS Ester (Thermo Fisher Cat No A200006) following manufacturer's instruction.

For the MAAP, we set up initial titration curves with different concentrations of Alexa FluorTM 647-labeled CD7 (10nM and 40nm) for the simultaneous determination of hybridoma-secreted antibodies affinity (KD) and concentration. Hybridoma supernatants were thaw slowly on ice and spin down 5 minutes at 14000 x g. A series of 7 serum dilutions were prepared in 0.2% PBS-T ranging from 90% to 0% and incubated overnight at 4°C protected from the light. Serum dilutions for background determination were prepared as well. Each datapoint of the titration series was measured at medium flow setting (1.5 nm-8nm) on the Fluidity One-W system (Fluidic Analytics).

Once the data from the initial titration curves was collected a series of 3 MAAP experiments were performed for 142.24 and 142.9 hybridomas on the Fluidity One-W system (Fluidic Analytics), and 2 experiments for 124.1D1 hybridoma. MAAP on a Fluidity One-W Serum (Fluidic Analytics) was used to determine concentration of antibody-binding sites and KD of antibodies against CD7 in hybridoma supernatant samples as described previously.

Briefly, various concentrations of fluorescently labelled CD7 and supernatants of the cultured hybridoma, diluted in 0.2% PBS-T at different ratios, were mixed and incubated on ice for 1 hour. The binding affinity between the hybridoma-secreted monoclonal antibodies and CD7 measures were collected, and the KD values were determined by Bayesian inference.

### Design of anti-CD7 CARs and anti-CD7trap and lentiviral production

Second-generation CARs were designed to contain the anti-CD7 scFvs (124.1D1, 142.24 or 142.9), the CD8 hinge and transmembrane domains, along with the 4-1BB and CD3ζ endodomains. The CD7trap sequences included the signal peptide, the corresponding anti-CD7 scFv and the KDEL endoplasmic reticulum (ER) domain retention sequence. Each CD7trap was cloned in a bicistronic vector using the viral T2A sequence in combination with the GFP gene or the anti-CD7 CAR construct. All constructs were synthesized by GenScript and cloned into a third-generation lentiviral vector pCCL (kindly provided by Dr. Luigi Naldini; San Raffaele Hospital, Milan). The expression of the CAR or the CD7trap was controlled under the EF1α promoter.

The anti-CD7 CARs and anti-CD7 TRAPs lentiviral vectors were produced by transfecting HEK293FT cells using PEI (Cat# 23966-100; Polysciences, Warrington, PA, USA). The lentivirus-containing supernatant were collected after 48 hours and 72 hours and concentrated using Lenti-X Concentrator (Cat# 631232; Takara Bio, Shiga, Japan). Lentiviral vectors were tittered based on GFP expression (for CD7trap-GFP constructs) or superficial CAR expression using wild-type Jurkat cells or genome edited to ablate the CD7 gene, respectively, using the limiting dilution method.

### CAR-T cell generation

Human primary T lymphocytes were isolated from buffy coats of healthy volunteer donors (Banc de Sang i Teixits, Barcelona, Spain) using density gradient centrifugation with Lymphoprep (Cat# 07801; StemCell, Vancouver, Canada). CD3+, CD4+ and/or CD8+ lymphocytes were separated using RosetteSep for Human T cells (Cat# 15061, 15062 and 15063; StemCell) and stimulated with CD3/CD28 Human T-Activator Dynabeads (Cat# 11132D; Thermo Fisher Scientific, Waltham, MA, USA; or Cat# 11131D; Gibco). For some experiments, CD4+ and CD8+ T lymphocytes were cultured separately during T cell expansion. The CD8+ population contained 10% of CD4+ cells to support T cell proliferation. In other experiments, In other experiments, bulk CD3+ T cells were expanded, without separating CD4+ and CD8+ T cells in different cultures. T lymphocytes were cultured in complete RPMI 1640 media (Cat# 21875034; Gibco BRL Life Technologies, Gaithersburg, MD, USA) containing 10% heat-inactivated fetal bovine serum (FBS) (Cat# F4135; Merck, Darmstadt, Germany), 1% penicillin-streptomycin (Cat# 15140-122; Gibco BRL Life Technologies), 1% HEPES (Cat# H0887; Sigma-Aldrich, Saint Louis, MO, USA), 1% GlutaMax (Cat# 35050-061; Thermo Fisher Scientific), human IL-7 (10 ng/mL, Cat# 130-095-362; Miltenyi Biotec, Bergisch Gladbach, Germany; or 500U/MI, Cat# 1410-050, CellGenix, Freiburg, Germany) and human IL-15 (10 ng/mL, Cat# 130-095-764; Miltenyi Biotec; or 200U/mL, Cat# 1413-050, CellGenix, Freiburg, Germany). Stimulated T lymphocytes were then transduced with the corresponding lentiviral vector encoding the scFv-based constructs targeting CD7 on either day 1 or day 3 of expansion, as specified. Activation beads were magnetically removed from all groups between day 2 and day 6.

To generate CD7-edited CAR-T cells, the CD7 gene was genetically disrupted using the CRISPR/Cas9 system on day 2 of expansion. In this case, the culture media was supplemented with dasatinib (50nM; Cat# BMS-354825; Adooq Bioscience, Irvine, CA, USA) from day 1 until day 6, with dosing every 48 hours. In the groups where we generated unedited CD7 CAR-T cells in the presence of a pharmacologic inhibitor, dasatinib was added from day 1 until the end of the expansion protocol (day 3 or day 10, as specified), also with dosing every 48 hours. The concentration of dasatinib was determined based on titration experiments to determine the lowest dosage that inhibits CAR signaling. CAR-T cells were fed with media containing IL-7 and IL-15 daily until cryopreservation at day 3 or day 10 for further experiments. For the expansions in which CD4+ and CD8+ cells were expanded separately, CD4+ and CD8+ cells were mixed at a 1:1 ratio before freezing. The expression of CAR and CD7 in the T cell membrane was analyzed by flow cytometry between day 8 or 10 of T cell expansion. Cells were maintained in a humidified atmosphere containing 5% carbon dioxide (CO2) at 37°C.

To obtain naturally occurring CD7-negative CAR-T cells from healthy donors, the RosetteSep^{™} Human T cells Enrichment Cocktail (Stemcell technologies, 15061) was first used to isolate CD3+ T cells from whole blood by negative selection. T cells were then labeled with biotin-conjugated CD7 antibody (Miltenyi Biotec, 130-123-562) followed by anti-Biotin beads (Miltenyi Biotec, 130-090-485). CD7 depletion was performed using the LD columns (Miltenyi Biotec, 130-042-901) on a QuadroMACS Separator instrument (Miltenyi Biotec) following manufacturer's instructions. A solution containing phosphate-buffered saline (PBS), pH 7.2, 0.5% fetal bovine serum albumin (FBS), and 2 mM EDTA was employed as isolation buffer. Freshly resulting isolated CD7-negative T cells were used for CAR T cell generation.

### Genomic disruption of CD7 gene in T lymphocytes

A single guide RNA (sgRNA) targeting the CD7 gene (CCACGGGGACAGUCGUGCAG) was designed and synthesized using Synthego Knockout Guide Design algorithm (Synthego, CA, USA). Between 5-7 × 10⁶ of T lymphocytes were electroporated on day 2 of expansion after bead removal, using 2 µL of gRNA (100pmol/µL) and 2 µL of TrueCat Cas9 protein v2 (Cat# A36499; Thermo Fisher Scientific). The molar ratio of Cas9:gRNA was 1:3.3. The ribonucleoprotein (RNP) was prepared immediately before electroporation by incubating the gRNA and Cas9 at room temperature for 15 minutes. The Neon Transfection System (Thermo Fisher Scientific) was used for electroporation. Activated T cells were electroporated with the RNP complexes in 50 µL of buffer R using 3 pulses of 1600-V 10-ms each. CAR-T cells were quickly transferred into their conditioned media at a concentration of 1.5 × 10⁶ M/mL. Knockout efficiency was assessed by flow cytometry at day 8-10 of T cell expansion.

### Cell lines maintenance

Cell lines for this study were obtained from the American Type Culture Collection (ATCC) and Invitrogen. The Jurkat cell line (Clone E6.1; ATCC TIB-152), MOLT-4 cell line (ATCC CRL-1582) and CCRF-CEM (ATCC CRM-CCL-119) cell line were maintained in RPMI 1640 media (Gibco BRL Life Technologies) supplemented with 10% heat-inactivated FBS (Cat# F4135; Merck) and 1% penicillin-streptomycin (Gibco BRL Life Technologies). The 293FT cell line (Cat# R70007, Invitrogen, Waltham, MA, USA) was maintained in DMEM media (Cat# 41965062; Thermo Fisher Scientific) containing 10% heat-inactivated FBS (Cat#, F9665; Merck), 1% penicillin-streptomycin (Gibco BRL Life Technologies), 1% HEPES (Sigma-Aldrich), 1% non-essential amino acids solution (Cat# 11140035; Thermo Fisher Scientific) and 1% sodium pyruvate (Cat# 11360070; Thermo Fisher Scientific). All cell lines were expanded according to ATCC recommendations. Cells were maintained in a humidified atmosphere containing 5% carbon dioxide (CO2) at 37°C. All cell lines were routinely tested for Mycoplasma.

For the titration of the CD7 CAR lentiviral vector we generated a CD7KO Jurkat cell line using the previously mentioned sgRNA targeting the CD7 gene. 5 × 10⁶ cells were electroporated using the Neon Transfection System (Thermo Fisher Scientific) in 50 µL of buffer R using 3 pulses of 1325-V 10-ms each. CD7 expression was verified by flow cytometry and CD7-negative cells were sorted using fluorescence-activated cell sorting (FACS) on FACSAria II (BD Biosciences).

GFP and Luciferase-expressing cell lines (Jurkat, MOLT-4 and CCRF-CEM) were generated by transducing the parental cell line with a lentiviral vector encoding the Click Beetle Green (CBG) luciferase and GFP, connected by a T2A self-cleavage peptide, under the EF1α promoter. Transduction efficiency was confirmed by flow cytometry and GFP positive cells were sorted to establish the Jurkat-GFP-LUC, MOLT4-GFP-LUC and CCRF-CEM-GFP-LUC cell lines.

### Flow cytometry and antibodies

To assess CAR expression on the cell surface, CAR-T cells were incubated with biotin-SP AffiniPure Goat Anti-Mouse IgG (Cat# 115-065-072; Jackson ImmunoResearch, West Grove, PA, USA) for 30 minutes at 4°C. Subsequently, cells were stained with fluorochrome-conjugated antibodies for 30 minutes at 4°C or 20 minutes at room temperature. The antibodies used in this study are listed below: CD7-APC (Cat# 561604; BD Biosciences, San Jose, CA, USA), CD7-BV421 (BD Biosciences, 562635), CD4-FITC (Cat# 557695; BD Biosciences), CD4-PE (Cat# 12-0048-42; Invitrogen), CD4-PECy5 (BD Biosciences, 555348), CD8-PECy7 (Cat# 557746; BD Biosciences), CD8-APC (Cat# 300912; Biolegend, San Diego, CA, USA), CD8-PE (BD Biosciences, 345773), and streptavidine-PE (Cat# 12-4317-87; Invitrogen). Viability dye was performed using eBioscience Fixable Viability Dye eFluor450 (Cat# 65-0863-18; Thermo Fisher Scientific) or Far Red (Invitrogen, L34974). To quantify cell counts by flow cytometry in the proliferation assays, CountBright Absolute Counting Beads (Cat# C36950; Invitrogen) were added to the stained sample. All antibodies were diluted in FACS buffer solution made from phosphate-buffered saline (PBS) and 2% FBS, except for the viability dye, which was resuspended in PBS. All flow cytometry data were obtained in BD FACSCantoll (BD Biosciences) or BD LSR FORTESSA SORP (BD Biosciences) or Attune NxT (ThermoFisher Scientific) and analyzed with FlowJo software.

### In vitro coculture experiments

For luciferase-based assay coculture experiments, cryopreserved CAR-T cells were thawed and incubated overnight in media containing IL-7 (10 ng/mL) and IL-15 (10 ng/mL). After 16 hours of resting, T cells were washed and resuspended in fresh media in the absence of cytokines and no cytokines are added during coculture experiments. The CD4+:CD8+ ratio was maintained at 1:1 in all experiments, unless specified. In the luciferase-based cytotoxicity assay, 2.5 × 10⁴ untransduced (UTD) or CAR-T cells were cocultured with tumor cell expressing GFP-LUC at effector:target (E:T) ratios of 1:1 and 1:3. Cells were seeded in triplicate in a white opaque plate. After 24 hours, cytotoxicity was assessed using a luciferase-based assay with the addition of 10 µL of XenoLight D-luciferin (2 mg/mL; Cat# 122799; PerkinElmer, MA, USA). For the cytokine production assay, 7.5 × 10⁴ UTD or CAR-T cells were seeded with tumor cells at an E:T ratio of 3:1. After 24 hours of stimulation, cells were harvested in duplicate, and supernatants collected. The secretion of IL-2, IFNy, was measured using the Human IL-2 DuoSet ELISA kit (Cat# DY202; R&D Systems, MN, USA) and the Human IFNγ Duoset ELISA kit (Cat# DY285B; R&D Systems) following manufacturer's instructions. In the long-term proliferation assays, 2.5 × 10⁵ UTD or CAR-T cells were cocultured in duplicate with tumor cells at an E:T ratio of 1:2 for 4 and 7 days. At the end of the coculture period, the content of each well was collected, and the cells were stained with a viability dye, CD4-PE and CD8-APC. Tumor cells were differentiated from T cells in flow cytometry analysis as they express GFP. CountBright beads were added to the samples for cell count quantification prior to acquisition by flow cytometry using BD LSR FORTESSA SORP.

For GFP-based cytotoxic assays, freshly collected CAR-T cells from a day 8 expansion were seeded at different E:T ratios, maintaining a fixed number of target T cells (2 × 10⁵). E:T ratios of 2:1, 1:1, 0.5:1 and 0.25:1 were tested. After 24 hours cells were harvested in duplicate, and supernatants collected. Then, cells were incubated with LIVE/DEAD Fixable Aqua (Invitrogen, L34966) or Far Red (Invitrogen, L34974) reagent (Life Technologies). Cytotoxicity was determined by flow cytometry, calculating the number of surviving target T cells (identified as Fixable Aqua-negative and GFP-positive cells). For that, acquisition was stopped after a fixed volume in the cytometer. The remaining target cell number in each condition was compared to control target cells grown alone without effector cells, allowing comparison between different E:T ratios. Attune NxT (ThermoFisher Scientific) cytometer was employed. Coculture supernatants from the cytotoxic assays at different E:T ratios were analyzed for cytokine production (IFNγ, TNFα, and Granzyme B) by ELISA, following the manufacturer's instructions (R&D systems duo set, Human IFNγ DTY285B-05, Human Granzyme B DY2906-05, TNF-α DY210-05). All experiments were run in triplicate.

### T cells acute lymphoblastic leukemia (T-ALL) mouse xenograft model

For the in vivo experiments, immune-compromised NOD-SCID-Gamma (NSG) male mice (aged 8-12 weeks) were engrafted with 1 × 10⁶ Jurkat-GFP-LUC cells by intravenous (IV) injection. After one week, a single dose of 2-5 × 10⁶ total UTD or CAR-T cells was injected IV into the mice. The cell populations injected consisted of 50% CD4+ and 50% CD8+, with CAR expression ranging between 50-70%. For in vivo experiments including the CD7-negative selected anti-CD7 CAR-T cells, the initial cell populations were isolated from CD3+ T lymphocytes and therefore, the CD4:CD8 ratio was not 1:1 as specified. Treated mice were imaged weekly for tumor bioluminescence. Mice were injected intraperitoneally with 100 µL of D-luciferine (20 mg/mL; PerkinElmer, Waltham, MA, USA) and tumor burden was monitored using an IVIS Lumina LT In vivo Imaging System (PerkinElmer). Living Image software (PerkinElmer) was used to visualize and calculate tumor burden.

To analyze T cell persistence, peripheral blood samples were obtained by retro-orbital bleeding at the indicated timepoints. Blood was examined for T cell persistence using BD Trucount (Cat# 340334, BD Biosciences), and stained for CD45+, CD4+, CD8+ and CD7+ markers. All procedures were conducted in compliance with the Animal Ethics Committee of the Universitat de Barcelona.

### Statistical analysis

Graphs and statistical analysis were performed using GraphPad Prism Software version 9.0.1. (GraphPad Software, CA, USA). All data are presented as mean ± standard deviation (SD), except for mouse tumor photon flux quantification shown as mean ± standard error of the mean (SEM). One-way ANOVA test was used for three-group comparisons in a single condition. Statistical analysis of tumor flux was performed using two-way ANOVA test. Kaplan-Meier survival data were analyzed using a log rank (Mantel-Cox) test. P-values are represented as follows: not significant (ns), *p < 0.05, **p < 0.01, ***p < 0.001 or ****p < 0.0001.

1-Jones DB. Leucocyte typing IV-white cell differentiation antigens. W. Knapp et al. (Eds). Oxford University Press, Oxford, 1989. No. of pages: 1820. Price: £90. ISBN: 0192618679. The Journal of Pathology. 1990;162(2):181-.

### Results

### Design of the scFv targeting CD7

To establish the single chain variable fragments (scFvs) to be used in the design of the anti-CD7 chimeric antigen receptor (CAR) constructs, three different anti-CD7 hybridomas were sequenced. These three hybridomas, whose specificity was previously tested, are known as CD7_121.1D1, CD7_142.24, and CD7_142.9. Their corresponding cDNA was synthesized from total RNA of each hybridoma cells. Genes encoding light chain (VL) and heavy chain (VH) regions of each monoclonal antibody against CD7 were amplified by PCR and sequenced (see sequence listing above). VL fragments size were 312 bp for CD7_124.1D1 and CD7_142.24, and 318 bp for CD7_142.9. VH fragments size were 357 bp for CD7_124.1D1 and CD7_142.24, and 363 bp for CD7_142.9. Then, the amino acid sequences from the complementarity-determining regions (CDRs) 1, 2 and 3 of each variable region of both VL and VH were identified according to the method provided by the Integrated antibody sequence & structure analysis (abYsis) web-based platform, using Kabat scheme.

The predicted CDR1, CDR2 and CDR3 of VL domain were located at positions 24-34, 50-56, and 89-97 in the CD7_124.1D1 protein sequence; 24-34, 50-56, and 89-97 in the CD7_142.24 protein sequence; and 24-33, 49-55, and 88-96 in CD7_142.9 in the protein sequence, respectively (see sequence listing above). The prediction of CDR1, CDR2 and CDR3 of VH domain was specified at positions 31-36, 51-66, and 99-108 in the CD7_124.1D1 of the protein sequence; 31-35, 50-66 and 99-110 in the CD7_142.24 of the protein sequences; and 31-35, 50-66 and 99-110 in CD7_142.9 of the protein sequence, respectively (see sequence listing above).

To produce full-length scFvs derived from the anti-CD7 antibodies, a 20 amino acid linker encoding (Gly4Ser)₄ was introduced between the VL and the VH domains.

### Quantification and characterization of the protein binding affinities of the anti-CD7 antibodies.

We performed a quantification and characterization of the protein binding affinity between the three anti-CD7 antibodies and the CD7 antigen. By analyzing the hydrodynamic radius change within a fluidic system, the dissociation constant (KD) of the three antibodies against the CD7 antigen was determined (Figures 1, 2 and 3). The data yield a defined KD for this interaction, being the 124.1D1 hybridoma-derived antibodies the ones showing the highest affinity (13.6 nM with 95% HDI of [2.1, 38.1] nM), followed by 142.24 (22.4 nM with 95% HDI of [10.5, 48.9] nM) and 142.9 (52.4 nM with 95% HDI of [17.5, 155.4] nM) hybridoma-derived antibodies. All affinities were in the nM range, indicating a similar affinity of the three antibodies against the CD7 target antigen.

### Development of CD7KO anti-CD7 CAR-T cells

To generate anti-CD7 CAR-T cells, the three scFvs were cloned into a second-generation CAR backbone. These anti-CD7 CARs contain a CD8 hinge and transmembrane domain, along with the 4-1BB and CD3ζ intracellular signaling domains. To compare the three CAR-T cell products (124.1D1, 142.24 and 142.9) (Figure 4A) while preventing T-cell fratricide, we generated anti-CD7 CAR-T cells by knocking out the CD7 gene following the procedure illustrated in Figure 4B. Primary CD4+ and CD8+ T lymphocytes were isolated and stimulated 24 hours prior to lentiviral transduction. At this point, the media was supplemented with dasatinib, a tyrosine kinase inhibitor of LCK1, to inhibit fratricide caused by antigen recognition. The CD7 gene of the CAR-T cells was knocked out using CRISPR/Cas9 technology on day 2. Since CD7 surface expression downregulation after knock-out is not immediate, dasatinib was added to the media every 48 hours until day 6 of expansion. Transduction of T lymphocytes with lentiviral vectors resulted in an efficient expression of the three CD7 CAR constructs (Figure 4C and 4D). Effective disruption of the CD7 gene was confirmed by flow cytometry on day 9 of T cell ex *vivo* expansion. This protocol enabled the generation of CD7KO anti-CD7 CAR-T cells. However, the final population doublings of these cells were significantly lower compared to control T cells (Figure 4E). This may be due to cell death caused by electroporation of the RNA and/or some level of antigen-driven fratricide.

### CD7KO anti-CD7 CAR-T cells get activated and kill T-ALL cells in vitro.

To assess the antitumor efficacy of the CD7KO anti-CD7 CAR-T cells, these T cells were cocultured with the CD7-expressing T-cell acute lymphoblastic leukemia (T-ALL) derived Jurkat, MOLT-4 and CCRF-CEM cell lines. The cell lines were labeled with Click Beetle Green luciferase and GFP. The three anti-CD7 CAR constructs exhibited potent cytotoxicity against the T-ALL cell lines after 24 hours, as determined by a luciferase-based assay (Figure 5A). To evaluate cytokine production, CAR-T cells and tumor cells were cocultured for 24 hours at a 3:1 E:T ratio (Figure 5B). CAR-mediated activation was confirmed by the production of IFNγ and IL-2, indicating no differences among the CAR-T cell populations when cocultured with the three tumor cell lines. We next assessed T cell proliferation in a 7-day coculture experiment (Figure 5C-D). This experiment demonstrated complete eradication of the tumor population by all CAR-T cell populations. However, discrepancies were observed in terms of long-term proliferation, with the 124.1D1 and 142.24 constructs exhibiting superior fold-increase compared to the 142.9 construct (Figure 5D).

### CD7KO anti-CD7 CAR-T cells control disease progression in mouse xenograft models of T-ALL.

To assess the *in vivo* antitumor activity of CD7 CAR-T cells, NOD-SCID-Gamma (NSG) animals were engrafted with Jurkat-GFP-LUC cells (Figure 6A). One week after tumor engraftment, animals were treated with a single intravenous injection of untransduced (UTD) or CD7KO anti-CD7 CAR-T cells (70-80% CAR+). Tumor burden was monitored weekly using an IVIS imaging system. The 124.1D1 and 142.24 CAR-T cells were able to control tumor progression (Figure 6B-C), resulting in a significant reduction in tumor burden compared to the control UTD group. However, the 142.9 CAR-T cells did not exhibit potent *in vivo* antitumor activity. The robust antitumor activity of the 124.1D1 and 142.24 CAR-T cells led to a significant improvement in the survival of mice treated with these cells compared to the control and 142.9 group (median survival: UTD=28, 124.1D1=49, 142.9=28, and 142.24=58 days) (Figure 6D). The enhanced antitumor activity of the 124.1D1 and 142.24 CAR-T cells correlated with the increased proliferation rates observed *in vitro.* Although the 142.9 CAR-T cells demonstrated a potent cytotoxic activity *in vitro,* they were unable to proliferate and to eradicate tumors *in vivo.*

### Generation of anti-CD7 142.24 CAR-T cells by lentiviral transduction after CD7 gene editing using CRISPR/Cas9.

To avoid the use of pharmacological inhibitors such as dasatinib during T cell ex *vivo* expansion, lentiviral transduction was delayed and performed 24 hours after CD7 knock-out using CRISPR/Cas9 technology (Figure 7A). The 142.24 (LV Day 3) CAR-T cells were successfully generated using this expansion protocol. These CAR-T cells demonstrated the ability to recognize the target antigen when cocultured with CD7+ tumor cells (Jurkat and CCRF-CEM), as evidenced by the production of IL-2 in the supernatant (Figure 7B). To evaluate their in vivo antitumor activity, we utilized a Jurkat-GFP-LUC NSG xenograft mouse model (Figure 7C). A single dose of 2×10⁶ total T cells (50% CAR+ T cells) was intravenously injected seven days after tumor engraftment. Tumor burden was monitored weekly using an IVIS imaging system. The 142.24 LV Day 3 CAR-T cells effectively delayed tumor progression *in vivo,* resulting in a significant reduction in tumor burden compared to the UTD control (Figure 7D). The cytotoxic activity exerted by the 142.24 LV Day 3 CAR-T cells in vivo led to a significant increase in the survival of mice (28 days in the control group vs. 42 days in the LV Day 3 group) (Figure 7E). These findings collectively suggest that the generation of CD7KO anti-CD7 CAR-T cells is feasible in the absence of tyrosine kinase inhibitors by delaying lentiviral transduction by 48 hours.

### Generation and functional characterization of the CD7trap constructs

A non-gene editing strategy was developed to retain CD7 cell surface expression in order to avoid fratricide. Three different CD7trap constructs were designed, one for each anti-CD7 scFv available (Figure 8A). To assess the CD7trap constructs in human T cells, peripheral blood mononuclear cells (PBMCs) were obtained from healthy donors. CD3+ T cells were transduced with lentiviral vectors expressing the CD7trap along with GFP and expanded ex *vivo.* CD7 cell surface expression was decreased by all CD7trap constructs. The highest retention in terms of CD7 MFI decrease was reached by the 124.1D1 construct (Figure 8B). Therefore, the 124.1D1 CD7trap was selected for further experiments.

To characterize the CD7 intracellular retention capacity of the 124.1D1 CD7trap, Jurkat cells were transduced with a lentiviral vector expressing the CD7trap along with a GFP protein to track transduced cells. We tested 4 different MOls to determine the most suitable for transduction efficiency (Figure 8C). We also evaluated the CD7 expression in the cell surface, which was MOI dependent (Figure 8D), and intracellularly, which was not altered by the 14.1D1 CD7trap system (Figure 8E).

### Generation of anti-CD7 CAR-T cells containing the CARCD7 142.24trap or 142.9trap

CAR constructs designed using the scFv derived from the 142.24 and 142.9 antibodies were fused to 124.1D1 CD7trap. This resulted in two different bicistronic constructs: CARCD7 142.24trap and CARCD7 142.9trap (Figure 9A). The 124.1D1 scFv was excluded from the CAR design in these bicistronic constructs because it was already selected for the CD7trap and the presence of two identical nucleotide sequences along the same final construct could lead to homologous recombination and hinder the proper co-expression of both proteins (anti-CD7 CAR and CD7trap). Lentiviral vectors encoding for the CARCD7 142.24trap and 142.9trap constructs were generated and used to transduce T cells. Analysis of CAR expression by flow cytometry confirmed that both CARs were efficiently expressed in the T cell membrane (Figure 9B).

To study the *in vitro* expansion capacity and fratricide evasion of CARCD7 142.24trap and 142.9trap T cells, their population doublings over time were analyzed in comparison to UTD control T cells and the three anti-CD7 CAR-T cell products (124.1D1, 142.24 and 142.9). While anti-CD7 CAR-T cells lacking a trap were unable to expand due to T cell fratricide, both CARCD7trap T cells were able to expand similarly to control T cells (Figure 9C-D). Our results suggest that the 124.1D1 CD7trap construct enables the CD7 intracellular retention, and consequently prevents fratricide, allowing a better T cell expansion.

### CARCD7 142.24trap antitumor activity in vitro.

To demonstrate that T cells expressing the CARCD7 142.24trap or the CARCD7 142.9trap constructs generate cytotoxic activity against target cells expressing the CD7 antigen, cytotoxicity assays were conducted by challenging CAR-T cells with Jurkat and MOLT4, both CD7-expressing T-ALL cell lines. The cytotoxic capacity of T cells transduced with the CARCD7 142.24trap was determined by the in vitro elimination of the CD7-positive Jurkat (Figure 10A) and MOLT4 cell lines (Figure 10B). On the other hand, T cells expressing CARCD7 142.9trap were not able to kill these tumor cell lines (data not shown) and, thus, this construct was discarded.

Next, CARCD7 142.24trap T cell production of cytokines was measured in the supernatant of effector-target cocultures after 24 hours and analyzed using ELISA. Cytokine levels from cocultures with both Jurkat (Figure 10C) and MOLT4 (Figure 10D) target cell lines using CARCD7 142.24trap or UTD T cells were compared. While UTD T cells did not show an increase in IFNγ and Granzyme-B, CARCD7 142.24trap T cell cocultured with Jurkat and MOLT4 showed a significant increase in these two pro-inflammatory cytokines. CARCD7 142.24trap T cells also showed an increase in TNFα when cocultured with MOLT4 cell line.

### CARCD7 142.24trap antitumor activity in vivo.

In order to assess the antitumor activity of the CARCD7 142.24trap, NSG mice were injected intravenously with 1×10⁶ Jurkat-GFP-LUC cells to generate a T-ALL mouse xenograft model. Seven days later, animals were treated with a single injection of 3-5×10⁶ of control T cells or 142.24trap CAR-T cells. Tumor growth was monitored weekly using an IVIS imaging system (Figure 11A). CARCD7trap 142.24 T cells group significantly delayed tumor progression compared to control T cells (Figure 11B). This potent antitumor effect was reflected in a higher survival rate of CARCD7trap 142.24 treatment group compared to control T cells (Figure 11C).

### Generation of CD7-negative T cells containing the CARCD7 124.1D1, 142.24 or 142.24trap.

Recently, Freiwan A. et al have reported that naturally occurring CD7-negative T cells can be used in CAR-T therapy, as they exhibit a promising antitumor profile against hematological malignancies. To explore this strategy, we performed *in vitro* experiments with CD7-negative T lymphocytes isolated from healthy donors. In these experiments, we used T cells modified to express the CAR derived from antibodies 124.1D1 and 142.24. However, as naturally occurring CD7-negative T lymphocytes do express certain levels of CD7 during their stimulation with CD3/CD28 beads, the CARCD7 142.24trap construct was also included in these experiments.

The *in vitro* characterization of CD7-negative T cells transduced to express CARCD7 124.1D1, 142.24 and 142.24trap demonstrated significant differences compared to control T cells at different ratios in terms of cytotoxicity (Figure 12A) and pro-inflammatory cytokine secretion (Figure 12B). The cytotoxicity of CD7-negative T cells transduced with CARCD7 124.1D1, 142.24 and 142.24trap was determined by the *in vitro* elimination of the CD7-positive Jurkat cell line and cytokines cell secretion was measured in the supernatant of these effector-target cells cocultures. While control T cells did not show an increase in IFNγ and Granzyme-B, CARCD7 124.1D1, 142.24 and 142.24trap T cells showed a significant increase in these two pro-inflammatory cytokines at E:T ratios of 2:1 and 1:1. CARCD7 124.1D1, 142.24 and 142.24trap T cells also showed an increase in TNFα in the cocultures with Jurkat at a ratio E:T of 2:1.

### Generation of anti-CD7 142.24 CAR-T cells through pharmacological inhibition of fratricide.

As pharmacological inhibitors can be useful for suppressing CAR signaling, our next approach aimed to expand anti-CD7 142.24 CAR-T cells without relying on gene editing strategies like CRISPR/Cas9 technology mentioned previously. We compared two expansion protocols that involved the supplementation of dasatinib in the culture media (Figure 13A). In both protocols, lentiviral transduction was performed 24 hours after T cell stimulation, and dasatinib (50nM) was added to the media every 48 hours from day 1 until the end of the ex *vivo* expansion protocol. The long-term expanded cells were maintained in cell culture for 10 days, while the short-term expanded cells were cryopreserved on day 3. To assess the antitumor efficacy of these cells, they were cocultured with Jurkat and MOLT4 tumor cells for 24 hours (Figure 13B). Cytotoxicity was evaluated using a luciferase-based assay, and both groups exhibited similar killing rates against both cell lines. Cytokine production (IFNy and IL-2) was measured in the media after 24 hours of coculture with three CD7+ T-ALL cell lines (Figure 13C). IFNγ production levels were comparable between the long-term and short-term expanded T cells, while IL-2 secretion was higher in the short-term expanded T cells, indicative of a less differentiated phenotype.

### Non-genetically modified anti-CD7 142.24 CAR-T cells control progression of systemic T-ALL.

Proliferation of the long-term and short-term expanded anti-CD7 CAR-T cells in the presence of dasatinib was assessed in a 7-day coculture experiment (Figure 14A). Short-term (D3) cells were expanded until day 3, while long-term (D10) cells were expanded until day 10. By day 4 of the coculture, we observed that the short-term group had almost completely eradicated the tumor cells, whereas the long-term group still had approximately half of the total tumor cell population. However, by the end of the 7-day coculture, no tumor cells were detected in either group. Differences in proliferation rates were found between the D10 and D3 T cells after coculture with three CD7+ T-ALL cell lines (Jurkat, CCRF-CEM, and MOLT-4) (Figure 14B). The short-term cells exhibited higher T cell proliferation rates compared to the long-term expanded T cells. This suggests that shortly expanded T cells are functional and retain a greater proliferative capacity compared to T cells expanded until day 10. The antitumor activity was also evaluated *in vivo* using the Jurkat model mentioned previously. NSG mice were treated with a single intravenous dose of 3×10⁶ total T cells from either the D10 or D3 groups (Figure 14C). Tumor burden was monitored weekly using an IVIS imaging system. The long-term group significantly delayed tumor progression compared to control T cells (Figure 14D). In contrast, the short-term group achieved complete control of disease progression, as all treated mice were free of disease by the end of the experiment. These findings regarding tumor burden were reflected in a higher survival rate for the short-term expanded T cells compared to the long-term group (Figure 14E). While the median survival of the long-term group was 28 days, none of the mice in the short-term group died. These results indicate that pharmacological inhibition during T cell expansion enables the generation of functional anti-CD7 CAR-T cells that exhibit strong antitumor activity and proliferation both *in vitro* and *in vivo.* Additionally, the duration of ex *vivo* expansion may be crucial in achieving long-lasting responses, as evidenced by the differences observed between long-term and short-term expanded anti-CD7 CAR-T cells.

### Different strategies of non-genetically modified anti-CD7 142.24 CAR-T cells are able to control progression of systemic T cell acute lymphoblastic leukemia (T-ALL) in a mouse xenograft.

The next approach was to characterize and compare the functionality of the anti-CD7 CAR-T cells obtained using two non-gene editing expansion strategies: a) a CD7-negative (CD7neg) selection following T cells isolation and b) short-term expanded CAR-T cells (day 3). Primary CD3+ T lymphocytes were isolated and the CD7neg population was further purified using anti-CD7 microbeads. In all groups, T cells were stimulated 24 hours prior to lentiviral transduction. Control T cells and CD7neg CAR-T cells were maintained in culture until day 11. However, the short-expanded cells were the only group expanded for three days. These strategies prevented T cell fratricide during the manufacturing process allowing the generation of anti-CD7 CAR T cells.

To compare the efficacy of these CAR-T cells, NSG mice were treated with 3×10⁶ control T, CD7neg anti-CD7 142.24 CAR-T cells, or Day 3 anti-CD7 142.24 CAR-T cells one week after Jurkat tumor engraftment (Figure 15A). As the anti-CD7 CAR-T cells expanded for 3 days did not undergo *in vitro* expansion, we hypothesized that these T cells retained superior proliferative capacity, which would allow to lower the dosage while obtaining the same antitumor response. For that purpose, a cohort of animals was treated with one-third of the dose of the anti-CD7 CAR-T D3 cells. All groups showed a significantly enhanced *in vivo* antitumor activity compared to the control group (Figure 15B). The CD7neg-infused T cells exhibited great antitumor activity. Both dosages of the CD7 CAR-T D3 groups demonstrated total control of tumor progression *in vivo,* suggesting that the T cell dosage of shortly expanded T cells can be reduced without losing antitumor efficacy. T cell persistence in the peripheral blood of treated mice was measured at different timepoints after CAR-T cell treatment (Figure 15C). Significant differences in T cell persistence were observed 16 days after CAR-T cell treatment. The D3 CAR-T cells demonstrated robust persistence, suggesting that T cell expansion took place *in vivo* instead of *in vitro.* According to the cell counts obtained in the different timepoints, T cells of this group proliferated exponentially in the treated animals. On the other hand, no persistence was observed in the CD7neg anti-CD7 CAR-T cells, showing similar counts to those of untransduced T cells. The antitumor activity of these treatment groups was also observed in terms of survival (Figure 15D). The CD7neg anti-CD7 CAR-T cells and the two dosages of the Day 3 strategy showed increased survival compared to the control group, suggesting that these strategies could be good therapeutic options for T-ALL malignancies.

### Humanized anti-CD7 CAR-T cells exhibit in vitro efficacy against T-ALL

Based on the murine sequences obtained from the CD7 hybridomas (124.1D1, 142.24 and 142.9) we humanized sequences by CDR grafting, while retaining those murine framework residues that influence the antigen-binding activity and the structural configuration (see sequence listing above). Murine 142.24 and humanized 142.24 anti-CD7 CAR-T cells were generated through the disruption of CD7 expression using CRISPR/Cas9 technology. The effectiveness of the humanized scFv and its distinctions from the murine counterpart were evaluated *in vitro* by co-culturing T cells with CD7+ T-ALL cell lines expressing GFP-LUC. Both anti-CD7 CAR-T cells demonstrated potent cytotoxicity against T-ALL cell lines, as observed through a luciferase-based assay after 24 hours of coculture (Figure 16A). For the assessment of cytokine production, CAR-T cells were cocultured with tumor cells at an E:T ratio of 3:1 for 24 hours (Figure 16B). The specificity was validated by IFNγ production, revealing consistent results across CAR-T cell populations in all three tumor cell lines. Given the significance of CAR-T cell expansion in achieving a comprehensive response, we conducted a 4- and 7-day coculture experiment to evaluate T cell proliferation (Figures 16C-D). This investigation unveiled the total eradication of the tumor population by both the murine and humanized constructs, with comparable proliferation rates observed at the end of the coculture.

### Bibliography

1. Mestermann, K. et al. The tyrosine kinase inhibitor dasatinib acts as a pharmacologic on/off switch for CAR-T cells. 11, (2020).
2. Freiwan, A. et al. Engineering naturally occurring CD7- T cells for the immunotherapy of hematological malignancies. Blood 140, 2684-2696 (2022).

## Claims

1. A **CD7 targeting-moiety,** wherein the CD7 targeting-moiety is an antibody, F(ab')2, Fab, scFab or scFv, comprising a VL domain and a VH domain, wherein:
- the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, or
- the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9.

2. The CD7 targeting-moiety according to claim 1, wherein the CD7 targeting-moiety is a scFv comprising a VL domain and VH domain, wherein:
- the VL domain comprises SEQ ID NO: 20 **(CD7_142.24 VL)** and the VH domain comprises SEQ ID NO: 21 **(CD7_142.24 VH),** or
- the VL domain comprises SEQ ID NO: 1 **(CD7_124.1D1 VL)** and the VH domain comprises SEQ ID NO: 2 **(CD7_124.1D1 VH).**

3. The CD7 targeting-moiety according to any one of claims 1 or 2, wherein the CD7 targeting-moiety is a scFv consisting of SEQ ID NO: 11 **(CD7_142.24)** or SEQ ID NO: 3 **(CD7_124.1D1).**

4. A **chimeric antigen receptor (CAR)** comprising:
a. an extracellular domain comprising a CD7 targeting-moiety, wherein the CD7 targeting-moiety is as defined in any of claims 1 to 3;
b. a transmembrane domain, preferably the transmembrane domain of CD8,
c. at least one intracellular signaling domain, preferably the intracellular domain of CD3ζ, and
d. optionally, a costimulatory signalling domain, prefrably the intracellular domain of CD137.

5. A **CD7-trap protein** comprising the CD7 targeting moiety according to any one of claims 1 to 3, and further comprising a CD7-retention sequence comprising SEQ ID NO: 30 (ER retention sequence).

6. A **combination therapy** comprising:
i) the CAR against CD7 as defined in claim 4, or a nucleic acid molecule encoding thereof, and
ii) the CD7-trap protein as defined in claim 5, or a nucleic acid molecule encoding thereof.

7. The combination therapy according to claim 6, wherein:
i) the CAR against CD7, or a nucleic acid molecule encoding thereof, comprises:
(a) an extracellular domain comprising a CD7 targeting moiety that is a ScFv comprising a VL domain and a VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, and
(b) a transmembrane domain, preferably the transmembrane domain of CD8;
(c) an intracellular signaling domain, preferably the intracellular domain of CD3ζ, and
(d) optionally, at least a costimulatory signaling domain, preferably the intracellular domain of CD137, and
ii) the CD7-trap protein, or the nucleic acid molecule encoding thereof, comprises:
(a) a ScFv comprising a VL and a VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
(b) a retention sequence that comprises SEQ ID NO: 30 [AEKDEL].

8. A **nucleic acid** encoding for one or more of the CD7 targeting moiety according to any one of claims 1 to 3, the CAR according to claim 4, or the CD7-trap protein according to claim 5.

9. The nucleic acid according to claim 8, wherein said nucleic acid is a bicistronic nucleic acid and comprises, in the 5' to 3' direction:
- a first nucleotide sequence encoding for a CAR against CD7 that comprises:
(a) an extracellular domain comprising a CD7 targeting moiety that is a ScFv comprising a VL domain and a VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 (CD7_142.24 VH), and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, and
(b) a transmembrane domain, preferably the transmembrane domain of CD8;
(c) an intracellular signaling domain, preferably the intracellular domain of CD3ζ, and
(d) optionally, at least a costimulatory signaling domain, preferably the intra-cellular domain of CD137, and
- a second nucleotide sequence encoding for the CD7-trap protein that comprises:
(a) a ScFv comprising a VL and a VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
(b) a retention sequence comprising SEQ ID NO: 30 [AEKDEL].

10. A **cell** comprising and/or expressing one or more of:
- the CD7 targeting-moiety as defined in any one of claims 1 to 3,
- the CAR as defined in claim 4,
- the CD7-trap protein as defined in claim 5,
- items i) and ii) of the combination therapy as defined in any one of claims 6 or 7, and/or
- the nucleic acid as defined in any one of claims 8 or 9.

11. The cell according to claim 10, comprising and/or expressing:
- a CAR against CD7 that comprises:
(a) an extracellular domain comprising a CD7 targeting moiety that is a ScFv comprising a VL domain and a VH domain, wherein the VL domain comprises a sequence with at least 85% sequence identity to SEQ ID NO: 20 **(CD7_142.24 VL),** and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 **(CD7_142.24 VH),** and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, and
(b) a transmembrane domain, preferably the transmembrane domain of CD8;
(c) an intracellular signaling domain, preferably the intracellular domain of CD3ζ, and
(d) optionally, at least a costimulatory signaling domain, preferably the intra-cellular domain of CD137, and
- a CD7-trap protein that comprises:
(a) a ScFv comprising a VL and a VH domain, wherein the VL domain comprises a sequence with at least 85% sequence identity to SEQ ID NO: 1 **(CD7_124.1D1 VL),** and wherein said VL domain comprises CDR regions consisting of LCDR1 as set forth in SEQ ID NO: 4, LCDR2 as set forth in SEQ ID NO: 5, LCDR3 as set forth in SEQ ID NO: 6; and wherein the VH domain comprises a sequence with at least 85% sequence identity to SEQ ID NO: 2 **(CD7_124.1D1 VH),** and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9; and
(b) a retention sequence comprising SEQ ID NO: 30 [AEKDEL].

12. The CD7 targeting-moiety according to any one of claims 1 to 3, the CAR according to claim 4, the CD7-trap protein according to claim 5, the combination therapy according to any one of claims 6 or 7, the nucleic acid according to any one of claims 8 or 9, the cell according any one of claims 10 or 11, **for use** in therapy.

13. The CD7 targeting-moiety according to any one of claims 1 to 3, the CAR according to claim 4, the CD7-trap protein according to claim 5, the combination therapy according to any one of claims 6 or 7, the nucleic acid according to any one of claims 8 or 9, the cell according any one of claims 10 or 11, **for use** in a method of treating a CD7-positive cancer.

14. The CD7 targeting moiety, the CAR, the CD7-trap, the combination therapy, the nucleic acid, or the cell for use according to claim 13, wherein the CD7-positive cancer is T cell acute lymphoblastic leukemia (T-ALL).
